# EUROPEAN PATENT SPECIFICATION

(11) **EP 1 182 931 B1**
(45) Date of publication and mention of the grant of the patent: **28.12.2005**
(21) Application number: 00931765.2
(22) Date of filing: 02.06.2000
(51) Int. Cl.: A01N 43/90, A01N 43/52, A01N 25/02, A61K 31/4184, A61K 31/429

(54) **STABLE BIOCIDAL COMPOSITIONS**
STABILE BIOZIDALE ZUSAMMENSETZUNG
COMPOSITIONS BIOCIDES STABLES

(30) Priority: 04.06.1999 NZ 33613999; 10.06.1999 NZ 33621399; 19.07.1999 NZ 33681499
(43) Date of publication of application: 06.03.2002
(73) Proprietor: NUFARM LIMITED, Laverton North VIC 3026 (AU)
(72) Inventor: SORENSEN, Allen Paul, Auckland (NZ); VICKERS, Mark Colin, Auckland (NZ)
(74) Representative: Hayes, Adrian Chetwynd
(86) International application number: PCT/NZ2000/000087
(87) International publication number: WO 2000/074489

(56) References cited:
- EP-A- 0 209 462
- EP-A1- 0 224 249
- WO-A-86/07525
- US-A- 3 980 791
- US-A- 4 278 684
- US-A- 4 395 407
- ZA-A- 8 402 571
- DATABASE WPI Derwent Publications Ltd., London, GB; Class P75, AN 1992-313277/38, XP002906242 & JP 4 220 398 A (FUJI PHOTO FILM CO LTD) 11 August 1992
- DATABASE WPI Derwent Publications Ltd., London, GB; Class B07, AN 1977-12122Y/07, XP002906243 & JP 52 001 046 A (OSHIO SANGYO KK) 06 January 1977
- DATABASE WPI Derwent Publications Ltd., London, GB; Class B02, AN 1983-26659K/11, XP002906244 & JP 58 021 615 A (FUJISAWA PHARM KK) 08 February 1983
- DATABASE WPI Derwent Publications Ltd., London, GB; Class Q31, AN 1983-26424K/11, XP002906245 & JP 58 020 622 A (OSHIO SANGYO KK) 07 February 1983
- DATABASE WPI Derwent Publications Ltd., London, GB; Class C03, AN 1995-093720/13, XP002906246 & JP 7 017 812 A (SHINTO TORYO KK) 20 January 1995
- DATABASE WPI Derwent Publications Ltd., London, GB; Class B02, AN 1999-081974/08, XP002906247 & CN 1 194 832 A (WANG Y) 07 October 1998

## Description

The present invention relates to pesticidal compositions, their preparation and their use.

More particularly the present invention relates to multiple active ingredient liquid formulations of a kind where for stability purposes different environments are required for at least two of the active ingredients. For instance, when in an aqueous phase pH requirements for stability differ significantly, eg; a low pH is required for levamisole and a neutral pH is required for abamectin, a macrocyclic lactone (ML) (see Table 2 hereafter). Also of the common anthelmintics, levamisole salts are usually much more soluble than morantel salts in aqueous systems, M.L anthelmintics are generally more soluble in organic systems than aqueous systems and benzimidazoles are sparingly soluble.

A particular (but not only) area of interest in stable formulation are those formulations of biocides useful in controlling helminths including nematodes, cestodes, trematodes, and/or ectoparasites. In this respect the mode of administration including oral, injectable or pour-on (transdermal) routes of such a formulation (whether after aqueous dilution or not) is not critical to the invention.

It is known that simultaneous administration of levamisole and mebendazole [E M Bennet, C Behm, and C Bryant, Int J Parasitology, 1978, 8, 463-466] enhances the anthelmintic activity of benzimidazoles. New Zealand Patent 208288 discloses compositions which contain levamisole and at least one substituted benzimidazole carbamate.

It is also known, based on mathematical modelling that combining 3 or more actives that act to kill a pest in different ways reduces the risk of parasite developing resistance to each of the individual actives if used separately and on their own.

It is also known that it is desirable for ease of use that these actives are combined together in one stable formulation so that administration is by one single administration of an oral, injectable, parenteral or pour-on formulation. It is also desirable that this combined formulation is sufficiently stable so that it can be stored and the formulation used or reused at a later date without degradation of the actives or significant physical changes to the formulations.

Anderson et al in "Mixtures of Anthelmintics: A Strategy Against Resistance" Australian Veterinary Journal, Vol 65, No.2, February 1988, Pages 62-64 suggested that where multiple resistance to broad spectrum nematocides had arisen, treatment with mixtures of nematocides provided effective control of the nematode infections sufficient for use in resistance preventative programs. Hence the need now, much later than 1988, for combined active formulations.

Ancare New Zealand Limited formulated a double active anthelmintic of levamisole and niclosamide in paraffin oil (LEVITAPE™) to target both tapeworm and roundworm found any water contamination rendered the product too viscous to use. They tried PEG 6000 wax encasement of niclosamide to protect it from water to no avail. They even tried a detergent based mixture of niclosamide and levamisole but whilst stable it was not safe nor easy to use as a oral drench.

Ancare found that combining benzimidazole drenches with levamisole drenches results in an unstable product due to the different pH values needed to maintain the stability of the individual products. Often, on standing for an hour or two the drenches separate out with the levamisole on top and the benzimidazole left as a sludge on the bottom. It these are not thoroughly mixed again before use the animal may be under or overdosed. A possible consequence of underdosing with the benzimidazole is the build up of a parasite resistant to the benzimidazole.

Merck NZ 183847 discloses avermectin anthelmintic compounds for parenteral administration where they may be carried by a vegetable oil such as peanut oil, cotton seed oil and the like.

Sankyo NZ 199817 refers to oral formulations of two or more anthelmintics where one is a macrolide (or ML) anthelmintic and another may be, for example, a benzimidazole (eg; albendazole), a salicylamide (eg; niclosamide) or an isoquinoline compound (eg; praziquantel). Such formulations are not exemplified by any storage stable formulation although reference is made to formulation as an aqueous solution, as a solution in another suitable non-toxic solvent or as a suspension or dispersion incorporating a suspension aid and a wetting agent (eg; bentonite) or other constituents.

Doramectin is available from Pfizer as DECTOMAX™ as a parenteral formulation of sesame oil and ethyl oleate.

Ashmont NZ 280085/280134 discloses injectable ML anthelmintic formulations (eg; abamectin or ivermectin) where a alcohol (such as benzyl alcohol - long since used as a preservative in injectable formulations) acts as a co-solvent with a vegetable oil vehicle (eg; soyabean oil, sesame oil or corn oil). The optional inclusion of ethyl oleate is also disclosed.

Ashmont further discloses (w/w %) a formulation as follows;

| | |
|---|---|
| ML anthelmintic active | 0.5 to 5% w/w |
| benzyl alcohol | 1 to 30% w/w |
| vegetable oil | to 100% w/w |
| ethyl oleate | zero or 5 to 30% w/w |

US-A-4395407 discloses compositions of levamisole and/or tetramisole and phosmet in an aliphatic carboxylic acid and a solvent such as 2-(2-butoxyethoxy)ethanol.

CN-A-1194832 discloses compositions of avermectin or ivermectin, optionally with levamisole or albendazole, eg. as emulsions. The emulsion or ointment composition contains avermectin/ivermectin, 2-80% organic solvent, other adjuvants including emulsifiers, and water to 100%.

EP-A-209462 discloses anthelmintic microemulsions containing bithionol sulphoxide, a thiazole compound such as levamisole, emulsifier, organic solvent, buffering agent and water. The buffering agent, eg. citrate system, keeps the pH of the aqueous phase at between 2 and 5, in order to achieve the stability of levamisole.

WO-A-8607525 discloses pour-on ecto- and endoparasiticidal compositions which comprise (i) tetramisole or levamisole and (ii) at least one pyrethroid in a carrier system comprising water, at least one organic water-miscible solvent, and optionally at least one organic water-immiscible solvent.

Despite combinations above being developed no stable ML anthelmintic and levamisole combination has been marketed. Previous attempts at such a combination resulted in an unstable formulation.

The present invention is directed to compositions having at least two actives preferably each usable as an anthelmintic active (albeit with a different spectrum of efficacy with respect to nematodes, trematodes, flukes, etc.) and preferably involving other therapeutic agents.

In another aspect the present invention relates to a benzimidazole containing composition (eg; for pour-on use) where the benzimidazole is solubilised in an organic acid containing phase (eg; lactic acid).

As used herein the term "anthelmintic" and derivatives thereof shall encompass, where the context allows any one or more of a nematocidal, trematocidal and cestocidal active compounds. Where the context so allows "pesticidal" and derivatives thereof shall include any such anthelmintic and any ectoparasitocidal compound. Where the context allows "ectoparasitocidal" shall include compounds effective against any one or more of ticks, lice, flies, *et al*.

As used herein the term "stable" means at least 3 months (preferably at least 18 months) chemical stability (eg; within plus or minus 10% w/w of its stated composition) of the active ingredients when stored at 25°C or below and at ambient humidity and of reasonable physical stability such that the composition is substantially homogeneous (despite any option particulate inclusion(s) and/or can readily be agitated to such condition.

As used herein the term "particle", "particles" and "particulate" means true particles (ie; parts of solids) as well as liposomes or the equivalent optionally which may be or carry an active ingredient. Preferably said particles are almost exclusively less than 100µ (microns). Ideally the smaller the better (eg; preferably substantially all less than 50µ, more preferably less than 30µ, still more preferably than 20µ and even more preferably less than 10µ).

As used herein the term "solubility" refers to the ability of a compound to be dissolved in a specific phase.

As used herein "lipophilic" means a greater tendency to an organic oil or the like phase as opposed to the other phase (preferably aqueous).

As referred to herein the term "pourable" or "flowable" in respect of a fluid or liquid covers viscosities ranging from a free flowing liquid to a gel or paste consistency that is able to be expelled by syringe, drench or paste gun. The term "pourable" is irrespective of whether it is to be used as a pour on or otherwise.

In a first aspect the invention consists in **a pesticidal composition** comprising or including
(i) at least one active ingredient that is lipophilic in character,
(ii) at least one organic liquid carrier which carries at least most of the lipophilic active ingredient(s), thereby defining an organic liquid phase,
(iii) levamisole, and
(iv) at least water which carries at least most of said levamisole thereby defining an aqueous phase,
**wherein** said aqueous phase has a pH of less than 7,
**and wherein** there is present in said aqueous phase an emulsifying agent or agents,
**and wherein** said phases exist in, or can be shaken or agitated into, the form of an emulsion with said particulate content, if any, at least substantially present in the aqueous phase.

Preferably said pesticidal composition (or indeed any biocidal composition) disclosed herein is a veterinary composition.

Preferably the lipophilic active ingredient is chosen from the class of macro cyclic lactones (hereafter "ML").

Preferably there is present in said aqueous phase a particulate content.

Ideally said aqueous phase has a pH of less than 6 (more preferably less than 5 and most preferably 4 or below).

Preferably said aqueous phase includes a buffering system to buffer the pH to preferably a pH suitable for the levamisole.

Preferably said buffering system is a citric acid/citrate salt system.

Preferably said at least one organic liquid carrier is an oil (e.g. vegetable or mineral, or mixtures thereof).

Preferably said organic liquid phase includes a co-solvent.

Preferably said co-solvent is benzyl alcohol.

Preferably said aqueous phase includes a particulate content of either an active agent or an inert substance.

Preferably the particulate active agent is a biocide.

Optionally the composition includes in one or other, or both, of the partitioned phases one or more of the group comprising minerals and vitamins.

In another aspect the invention is **an anthelmintic composition** having
about 0.08% ivermectin,
about 3% levamisole, and
about 2% albendazole
where different liquid carrier phases substantially partition the ivermectin from the levamisole, and
where the albendazole is particulate and is at least in part in an aqueous phase with the levamisole, such aqueous phase being buffered to a pH appropriate for the levamisole and its stability.

All percentages are expressed as w/v except where otherwise stated.

In another aspect the present invention consists in **a storage stable pourable pesticidal composition** having an organic first liquid phase and another ("second") liquid phase, said first phase including at least one active ingredient (and optionally a co-solvent for said active ingredient) and said second phase including a second active ingredient
**wherein** the presence of an emulsifying agent and/or anti-flocculants ensures stability of the two phases with the first phase with its first active ingredient as an emulsion within said second phase with its second active ingredient.

Preferably said second phase is an organic acid and/or aqueous phase.

Preferably said second phase comprises or includes an organic acid such as lactic acid.

Preferably said first phase comprises an oil and/or emollient ester.

Preferably said active ingredients in each of said phases or which might be present as an additional active in both or either phase comprises any of the examples hereinafter provided.

In another aspect the invention consists in **a stable mix of two or more immiscible liquids or liquid phases** held together by an emulsifier or emulsifiers where one phase is an organic water immiscible phase and another phase is an aqueous or organic phase (eg; water or organic acid(s) such as lactic acid).

In another aspect the present invention consists in **a benzimidazole composition** comprising an organic acid (such as lactic acid) and the benzimidazole dissolved therein.

In yet a further aspect the present invention consists in **a topical or pour-on benzimidazole composition** where the benzimidazole is solubilised in an organic acid (such as lactic acid).

In yet a further aspect the present invention consists in **a benzimidazole containing composition** where at least one phase is that of an organic acid (such as lactic acid) which solubilises at least one benzimidazole active ingredient.

In another aspect the invention consists in **a ready to use pesticidal veterinary liquid composition** of at least a first active in a first liquid phase and at least a second active in a second liquid phase, the phases forming a stable emulsion.

Optionally a third active may be present in one or other or both of said phases.

Optionally such third active may be suspended in at least one phase.

Preferably each of said first and second actives are dissolved (at least in part) in their respective phases.

In another aspect the present invention consists in **a preferably storage stable pourable composition** comprising or including
up to 25% w/v of at least one pesticide (hereafter "first active(s)") soluble in an organic phase,
1 to 60% w/v of an organic phase [such as a oil (vegetable or mineral) and/or emollient esters] (hereafter "first liquid phase'') in which said first active(s) is (are) at least substantially soluble,
0 - 5% w/v of a cosolvent for said first active(s),
1 to 15% w/v of an emulsifying agent,
0 to 20% w/v of at least one further pesticidal active (hereafter "second active(s)") not substantially soluble in said first phase,
and,
means providing a second liquid phase,
said composition having at least most of said first active(s) in a first phase and said first phase being emulsified in the second phase which includes said second active(s).

In another aspect the present invention consists in **a preferably storage stable composition** (whether as a concentrate for aqueous dilution or otherwise) comprising or including
up to 25% w/v of at least one pesticide (hereafter "first active(s)") chosen from the class of at least partly oil or emollient ester soluble actives,
1 to 60% w/v of at least one water immiscible organic phase [such as a oil (vegetable or mineral) and/or emollient esters] in which said first active(s) is (are) at least substantially soluble,
0 - 5% w/v of a cosolvent for said first active(s),
1 to 15% w/v of an emulsifying agent,
0 to 20% w/v of at least one further pesticidal active (hereafter "second active(s)") not substantially soluble in said organic phase which is (i) dissolved in said water and/or (ii) suspended in said water,
and,
water,
said composition having an organic phase with at least most of said first active(s), said organic phase being emulsified in an aqueous phase of said water and said second active(s).

Preferably is one embodiment said first and second active are anthelmintics.

Preferably said organic phase at least includes an oil.

Preferably additional active ingredients are included in said composition whether in said organic phase or said aqueous phase or a mixture of them both.

In one preferred form of the present invention an additional active ingredient may be included in such a way that the composition is a suspo-emulsion.

In another aspect the present invention consists in **a storage stable pesticidal composition** having an organic phase and an aqueous phase, said organic phase being of an oil and/or emollient ester which includes at least one active ingredient (and optionally a co-solvent for said active ingredient) and an aqueous phase including a second active ingredient which is substantially insoluble in said organic phase **wherein** the presence of an emulsifying agent and/or anti-flocculants ensures stability of the two phases with the organic phase as an emulsion within said aqueous phase.

Preferably said composition includes still further actives which as particles, liposomes or as liquid globules are suspended in one or other or both of said phases. For instance preferably said first active may be a broad spectrum anthelmintic such as an ML anthelmintic (eg; abamectin) and said second active is a water soluble active also capable of killing nematodes such as levamisole. An additional active or additional actives may include one or more of suitable actives dissolvable or suspendible or emulsifiable in one or other of the phases having, for example, an additional anthelmintic, or an active with an ectoparasitocidal effect or a flukicidal effect.

Equally this may contain two or more actives that have activity against trematodes such as Flukes or Cestodes, or Ectoparasites.

In another aspect the present invention consists in **a pourable pesticidal composition** comprising or including
at least one active ingredient chosen from the class of macro cyclic lactones (hereafter "ML"),
at least one active ingredient chosen from the tetramisole/levamisole class,
at least one organic liquid carrier (preferably at least one oil and, optionally, at least one organic co-solvent) to provide a first liquid phase, and
means providing a second liquid phase,
and, optionally, an emulsifying agent or agents,
**wherein** said ML active ingredient(s) is(are) at least primarily in the first phase in solution,
**and wherein** said tetramisole/levamisole class active ingredient(s) is(are) at least primarily in solution in the second phase,
**and wherein** said phases exist in, or can be shaken or agitated into, the form of an emulsion.

In another aspect the present invention consists in **a pesticidal composition** (whether as a concentrate for aqueous dilution or otherwise) comprising or including
at least one active ingredient chosen from the class of macro cyclic lactones (hereafter "ML"),
at least one active ingredient chosen from the tetramisole/levamisole class,
at least one organic liquid carrier (preferably at least one oil and, optionally, at least one organic co-solvent), and
water,
and, optionally, an emulsifying agent or agents,
**wherein** said ML active ingredient(s) is(are) at least primarily in the organic liquid carrier(s) in solution (hereafter referred to as "the organic phase"),
**and wherein** said tetramisole/levamisole class active ingredient(s) is(are) at least primarily in solution in the water (hereafter referred to as "the aqueous phase"),
**and wherein** said organic phase and said aqueous phase exist in, or can be shaken into, the form of an emulsion.

Optionally a co-solvent is present. Preferably such co-solvent is selected from the class of alcohols having multiple carbons (preferably 4 or more) (eg; benzyl alcohol), diols and glycol ethers.

Preferably a third active ingredient is included in the composition.

In one form preferably said third ingredient (if an anthelmintic) is other than an ML anthelmintic and other than an anthelmintic chosen from the tetramisole/levamisole class.

Preferably said third anthelmintic agent is selected from the group including benzimidazoles and probenzimidazoles (eg; albendazole, oxfendazole, triclabendazole), praziquantel, salicylamides (eg; closantel), organophosphates, (napthalophos), clorsulon, nitroxynil, morantel, pyrantel, et al. - see Table 2 - Spectrum of Activity.

Preferably said third anthelmintic active ingredient is suspended at least primarily in the aqueous phase.

Preferably said aqueous phase includes at least one suspension agent for said third anthelmintic active ingredient.

In still other aspects the present invention consists in **a (preferably storage stable) pourable composition** comprising or including
at least one anthelmintic active (hereafter "first anthelmintic active(s)"),
at least one liquid to provide a first phase in which said first anthelmintic active(s) is (are) at least substantially soluble,
an emulsifying agent,
at least one liquid to provide a second phase, and
at least one further anthelmintic active (hereafter "second anthelmintic active(s)") not substantially soluble in said first phase,
said composition having a first phase with at least most of said first anthelmintic active(s) and a second phase of at least most of said second anthelmintic active(s).

In still other aspects the present invention consists in **a (preferably storage stable) composition** (whether as a concentrate for aqueous dilution or otherwise) comprising or including
at least one anthelmintic (hereafter "first anthelmintic active(s)") chosen from the class of at least partly oil soluble anthelmintic actives,
at least one oil (optionally also with an organic co-solvent) in which said first anthelmintic active(s) is (are) at least substantially soluble,
an emulsifying agent,
water, and
at least one further anthelmintic active (hereafter "second anthelmintic active(s)") not substantially soluble in said oil(s) which is (i) dissolved in said water and/or (ii) suspended in said water,
said composition having an organic phase of said oil(s) with at least most of said first anthelmintic active(s) and an aqueous phase of said water and at least most of said second anthelmintic active(s).

Preferably said oil is a vegetable oil or a mineral oil.

Preferably said composition includes a third active.

Preferably said third active is a therapeutic agent such as an antimicrobial and/or still further anthelmintic active (eg; flukicide) and/or an ectoparasitocide (hereinafter "third active(s)").

Preferably said third active(s) is primarily disposed within said aqueous phase although in other forms of the present invention it may be partitioned between the organic and aqueous phases or largely in the organic phase.

Preferably said second anthelmintic active(s) is dispersed substantially homogeneously as a suspension in the water.

Preferably said composition includes any one or more of the following:
- a co-solvent which is to form part of said aqueous phase, eg; as maybe preferable if the first anthelmintic active(s) is only partially soluble in the vegetable oil(s),
- mineral(s),
- vitamin(s),
- antimicrobial(s),
- anthelmintic(s),
- antifreeze(s),
- thickening agent(s),
- anti-focculant(s),
- pH stabiliser(s).

Preferably said vegetable oil(s) is selected from the group including soyabean oil, sesame seed oil, corn oil, sunflower oil, peanut oil and safflower oil.

Preferably said emulsifying agent is Tween 80 and/or Teric 380.

Preferably said second anthelmintic active is selected from the group consisting of levamisole or tetramisole (if a water phase soluble anthelmintic) or a water phase suspendible or dispersible anthelmintic such as a suitable benzimidazole typified by, for example, albendazole or oxfendazole. Alternatively said optional third active is a dispersed benzimidazole.

Preferably said cosolvent is selected from the group including high chain alcohols (such as benzyl alcohol), diols, glycol ethers, and esters (eg; PMP).

Preferably said antimicrobial is selected from the group including benzoic acid, potassium sorbate and parabens.

Preferably said minerals are selected from the group including mineral salts or chelates, eg; selenium, copper, cobalt, iodine and zinc.

Preferably said vitamins are selected from the group including vitamins A, D, E, B and C.

Preferably said antifreeze or freezes is or are selected from the group consisting of propylene glycol and glycerine.

Preferably said thickening agent are selected from the group of cellulose gum, xanthan gum, carbapol and algeinates.

Preferably said anti-flocculants are selected from the group consisting of Terasperse 2500 and 4896 (preferably both).

Preferably said pH stabilisers are selected from the group consisting of citric acid, phosphate salts, etc.

Preferably the or a said organic liquid carrier is an oil.

Preferably said oil is a vegetable oil. In other forms it is a mineral oil (eg; medium grade).

Preferably said vegetable oil is selected from a group consisting of or including soyabean oil, corn oil, sesame seed oil, sunflower oil, peanut oil and safflower oil.

In yet a further aspect the present invention is **a stable formulation** of
a first active in an organic ("first") phase,
a second active in a second liquid phase, and
additional actives in one or other, or both, said phase(s),
and wherein said phases provide a stable emulsion,
and wherein at least one (and preferably both) of said first and second actives is (are) an anthelmintic active.

Preferably a third active is a suspended active at least primarily in the second phase (eg; an anthelmintic or ectoparasiticide).

Preferably an emulsifying agent assists in the stability of the emulsion.

In yet a further aspect the present invention is **a stable formulation** of
a first active in an organic phase at least primarily of oil(s),
a second active in an aqueous phase, and
additional actives in one or other, or both, said organic and aqueous phase(s),
and wherein said organic and aqueous phases provide a stable emulsion,
and wherein at least one (and preferably both) of said first and second actives is (are) an anthelmintic active.

Preferably a third active is a suspended active at least primarily in the aqueous phase (eg; an anthelmintic or ectoparasiticide).

Preferably an emulsifying agent assists in the stability of the emulsion.

In another aspect the invention consists in **a storage stable and pourable pesticidal composition** comprising or including
(i) at least one active ingredient chosen from the class of macro cyclic lactones (hereafter "ML"),
(ii) at least one organic liquid carrier which carries at least most of said ML active ingredient(s), thereby defining an organic liquid phase,
(iii) levamisole, and
(iv) at least water which carries at least most of said levamisole thereby defining an aqueous phase,
**wherein** said aqueous phase has a pH of less than 7,
**and wherein** there is present in said aqueous phase either or both
(a) an emulsifying agent or agents, and
(b) a particulate content,
**and wherein** said phases exist in, or can be shaken or agitated into, the form of an emulsion with said particulate content, if any, at least substantially present in the aqueous phase.

In yet another aspect the invention is **an anthelmintic oil in water emulsion** carrying at least one macro cyclic lactone (ML) in the oil phase and particles of levamisole and an emulsifying agent for the levamisole in the aqueous phase.

In still another aspect the invention is **a partitioned biocidal composition** comprising in an organic phase a first biocide, and in an aqueous phase at acid pH a second biocide, said first biocide being unstable chemically at said acid pH.

Preferably said composition has particles suspended in at least the aqueous phase.

Preferably said particles are themselves biocidal.

Preferably said particles are of a benzimidazole anthelmintic.

In a further aspect the invention consists in **a method of formulating an anthelmintic composition** of a kind having
at least one anthelmintic (hereafter "first active(s)"),
a liquid or liquids to define a first phase in which said first active(s) is (are) at least substantially soluble,
(optionally) an emulsifying agent,
at least one further anthelmintic active (hereafter "second active(s)") not substantially soluble in said first phase
(optionally) anti-flocculant(s),
and,
a liquid or liquids to define a second phase,
said method comprising or including the steps of
(I)
   (a) providing a mix of said first active ingredient and at least the first phase liquid(s),
   (b) providing a mix of said second active ingredient and at least the second phase liquid(s), and
(II) by mixing at least the mixes of (I)(a) and (I)(b) forming an emulsion with at least most of said first active in the first phase and at least most of the second active in the second phase.

In a further aspect the invention consists in **a method of formulating an anthelmintic composition** of a kind having
at least one anthelmintic (hereafter "first active(s)") chosen from the class of (at least partly) oil soluble anthelmintic actives,
an oil or oils in which said first active(s) is (are) at least substantially soluble,
optionally a cosolvent for said first active(s),
an emulsifying agent,
at least one further anthelmintic active (hereafter "second active(s)") not substantially soluble in said oil(s),
optional anti-flocculant(s),
and,
water,
said method comprising or including the steps of
(I)
   (a) providing a mix of said first active ingredient, the oil(s), the optional co-solvent(s) and the emulsifying agent(s),
   (b) providing a mix of said second active ingredient the water, and the optional anti-flocculant(s), and
(II) by mixing at least the mixes of (I)(a) and (I)(b) forming an emulsion with at least most of said first active in the oil(s) and at least most of the second active in the aqueous phase.

In yet a further aspect the invention consists in **an anthelmintic composition** so made.

In still other aspects the invention is **a method of treating mammals for pests** which involves (whether with dilution or not) administering or having self administered to such mammals effective amounts of active(s) of compositions of the present invention.

In still a further aspect the invention consists in **the use of an anthelmintic composition** of any of the kinds previously defined.

In some forms said use is as an oral, a pour-on or as a parenteral composition (preferably with an anthelminticly effective amount of each active).

Preferred formulations of the present invention comprise:
**Component 1** - water insoluble but mostly or completely oil soluble anthelmintic active.
**Component 2** - An oil (vegetable or mineral)
**Component 3 -** an emulsifying agent
**Component 4 -** water
**Component 5A, 5B, etc. -** Additional active(s) of which at least one is preferably an anthelmintic active. This or these can be either dissolved in the water phase (such as Levamisole) or suspended in the water phase (such as Albendazole) if insoluble, or both (eg; Levamisole and Albendazole).

The water insoluble active is dissolved in the oil, which is emulsified in water. The oil protects the active against the pH or constituents present in the water phase.

Additional components can be added to this basic formulation.
**Addition 1 -** if component 1 is only partially soluble in the oil phase then a co-solvent may be necessary (eg; an organic co-solvent).
**Addition 2 -** Minerals/Vitamins
**Addition 3 -** An antimicrobial.
**Addition 4 -** Antifreezes.
**Addition 5 -** Thickening agents.
**Addition 6 -** Anti-flocculants.
**Addition 7 -** pH stabilisers.

Preferred components and their concentration ranges and examples are set out in **Table 1:**

**Table 1**

| | **Function** | **Examples** | **% W/V** |
|---|---|---|---|
| Component 1 | Active | Abamectin or ivermectin | 0 - 25% |
| Component 2 | Vegetable Oil Mineral Oil | Soya bean Medium Grade | 1 - 60% 1 - 60% |
| Component 3 | Emulsifying Agent | Tween 80 Teric 380 | 1-15% |
| Component 4 | Water | | To Volume |
| Component 5A, 5B, etc. | Additional anthelmintic | | |
| | Water soluble | Levamisole | 0 - 15% |
| | Water Insoluble | Albendazole or oxfendazole | 0 - 20% |
| Addition 1 | Co-Solvent | Includes high chain Alcohols (such as Benzyl Alcohol), diols such as PMP (promyristyl propionate), Glycol Ethers and Esters | 0 - 5% |
| Addition 2 | Minerals | Mineral salts or chelates | 0 - 5% |
| Addition 3 | Antimicrobials | Benzoic Acid, Potassium Sorbate and Parabens, Benzyl Alcohol | 0-1% |
| Addition 4 | Antifreeze | Propylene Glycol, Glycerine | 0 - 5% |
| Addition 5 | Thickening Agents | Cellulose Gum, Xanthan Gum, Carbapol and Algeinates | 0 - 3% |
| Addition 6 | Anti-flocculants | Terasperse 2500 and 4896 | 0 - 7% |
| Addition 7 | pH Stabilisers | Citric Acid, Phosphate salts etc. | 0 -05% |

Preferred actives by reference to activity are now tabulated.

**Table 2 -**

| **Spectrum of Activity** | | | | | | | | | |
|---|---|---|---|---|---|---|---|---|---|
| Compound | Nematodes | | Cestodes | Trematodes | Protozoa | Ectoparasites | | | |
| | Gastrointestinal Worms | Lung-worms | Tapeworms | Fluke | | Lice | Mites | Ticks | Flies |
| **A. Macrocyclic Lactones** | | | | | | | | | |
| *1. Avermectins* | | | | | | | | | |
| Ivermectin | + | + | - | - | | + | + | + | + |
| Abamectin | + | + | - | - | | + | + | + | + |
| Doramectin | + | + | - | - | | + | + | + | + |
| Eprinomectin | + | + | | | | + | + | + | + |
| *2. Milbemycin* | | | | | | | | | |
| Moxidectin | + | + | - | - | | + | + | + | + |
| **B. Benzimidazoles** | | | | | | | | | |
| *1. Probenzimidazoles* | | | | | | | | | |
| Febantel | + | + | | | | | | | |
| Netobimin | + | + | | | | | | | |
| *2. Benzimidazoles* (*thiazoles*) | | | | | | | | | |
| Thiabendazole | + | + | | | | | | | |
| Cambendazole | + | + | | | | | | | |
| *3. Benzimidazoles* (*carbamates*) | | | | | | | | | |
| Albendazole | + | + | + | + | +(Giardia) | - | - | - | - |
| Fendendazole | + | + | | + | +(Giardia) | - | - | - | - |
| Mebendazole | + | + | | | | | - | | - |
| Rycobendazole | + | + | + | + | | - | - | | |
| Oxfendazole | + | + | + | + | | - | - | | |
| Parrbendazole | + | + | | - | | | | | |
| Triclabendazole | - | - | - | + | | - | - | | - |
| | | | | | | | | | |
| **C. Imidazothiazole** | | | | | | | | | |
| Levamisole | + | + | | | | | | | |
| | | | | | | | | | |
| **D. Tetrahydropyrimidazole** | | | | | | | | | |
| Morantel | + | | + | | | | | | |
| Pyrantel | + | | | | | | | | |
| | | | | | | | | | |
| **E. Salicylamidides** | | | | | | | | | |
| Closantel | +(Haemonchus) | - | - | + | | | | | + |
| Oxydosanide | +(Haemonchus) | | | + | | | | | |
| Rafoxamide | +(Haemonchus) | | | | | | | | |
| Niclosamide | | + | | | | | | | |
| | | | | | | | | | |
| **F. Benzoenedisuplhonamide** | | | | | | | | | |
| Clorsulon | - | - | - | + | | | | | |
| | | | | | | | | | |
| **G. Nitrophenolic Compounds** | | | | | | | | | |
| Nitroxyinil | +(Haemonchus) | - | - | + | | | | | + |
| | | | | | | | | | |
| **H**. **Pyrazinoisoquinoline** | | | | | | | | | |
| Praziquantel | - | - | + | | | | | | |
| | | | | | | | | | |
| **I. Organophosphates** | | | | | | | | | |
| Napthalophos | + | | | | | | | | |
| Trichlorphon | + | | | | | | | | + |
| Propetamphos | | | | | | | | | + |
| Diazinon | | | | | | + | + | | + |
| *Cumaphos* | | | | | | + | + | + | + |
| | | | | | | | | | |
| **J. Synthetic Pyrethroids** | | | | | | | | | |
| Cypermethrin | | | | | | + | | | + |
| Alphaniethrin | | | | | | + | | | + |
| Flumethrin | | | | | | | | + | |
| **K. Insect Growth Regulators** | | | | | | | | | |
| Diflubenzuron | | | | | | + | | | + |
| Triflumuron | | | | | | + | | | |
| Cyromazine | | | | | | | | | + |
| | | | | | | | | | |
| **L. Amitraz** | | | | | | | | + | |
| | | | | | | | | | |
| **M. Cyhalothrin** | | | | | | | | + | + |
| | | | | | | | | | |
| **N. GABA Inhibitor** | | | | | | | | | |
| Fipronil | | | | | | + | + | + | + |

Preferred forms of the present invention will hereafter be described by reference to examples and the accompanying drawings in which
**Figure 1** is a plot from the cattle trial NCOO1 hereafter referred to of Avermectin B1a blood levels (ng/mL) against time (hours) for the different modes of administration,
**Figure 2** is a plot of Levamisole blood levels (µg/ml) against time (hours) for the different modes of administration in cattle,
**Figure 3** is a plot of Oxfendazole blood levels (µg/ml) against time (hours) for the different modes of administration in cattle,
**Figure 4** is a plot of Fenbendazole blood levels (µg/ml) against time (hours) for the different modes of administration in cattle,
**Figure 5** is a plot of Avermectin B1a blood level (ng/mL) against time (hours) for the oral combination against a commercial oral formulation in sheep,
**Figure 6** is a plot of Levamisole blood level (µg/ml) against time (hours) for the oral combination against a commercial oral formulation in sheep,
**Figure 7** is a plot of Albendazole blood levels (µg/ml) against time (hours) for the oral combination against a commercial oral formulation in sheep,
**Figures 8 to 10** are stability graphs for each of Abamectin, Levamisole and Albendazole against Time respectively for formulation LB98/92,
**Figure 11** is a plot of Levamisole content against Time for formulation LB98/92,
**Figure 12** is a plot of pH against Time for formulation LB98/92,
**Figure 13** shows stability results and graphs for formulation LB99/01 of Table 7,
**Figure 14** shows stability results and graphs for formulation LB99/02 of Table 8,
**Figure 15** shows stability results and graphs for formulation LB99/03 of Table 9,
**Figure 16** stability graphs of pH changes with time for formulations LB99/54A, LB99/54B, LB99/54C and LB99/54D of Table 10,
**Figure 17** shows (for a 50°C accelerated stability trial) the change of pH of formulations/drenches of Tables 12 and 13 with time,
**Figure 18** shows (for a 50°C accelerated stability trial) the change of viscosity (cp) of formulations/drenches of Tables 12 and 13 with time,
**Figure 19** shows (for a 50°C accelerated stability trial) the change of mean droplet/particle size of formulations/drenches of Tables 12 and 13 with time.
**Figure 20** shows (for a 50°C accelerated stability trial) the change of moisture content of formulations/drenches of Tables 12 and 13 with time,
**Figure 21** shows (for a 50°C accelerated stability trial) the change of levamisole content of formulations/drenches of Tables 12 and 13 with time,
**Figure 22** shows (for a 50°C accelerated stability trial) the change ofpraziquantel content of formulations/drenches of Tables 12 and 13 with time,
**Figure 23** shows (for a 50°C accelerated stability trial) the change of albendazole content of formulations/drenches of Tables 12 and 13 with time,
**Figure 24** shows (for a 50°C accelerated stability trial) the change of ivermectin content of formulations/drenches of Tables 12 and 13 with time,
**Figure 25** shows (for a 50°C accelerated stability trial) the change of abamectin content of formulation/drenches of Tables 12 and 13 with time,
**Figure 26** shows (for a 50°C accelerated stability trial) the change of benzyl alcohol content of formulations/drenches of Tables 12 and 13 with time,
**Figure 27** shows (for a 50°C accelerated stability trial) the change of selenium content of formulations/drenches of Tables 12 and 13 with time,
**Figure 28** shows (for a 50°C accelerated stability trial) the change of cobalt content of formulations/drenches of Tables 12 and 13 with time,
**Figure 29** shows the positive effect against clumping/aggregation from the presence of particles of Levamisole hydrochloride, the unclumped dish to the right being that (LB99/96C of Table 14) with Levamisole, the others (LB99/96A and LB99/96B of Table 14) having no such presence and clumping,
**Figure 30** shows the results of freeze/thaw trials (mLs separation - 200mL samples being cycled between minus 4°C and ambient for 4 days) for formulations/drenches of Table 15, and
**Figure 31** shows a photograph of phase separation(A) after freeze thawing compared with a stable homogeneous formulation(B) of the invention.

In the following examples all percentages are weight to volume. These examples show various formulations directed to a resistance strategy which nevertheless are stable and may have additional therapeutic and/or active inclusions.

### EXAMPLE 1

| **Combination Abamectin/Levamisole Drench** | |
|---|---|
| Abamectin | 0.20 % w/v |
| Tween 80 | 8.00 % w/v |
| Benzyl Alcohol | 3.00 % w/v |
| Propylene Glycol | 20.00 % w/v |
| Na₂HPO₄ | 1.03 % w/v |
| Citric Acid | 0.29 % w/v |
| Levamisole HCL | 8.00 % w/v |
| Sodium Selenate | 0.24 % w/v |
| Water | To Volume % w/v |

This was formulated as follows at ambient temperature.
- Mix 1 - Dissolved Abamectin in Benzyl Alcohol, mixed in Tween and Propylene Glycol.
- Mix 2 - Dissolved Levamisole, Na₂HPO₄, Citric acid and Sodium Selenate in water.
- Combined mix 1 and 2.
This resultant formulation was physically stable but considered too thin.
To correct this the following Examples 2-4 were thickened with Carbopol or gums.

### EXAMPLE 2

| **Combination Abamectin/Levamisole Drench** | |
|---|---|
| Abamectin | 0.20 % w/v |
| Tween 80 | 8.00 % w/v |
| Benzyl Alcohol | 3.00 % w/v |
| Propylene Glycol | 20.00 % w/v |
| Na₂HPO₄ | 1.03 % w/v |
| Citric Acid | 0.29 % w/v |
| Levamisole HCL | 8.00 % w/v |
| Sodium Selenate | 0.24 % w/v |
| Cellulose Gum CMC | 0.50 % w/v |
| Water | To Volume % w/v |

This was formulated as follows;
- Mix 1 - Dissolved Abamectin in Benzyl Alcohol then add Tween.
- Mix 2 - Dissolved Levamisole, Na₂HPO₄, Citric acid and Sodium Selenate in water.
- Mix 3 - Dispersed thickener in Propylene glycol.
- Combined Mix 2 and 3 then added Mix 1.

### EXAMPLE 3

| **Combination Abamectin/Levamisole Drench** | |
|---|---|
| Abamectin | 0.20 % w/v |
| Tween 80 | 8.00 % w/v |
| Benzyl Alcohol | 3.00 % w/v |
| Propylene Glycol | 20.00 % w/v |
| Na₂HPO₄ | 1.03 % w/v |
| Citric Acid | 0.29 % w/v |
| Levamisole HCL | 8.00 % w/v |
| Sodium Selenate | 0.24 % w/v |
| Carbopol 934 | 0.50 % w/v |
| Water | To Volume % w/v |

### EXAMPLE 4

| **Combination Abamectin/Levamisole Drench** | |
|---|---|
| Abamectin | 0.20 % w/v |
| Tween 80 | 8.00 % w/v |
| Benzyl Alcohol | 3.00 % w/v |
| Propylene Glycol | 20.00 % w/v |
| Na₂HPO₄ | 1.03 % w/v |
| Citric Acid | 0.29 % w/v |
| Levamisole HCL | 8.00 % w/v |
| Sodium Selenate | 0.24 % w/v |
| Xanthan Gum | 0.20% w/v |
| Water | To Volume % w/v |

The low pH of these formulations 2, 3 and 4 (pH<4) was identified as unsuitable for the long-term stability of Abamectin. It was decided to use a vegetable oil to attempt to encapsulate the Abamectin and possibly protect it from the low pH of the water phase. An additional active, albendazole, a benzimidazole anthelmintic, was added to the formulation.

### EXAMPLE 5

| **Combination Abamectin/Albendazole/Levamisole Drench** | |
|---|---|
| Abamectin | 0.10% w/v |
| Albendazole | 2.50% w/v |
| Benzyl Alcohol | 3.00% w/v |
| Teric 215 | 10.0 % w/v |
| Teric 216 | 10.0 % w/v |
| Propylene Glycol | 3.00% w/v |
| Na₂HPO₄ | 1.05% w/v |
| Citric Acid | 1.21% w/v |
| Levamisole HCL | 3.75% w/v |
| Sodium Selenate | 0.12% w/v |
| Cobalt EDTA | 0.36% w/v |
| Soyabean Oil | 3.00% w/v |
| Xanthan gum | 0.32% w/v |
| Water | To Volume % w/v |

This was formulated as follows:
- Mix 1 - Dissolve Abamectin in Benzyl Alcohol. Mix in Terics.
- Mix 2 - Dissolve Levamisole, Cobalt-ETDA, Citric acid, Na₂HPO₄ and Sodium Selenate in water.
- Mix 3 - Disperse Xanthan gum in Propylene glycol then add 5% of water to form gel.
- Combine Mix 1 and 2. Added Albendazole. Then added Mix 1.

This formulation after 2 months accelerated stability assays shows that the Abamectin was degrading. The physical stability of this formulation was also poor with Albendazole flocculating out and with the oil phase showing evidence of curdling.

### EXAMPLE 6

| **Combination Abamectin/Albendazole/Levamisole Drench** | |
|---|---|
| Abamectin | 0.10 % w/v |
| Albendazole | 1.90% w/v |
| Benzyl Alcohol | 3.00 % w/v |
| Teric 215 | 10.0 % w/v |
| Teric 216 | 10.0 % w/v |
| Propylene Glycol | 3.00 % w/v |
| Na₂HPO₄ | 1.05 % w/v |
| Citric Acid | 1.21 % w/v |
| Levamisole HCL | 3.75 % w/v |
| Sodium Selenate | 0.12 % w/v |
| Cobalt EDTA | 0.36 % w/v |
| Soyabean Oil | 3.00 % w/v |
| Xanthan gum | 0.32 % w/v |
| Water | To Volume % w/v |

This formulation like that of Example 6 after 2 month accelerated stability assays also showed that Abamectin was degrading. The physical stability of this formulation was also poor with Albendazole flocculating out and with the oil phase showing evidence of curdling.

To prevent such flocculation Terasperse 4896 and Terasperse 2500 were then trialed. These theoretically coat the Albendazole and improve the solubility in water*.*

Teric 380, a more appropriate emulsifying agent for stable vegetable oil emulsions.

The percentage of the oil phase was increased to 10% to increase the partition between the oil/water phase possibly improve Abamectin stability.

### EXAMPLE 7

| **Combination Abamectin/Albeadazole/Levamisole Drench** | |
|---|---|
| Abamectin | 0.10 % w/v |
| Albendazole | 1.90 % w/v |
| Benzyl Alcohol | 3.00 % w/v |
| Terasperse 4896 | 1.00 % w/v |
| Terasperse 2500 | 2.00 % w/v |
| Teric 380 | 5.00 % w/v |
| Propylene Glycol | 3.00 % w/v |
| Levamisole HCl | 4.00 % w/v |
| Sodium Selenate | 0.12 % w/v |
| Cobalt EDTA | 1.50 % w/v |
| Soyabean Oil | 10.00 % w/v |
| Xanthan gum | 0.20 % w/v |
| Water | To Volume % w/v |

This was formulated as follows:
- Mix 1 - Dissolved Abamectin in Benzyl Alcohol. Mix into soyabean oil. Mix in Teric 380.
- Mix 2 - Added Albendazole and Terasperses to water. Added Levamisole, Cobalt-ETDA. Citric acid and Sodium Selenate.
- Mix 3 - Dispersed Xanthan gum in Propylene glycol then added 5% of water to form gel.
- Combined Mix 1, 2 and 3.
   This formulation gave no flocculation but the oil phase readily separated out.
   The oil/water phase required further stabilisation. A series of water/oil/emulsifier blends were prepared to optimise this aspect of the formulation. Tween 80 was also trialed as a possible alternative emulsifier to Teric 380. All formulations were stored at ambient.

### EXAMPLE 8

| **Oil/Water Blends** | |
|---|---|
| Tween 80 | 10.0 % w/v |
| Soyabean Oil | 10.0 % w/v |
| Water | To Volume % w/v |

This was prepared as a simple mixture but the emulsion broke after 4 hours.

### EXAMPLE 9

| **Oil/Water Blends** | |
|---|---|
| Teric 380 | 5.0 % w/v |
| Soyabean Oil | 10.0 % w/v |
| Water | 85.0 % w/v |

This straight blend broke after 2 days.

### EXAMPLE 10

| **Oil/Water Blends** | |
|---|---|
| Tween 80 | 10.0 % w/v |
| Soyabean Oil | 50.0 % w/v |
| Water | 40.0 % w/v |

This straight blend emulsion broke after 4 days.

### EXAMPLE 11

| **Oil/Water Blends** | |
|---|---|
| Teric 380 | 5.0 % w/v |
| Soyabean Oil | 50.0 % w/v |
| Water | 45.0 % w/v |

This straight blend emulsion broke after 25 days.

As a result we concluded that
- Teric 380 was the better emulsifier, and
- the higher concentration of oil emulsion was the most stable option. Therefore we decided to trial with oil at from 35 to 60% w/v.

### EXAMPLE 12

| **40% Soyabean Oil** | |
|---|---|
| Abamectin | 0.10 % w/v |
| Albendazole | 1.90 % w/v |
| Benzyl Alcohol | 3.00 % w/v |
| Terasperse 4896 | 1.00 % w/v |
| Terasperse 2500 | 2.00 % w/v |
| Teric 380 | 5.00 % w/v |
| Propylene Glycol | 3.00 % w/v |
| Levamisole HCl | 4.00 % w/v |
| Sodium Selenate | 0.12 % w/v |
| Cobalt EDTA | 1.50 % w/v |
| Soyabean Oil | 40.00 % w/v |
| Xanthan gum | 0.30 % w/v |
| Water | To Volume % w/v |

This was formulated as follows at ambient temperature.
- Mix 1 - Dissolved Abamectin in Benzyl Alcohol. Mix into Soyabean oil. Mix in Teric 380.
- Mix 2 - Added albendazole and Terasperses to water. Added levamisole, Cobalt-ETDA, Citric acid and Sodium Selenate.
- Mix 3 - Dispersed Xanthan gum in Propylene glycol then added 5% of water to form gel. Combined Mix 1, 2 and 3.

With this formulation we found no flocculation occurred or separation of the oil was observed. This batch has remained stable after being recycling between 4°C, ambient and 30°C for 6 months.

Although formulation of Example 12 was showing indications of suitability another formulation option was tested. Xanthan Gum was replaced with Alginate as a alternative thickener.

### EXAMPLE 13

| **Combination Abamectin/Albendazole/Levamisole Drench with alginates** | |
|---|---|
| Abamectin | 0.10 % w/v |
| Albendazole | 1.90 % w/v |
| Benzyl Alcohol | 3.00 % w/v |
| Terasperse 4896 | 1.00 % w/v |
| Terasperse 2500 | 2.00 % w/v |
| Teric 380 | 5.00 % w/v |
| Propylene Glycol | 3.00 % w/v |
| Levamisole HCL | 4.00 % w/v |
| Sodium Selenate | 0.12 % w/v |
| Cobalt EDTA | 1.50 % w/v |
| Soyabean Oil | 10.00 % w/v |
| Alginate (Kelcolid) | 0.20 % w/v |
| Water | To Volume % w/v |

### Formulating Order:

- Mix 1 - Dissolved Abamectin in Benzyl Alcohol. Mix into soyabean oil. Mix in Teric 380.
- Mix 2 - Added Albendazole and Terasperses to water. Added Levamisole, Cobalt-ETDA, Citric acid and Sodium Selenate.
- Mix 3 - Dispersed Alginate in Propylene glycol then added 5% of water to form gel.
- Combined Mix 1, 2 and 3.

### Findings:

- Product appeared stable but a rapid viscosity drop with time was observed. This was followed by eventual slight separation of the water phase in long term stability samples.

### EXAMPLE 14 (Batch No. LB99/01)

| **Increasing Benzimidazole (Albendazole) Loading** | |
|---|---|
| Abamectin | 0.10 % w/v |
| Albendazole | 2.38 % w/v |
| Benzyl Alcohol | 3.00 % w/v |
| Terasperse 4896 | 1.00 % w/v |
| Terasperse 2500 | 2.00 % w/v |
| Teric 380 | 5.00 % w/v |
| Propylene Glycol | 3.00 % w/v |
| Levamisole HCL | 3.75 % w/v |
| Soyabean Oil | 40.00 % w/v |
| Xanthan gum | 0.30 % w/v |
| Water | To Volume % w/v |

### Formulating Order:

- Mix 1 - Dissolved Abamectin in Benzyl Alcohol. Mix into soyabean oil. Mix in Teric 380.
- Mix 2 - Added Albendazole and Terasperses to water. Added Levamisole and Citric Acid.
- Mix 3 - Dispersed Xanthan gum in Propylene glycol then added 5% of water to form gel.
- Combined Mix 1, 2 and 3.

### Findings:

- Product stable under an accelerated stability programme.

The robustness of this formulation concept was examined by substituting Albendazole with Oxfendazole and by increasing active loading.

### EXAMPLE 15 (Batch No. LB99/03)

| **Substituting Albendazole with Oxfendazole** | |
|---|---|
| Abamectin | 0.10 % w/v |
| Oxfendazole | 2.26 % w/v |
| Benzyl Alcohol | 3.00 % w/v |
| Terasperse 4896 | 1.00 % w/v |
| Terasperse 2500 | 2.00 % w/v |
| Teric 380 | 5.00 % w/v |
| Propylene Glycol | 3.00 % w/v |
| Levamisole HCL | 3.75 % w/v |
| Soyabean Oil | 40.00 % w/v |
| Xanthan gum | 0.30 % w/v |
| Water | To Volume % w/v |

Formulated similarly to Example 14.

### Findings:

- Product stable under an accelerated stability programme.

### EXAMPLE 16

| **Increasing Albendazole Loading** | |
|---|---|
| Abamectin | 0.10 % w/v |
| Albendazole | 5.00 % w/v |
| Benzyl Alcohol | 3.00 % w/v |
| Terasperse 4896 | 2.00 % w/v |
| Terasperse 2500 | 4.00 % w/v |
| Teric 380 | 5.00 % w/v |
| Propylene Glycol | 3.00 % w/v |
| Levamisole HCL | 3.75 % w/v |
| Soyabean Oil | 35.00 % w/v |
| Xanthan gum | 0.30 % w/v |
| Water | To Volume % w/v |

Prepared similarly to Example 14.

### Findings:

- Product stable under an accelerated stability programme.

### EXAMPLE 17

| **Benzimidazole Active in Lactic Acid** | |
|---|---|
| Carnola Oil | 40.00 % w/v |
| Teric 380 | 5.00 % w/v |
| Abamectin | 0.10% w/v |
| Promyristyl Propionate | 3.00 % w/v |
| Oxfendazole | 4.00 % w/v |
| Levamisole HC1 | 3.75% w/v |
| Lactic Acid | To volume |

### Formulated Order:

- Mix 1 - Dissolve Abamectin in warmed emollient Ester (Promyristyl Propionate) then mix into Oil. Mix in Teric 380.
- Mix 2 - To Lactic acid dissolve Oxfendazole and Levamisole (slight heat required).
- Mix 3 - Combine mixes 1 and 2 with high shear agitation

### Findings:

- These soluble forms of benzimidazoles are likely to have advantage (more easily dermally absorbed) as pour-on formulations. May include a thickener.

### EXAMPLE 18

| **Benzimidazole Active in Lactic Acid** | |
|---|---|
| Carnola Oil | 40.00 % w/v |
| Teric 380 | 5.00 % w/v |
| Abamectin | 0.10% w/v |
| Promyristyl Propionate | 3.00 % w/v |
| Triclabendazole | 0.50% w/v |
| Oxfendazole | 4.00% w/v |
| Levamisole HC1 | 3.75% w/v |
| Lactic Acid | To Volume |

### Formulated Order:

- Mix 1 - Dissolve Abamectin in warmed emollient Ester (Promyristyl Propionate) then mix into Oil. Mix in Teric 380.
- Mix 2 - To Lactic acid dissolve Oxfendazole or Levamisole and Triclabendazole (slight heat required).
- Mix 3 - Combine mixes 1 and 2 with high shear agitation

### Findings:

- As above, likely to have application as a pour-on formulation. May include a thickener.

### STABILITY

Stability testing with formulations such as Example 7 over 19 months shows a desirability for buffering and a buffering system (citric acid/NaOH) was added. Laboratory Batch abbreviated to LB and is used synonymously with Batch Number (B/N).

### Table 3

- LB98/92 - Abamectin 0.10%, Albendazole 1.9% and Levamisole 4.0% Triple Active Drench. Stability Conditions 4°C, Ambient and 30°C.

### Formulation

| **Material** | **LB98/92** |
|---|---|
| Soybean Oil | 40.00% w/v |
| Ethoxylated Caster Oil | 5.00% w/v |
| Abamectin @90% | 0.11% w/v |
| Benzyl Alcohol | 3.00% w/v |
| Albendazole @ 100% | 1.90% w/v |
| Terasperse 4896 | 1.00% w/v |
| Terasperse 2500 | 2.00% w/v |
| Cobalt - EDTA | 1.57% w/v |
| Sodium Selenate | 0.12% w/v |
| Levamisole HCl @ 100% | 4.00% w/v |
| Propylene Glycol | 3.00% w/v |
| Xanthan Gum | 0.30% w/v |
| Citric Acid | 0.10% w/v |
| Water to volume | As required (to 100%) |

### Summary:

See Table 4 (4A, 4B and 4C) for stability results at 4°C, Ambient and 30°C.

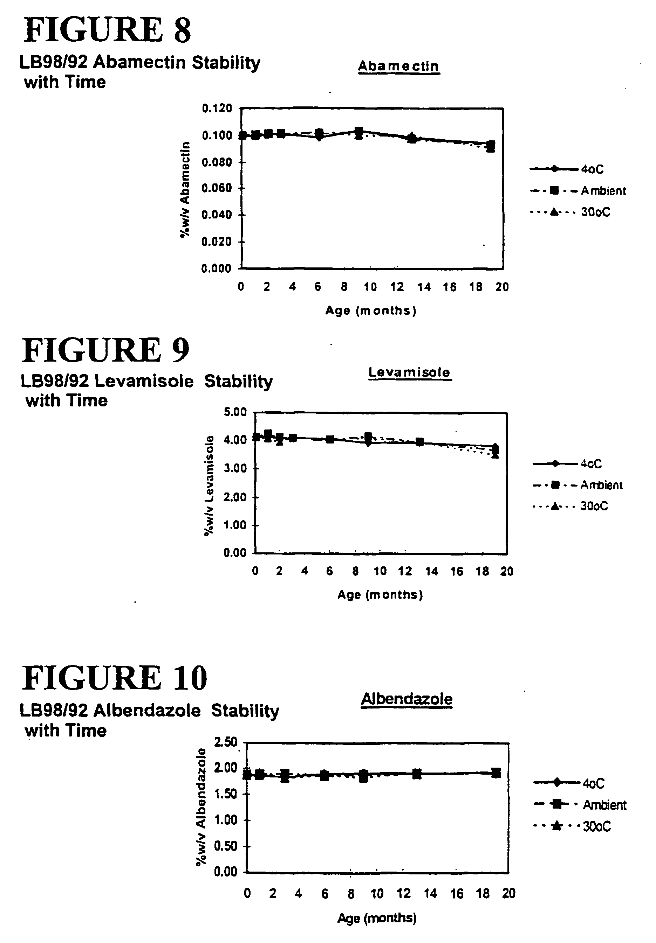

See Figures 8 to 10 for stability graphs for Abamectin, Levamisole and Albendazole respectively.

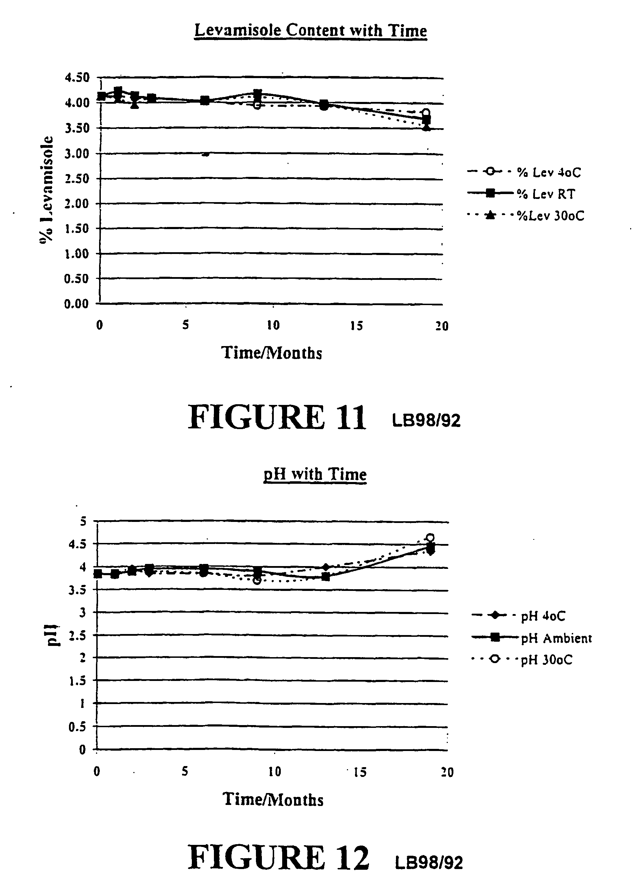

See Figures 11 and 12 for graphs with a comparison of pH (Figure 12) and Levamisole content (Figure 11).

Chemical stability appears satisfactory up until 13 month whereafter the pH drifts upwards and Levamisole contents drops. This pH drift/levamisole drop is believed related. Hence pH stabilisation is believed desirable.

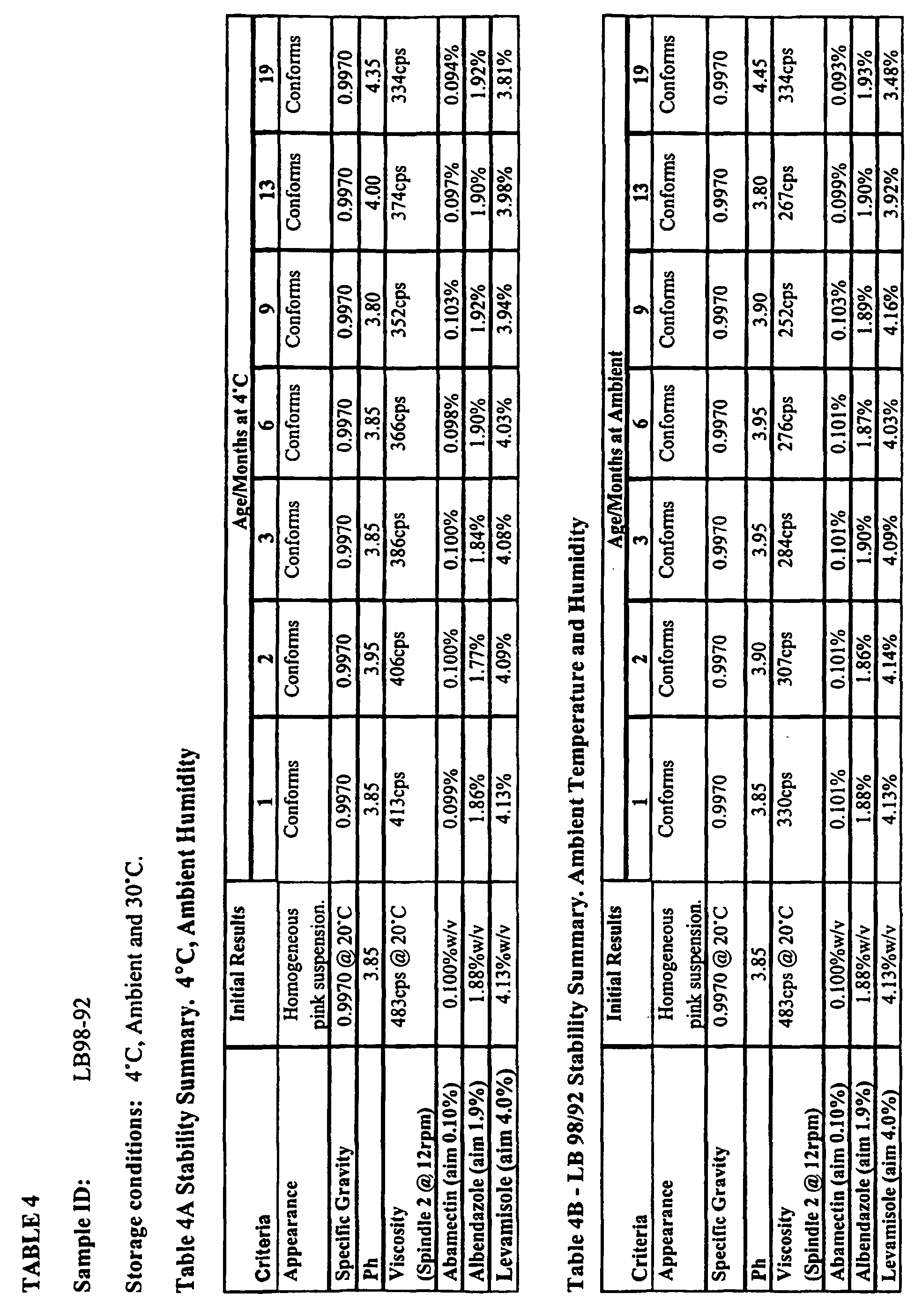

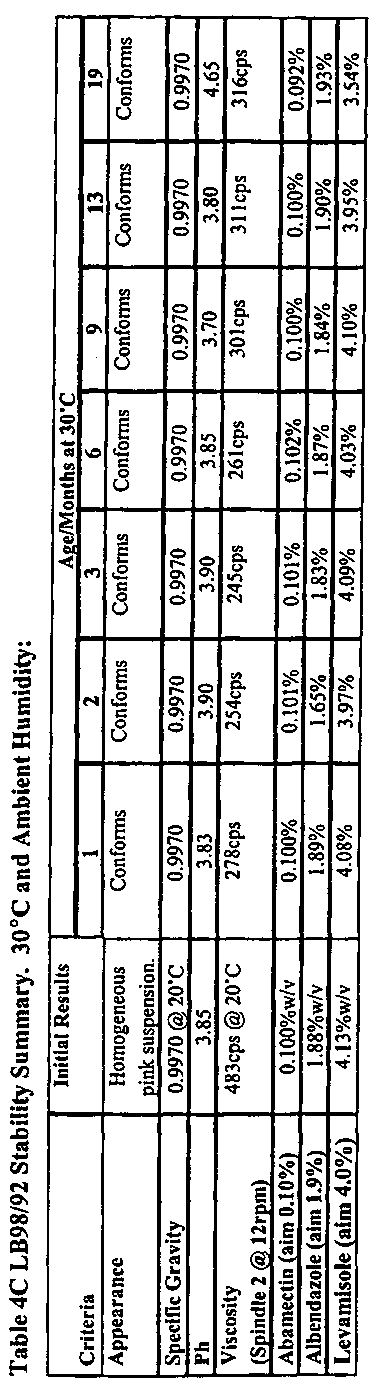

### VARIATION IN BENZIMIDAZOLE CONTENT

### Formulations

**Table 5**

| | | | |
|---|---|---|---|
| LB 99/01 | Abamectin 0.10% | Albendazole 2.38% | Levamisole 3.75% |
| LB 99/02 | Abamectin 0.10% | Albendazole 5.00% | Levamisole 3.75% |
| LB 99/03 | Abamectin 0.10% | Oxfendazole 2.26% | Levamisole 3.75% |

### Stability Conditions - Ambient and 30°C for Formulations of Table 5.

**Table 6**

| **Material** | **LB 99/01** | **LB 99/02** | **LB 99/03** |
|---|---|---|---|
| Soybean Oil | 40.00% | 35.00% | 40.00% |
| Ethoxylated Caster Oil | 5.00% | 5.00% | 5.00% |
| Abamectin @ 90% | 0.11% | 0.11% | 0.11% |
| Benzyl Alcohol | 3.00% | 3.00% | 3.00% |
| Albendazole | 2.38% | 5.00% | - - |
| Terasperse 4896 | 1.00% | 2.00% | 1.00% |
| Terasperse 2500 | 2.00% | 4.00% | 2.00% |
| Oxfendazole @ 100% | - - | - - | 2.26% |
| Levamisole HCl @ 100% | 3.75% | 3.75% | 3.75% |
| Propylene Glycol | 3.00% | 3.00% | 3.00% |
| Xanthan Gum | 0.30% | 0.30% | 0.30% |
| Citric Acid | 0.10% | 0.10% | 0.10% |
| Water to volume | As Required | As Required | As Required |

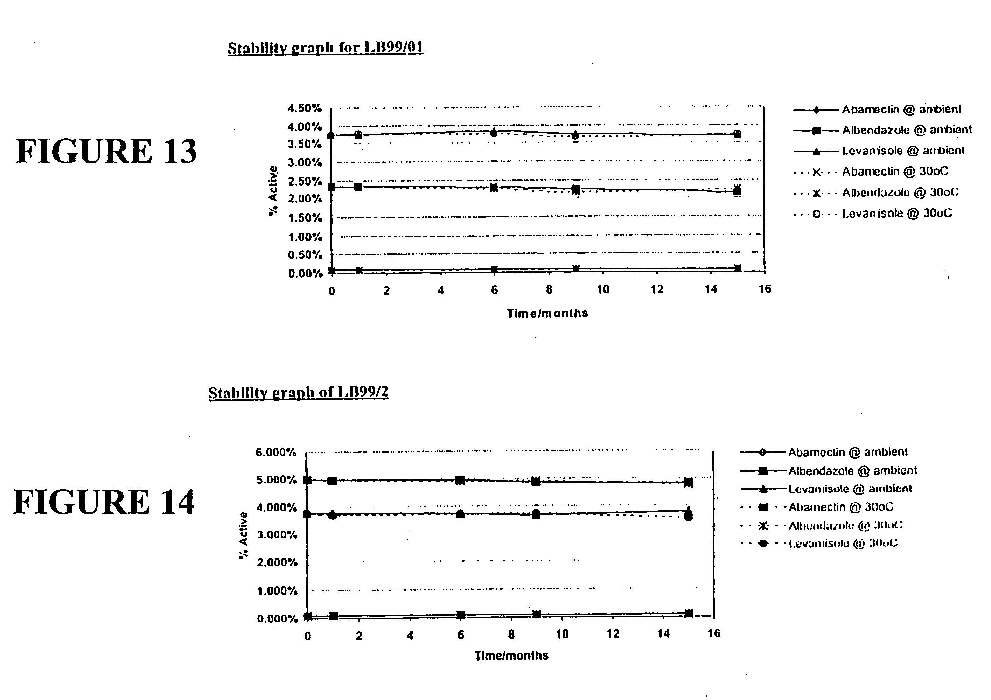

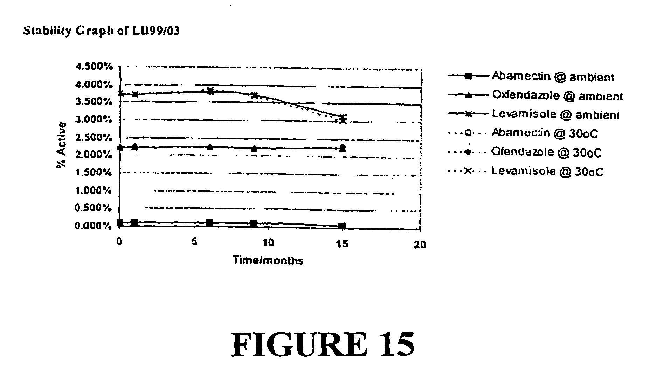

- See Figure 13 and Table 7 for stability results and graphs for LB 99/01.
- See Figure 14 and Table 8 for stability results and graphs for LB 99/02.
- See Figure 15 and Table 9 for stability results and graphs for LB 99/03.

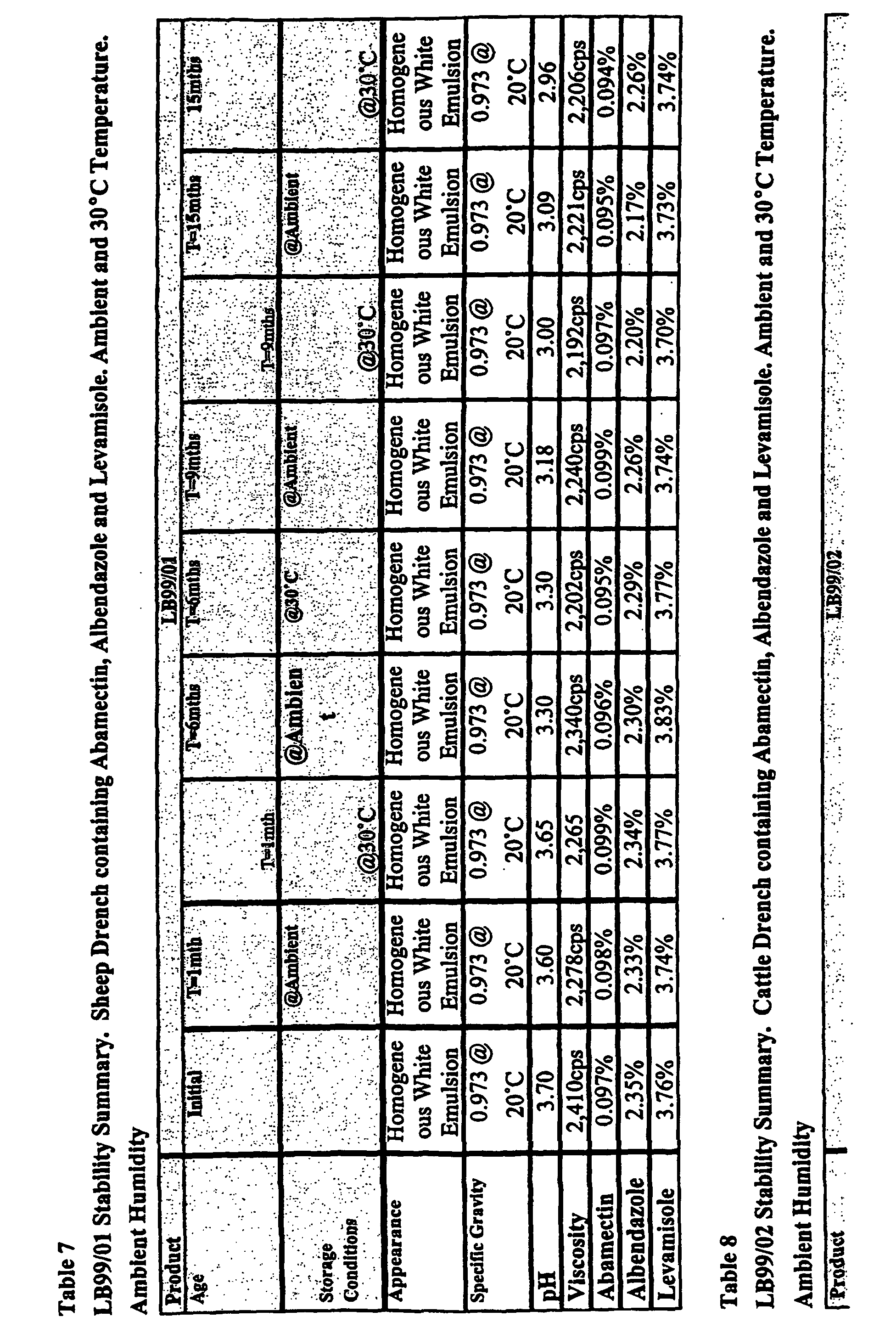

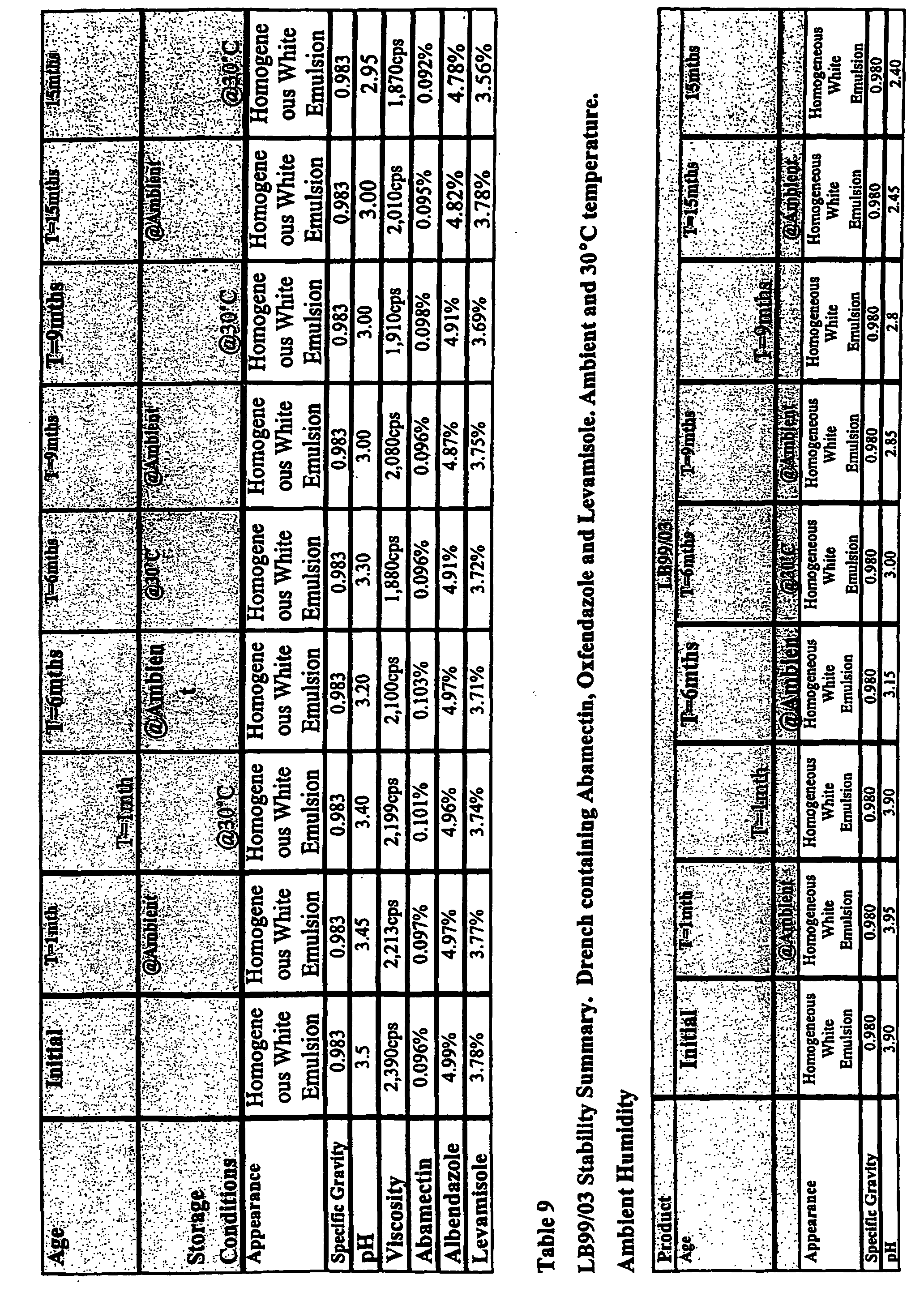

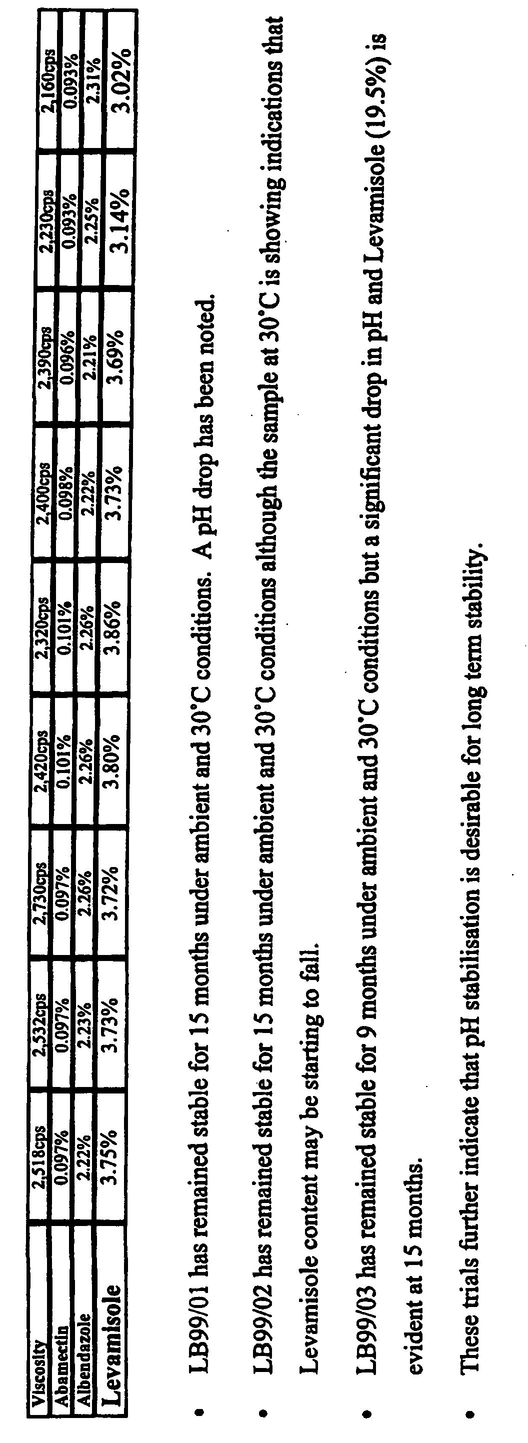

### pH STABILISATION TRIALS:

It is evident from the trends previously noted that some form of pH stabilisation is desirable (if not necessary).

A series of accelerated trials was carried out on a Triclabendazole, Abamectin and Levamisole formulation to study the effects of;
1) Chelated Minerals on pH stability.
2) Citrate Acid/Sodium Citrate buffer system on pH stability.

The trial formulations were stored at ambient and 40°C and pH tested regularly for 3 months.

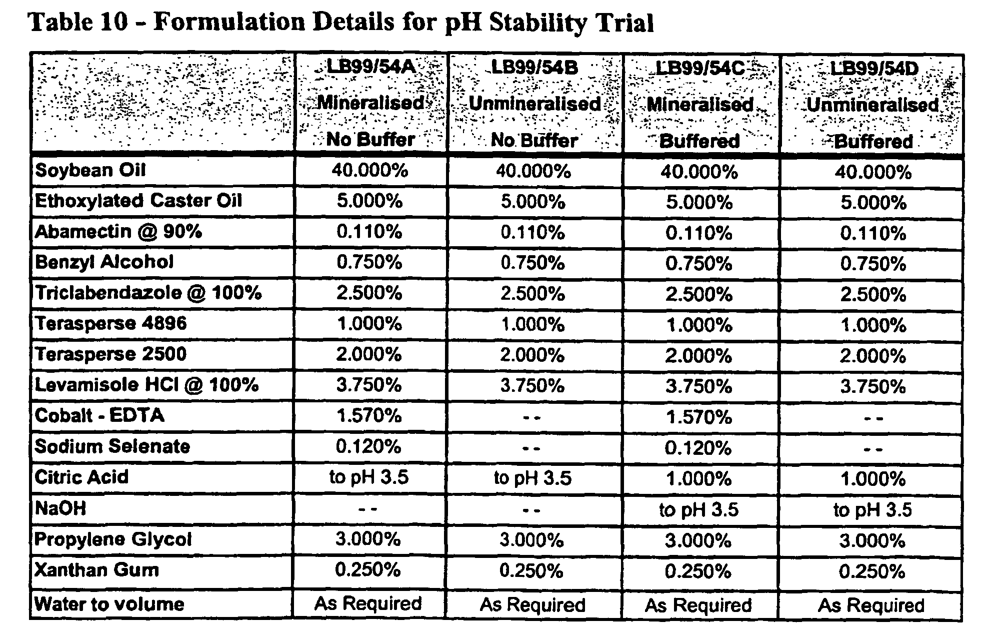

**Table 11 -**

| **Results: pH with time for compositions of Table 10. Ambient and 40°C Temperature. Ambient Humidity** | | | | | | | | |
|---|---|---|---|---|---|---|---|---|
| **Age (Days)** | **99/54A @ Ambient** | **99/54A @40°C** | **99/54B @ Ambient** | **99/54B @40°C** | **99/54C @ Ambient** | **99/54C @ 40°C** | **99/54D @ Ambient** | **99/54D @ 40°C** |
| **0** | 3.50 | 3.50 | 3.50 | 3.5 | 3.5 | 3.50 | 3.50 | 3.50 |
| **3** | 3.57 | 3.61 | 2.90 | 2.97 | 3.55 | 3.55 | 3.51 | 3.55 |
| **6** | 3.62 | 3.65 | 2.90 | 2.70 | 3.55 | 3.45 | 3.51 | 3.45 |
| **10** | 3.84 | 3.95 | 2.87 | 2.39 | 3.60 | 3.61 | 3.50 | 3.50 |
| **13** | 4.01 | 3.71 | 2.84 | 2.29 | 3.62 | 3.56 | 3.52 | 3.51 |
| **18** | 4.06 | 3.64 | 2.86 | 2.27 | 3.66 | 3.61 | 3.51 | 3.50 |
| **21** | 3.91 | 3.70 | 2.80 | 2.31 | 3.60 | 3.60 | 3.51 | 3.51 |
| **28** | 4.16 | 3.90 | 2.90 | 2.34 | 3.60 | 3.60 | 3.50 | 3.62 |
| **60** | 4.25 | 4.22 | 2.85 | 2.25 | 3.60 | 3.55 | 3.50 | 3.60 |
| **90** | 4.27 | 4.23 | 2.79 | 2.16 | 3.55 | 3.60 | 3.50 | 3.66 |

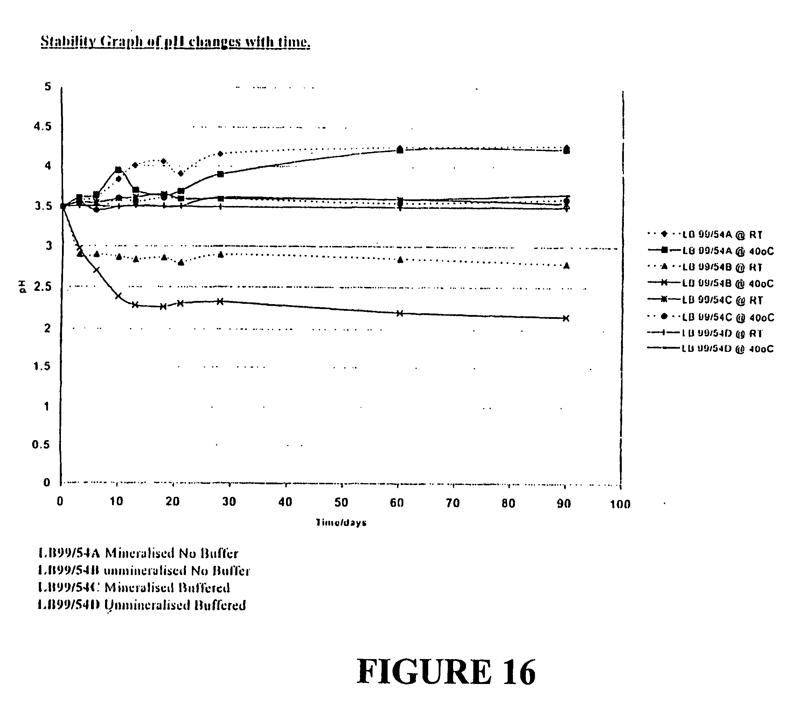

See Figure 16 for stability graph of pH change with time.

### Conclusions and Recommendations:

Some of the early batches of the present invention have shown a trend of significant pH changes with time. Mineral chelates also appear to affect the direction the pH change occurs, ie; with mineral chelates pH increases and without these pH decreases. In either case this is likely to have detrimentally effects on the stability of Levamisole which is normally expected around pH 3.0 to 4.0. Therefore some method of pH stabilising is desirable (if not necessary) for longer term stability.

The Citric Acid/Sodium Citrate system which uses Citric acid/sodium hydroxide (NaOH) appears suitable for this purpose in these formulations. Other pH stabilising systems (such as the Phosphate buffer system) have also been considered.

### Comparative Accelerated Stability

A study to compare the relative stability of a number of the formulations within the invention with standard commercial formulations at very high temperature (50°C) was conducted. The commercial formulations used have shelf-lives of 2 years or more. Six test formulations (A, B, C, D, E and J) and 4 commercial formulations consisting of 3 single active drenches (F, G, H) and 1 triple active drench (I) were placed under identical conditions, including identical high density polyethylene containers and identical closure systems and placed in a 50°C oven at ambient humidity. In the test formulations of the invention used either abamectin or ivermectin triple active formulations, with or without minerals, buffered or unbuffered, and one had the addition of praziquantel (E). These are detailed in Table 12 and 13.

### SUMMARY OF DRENCH PRODUCTS PLACED ON ACCELERATED (50 °C AMBIENT HUMIDITY) STABILITY:

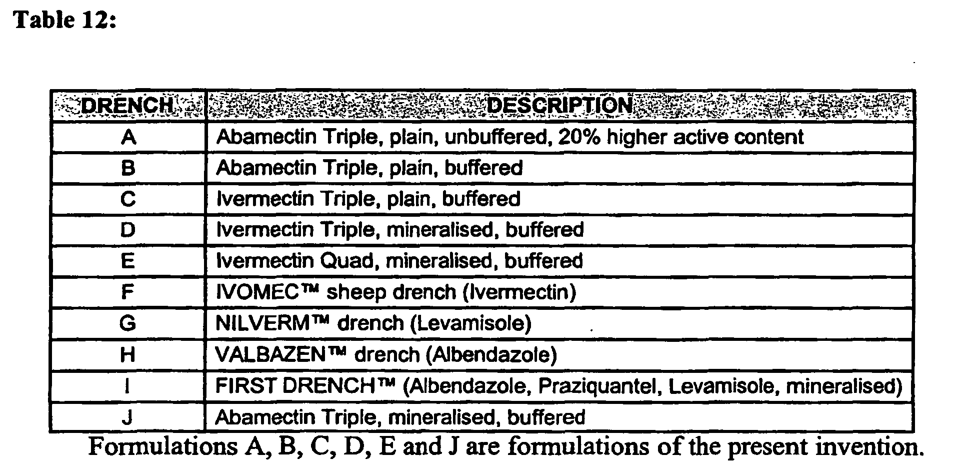

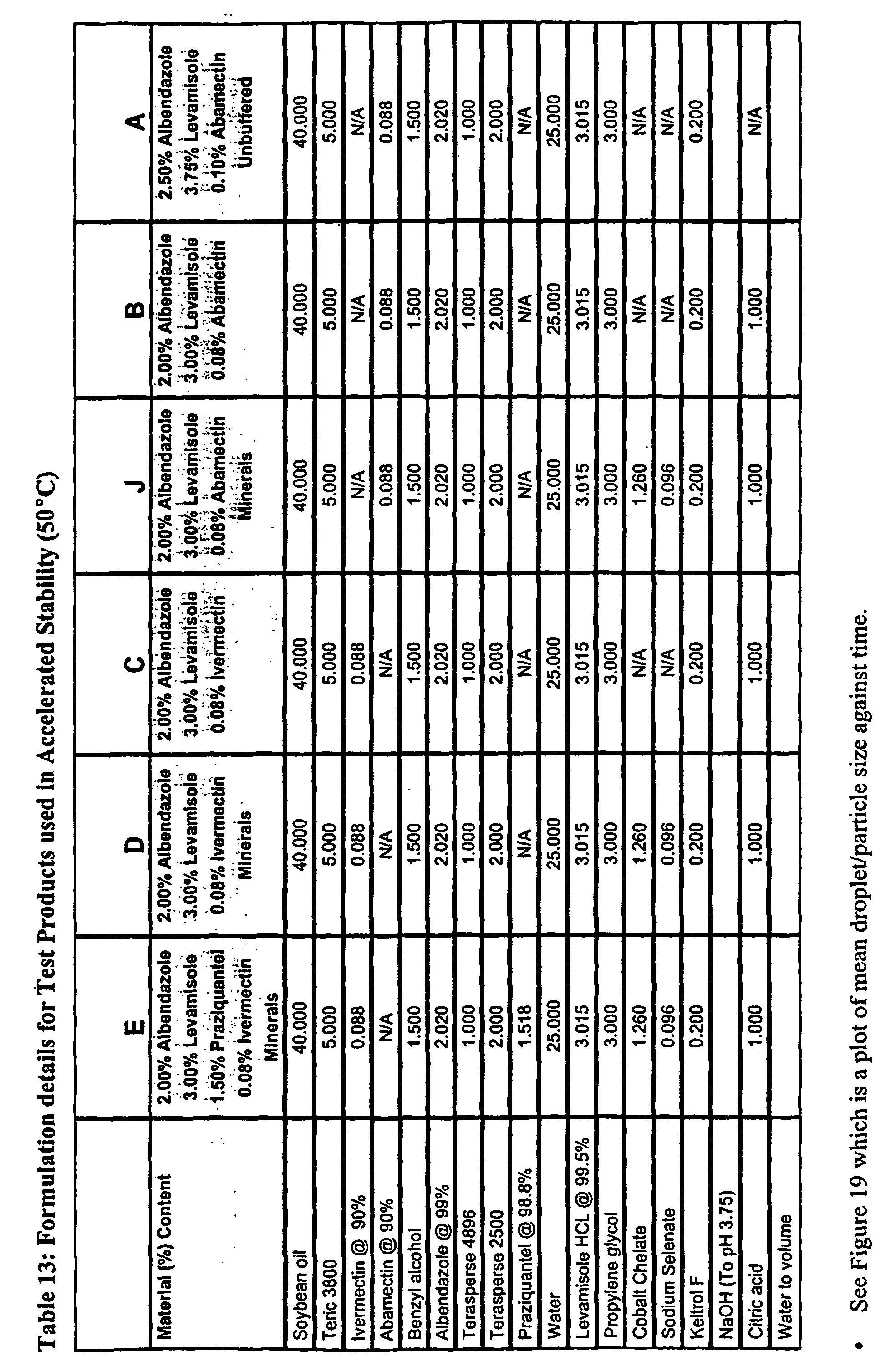

### POINTS TO NOTE REGARDING 4 WEEK ACCELERATED (50°C AMBIENT HUMIDITY) STABILITY RESULTS:

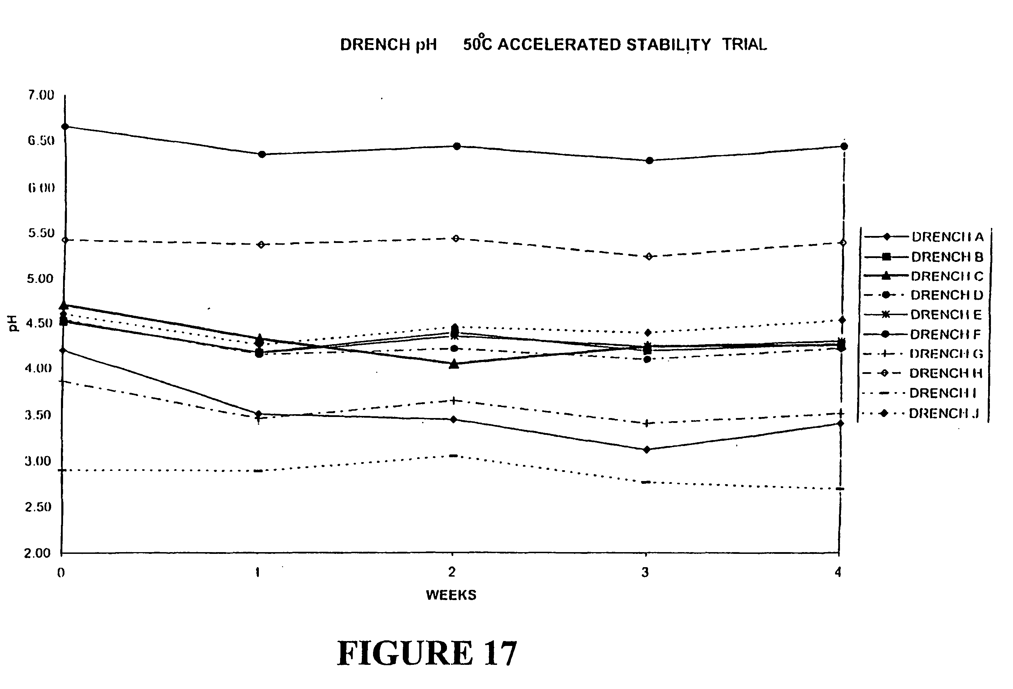

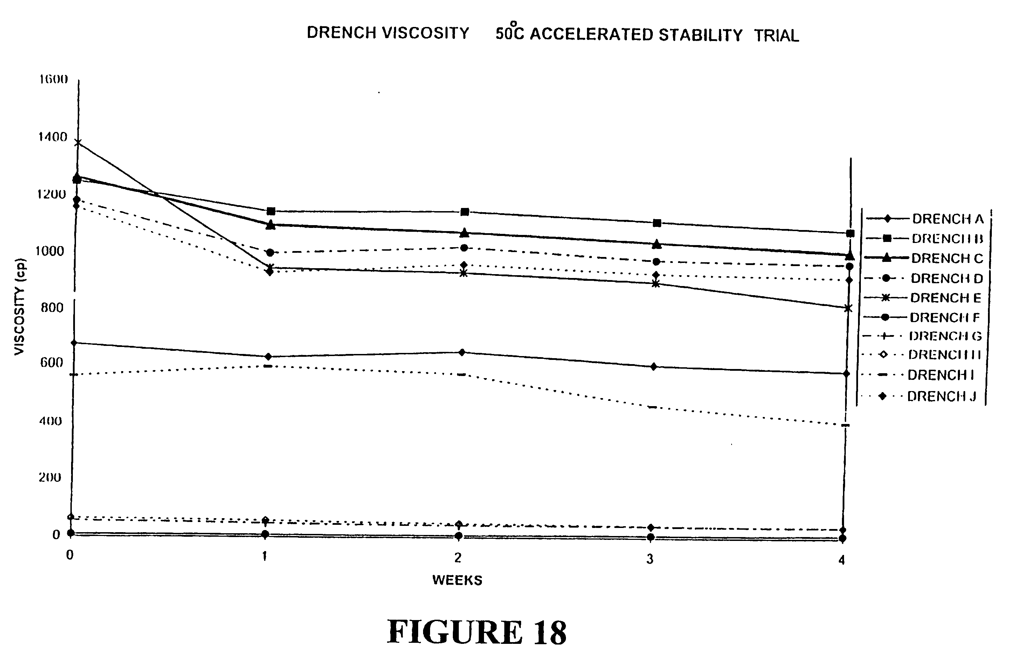

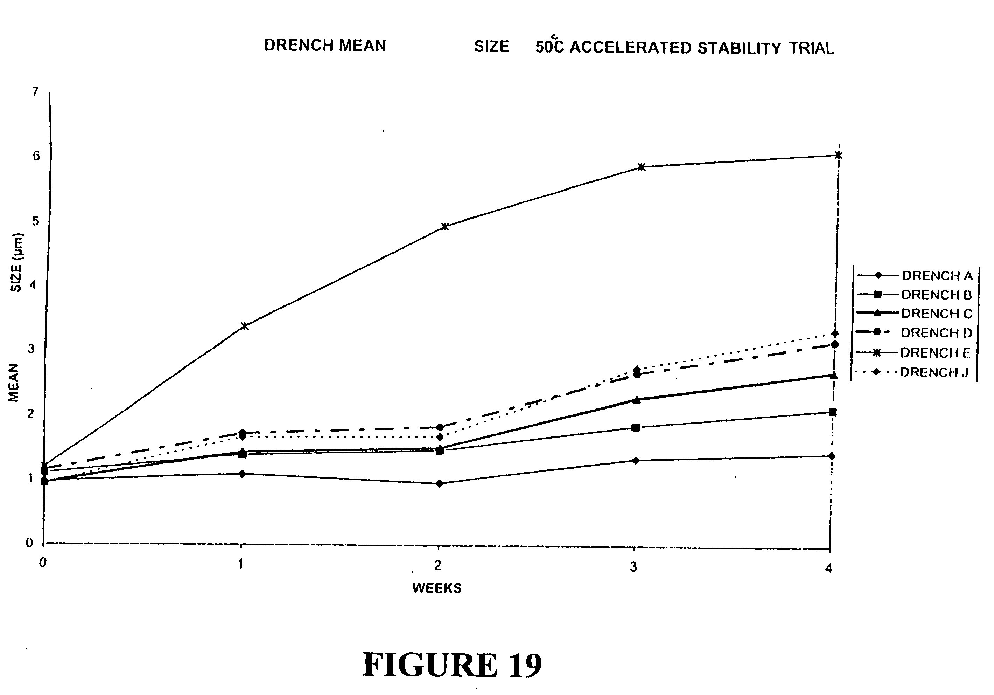

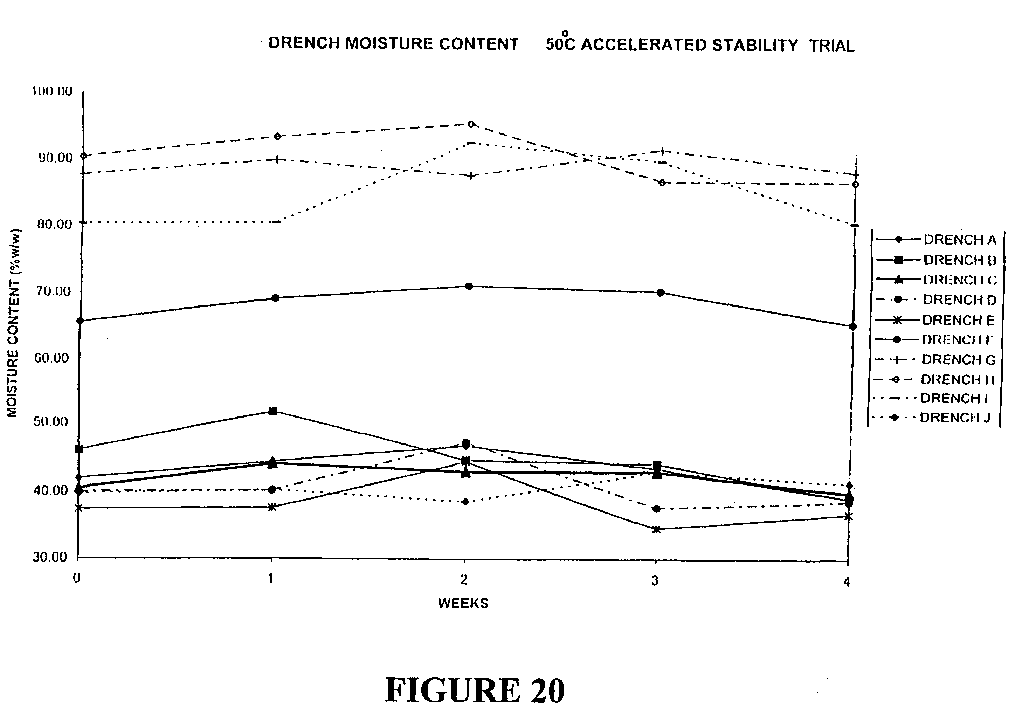

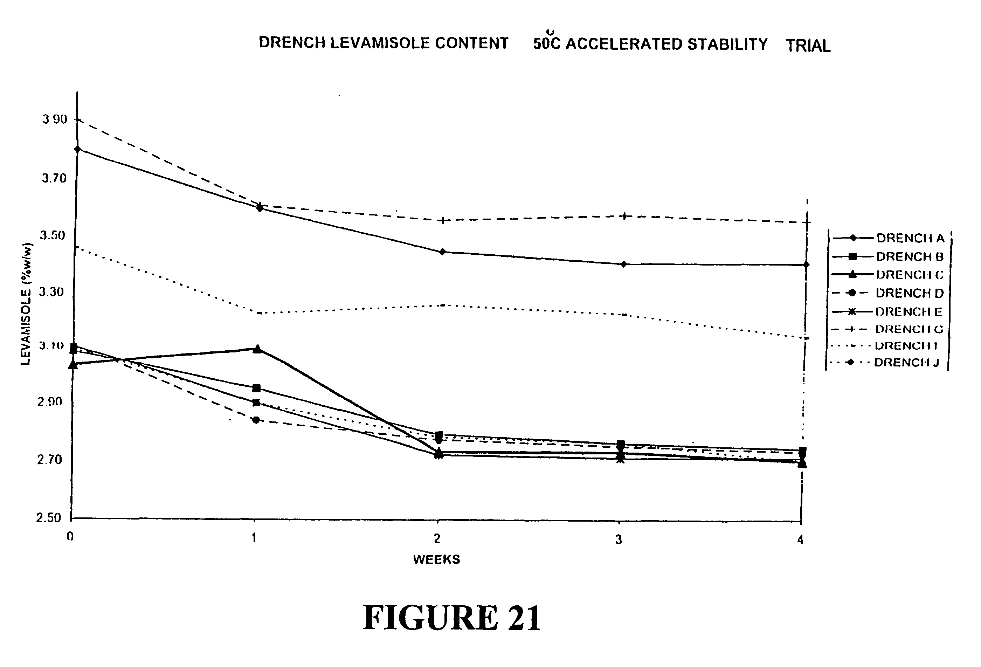

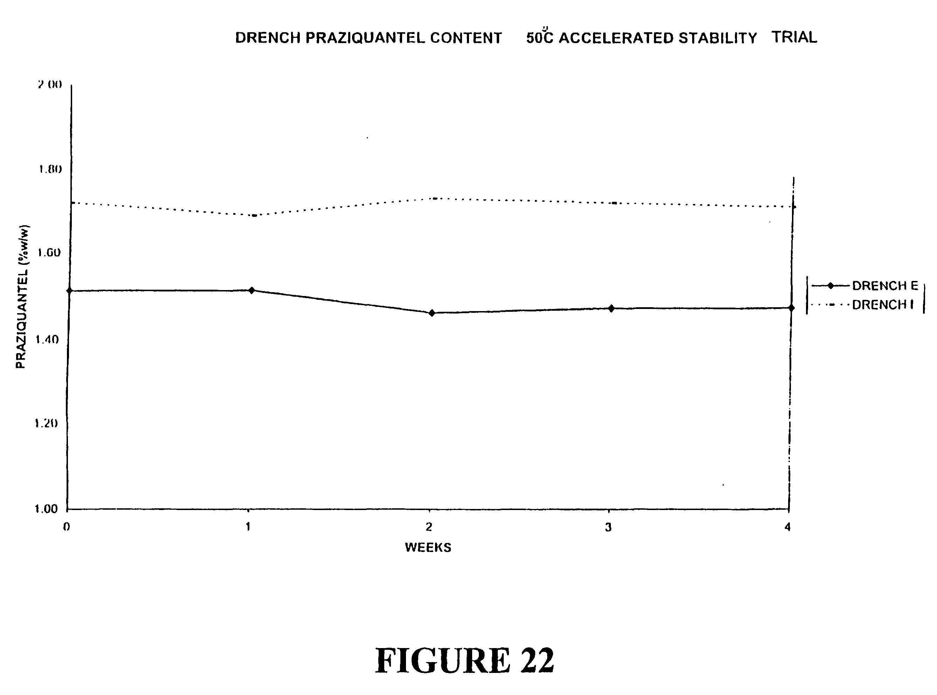

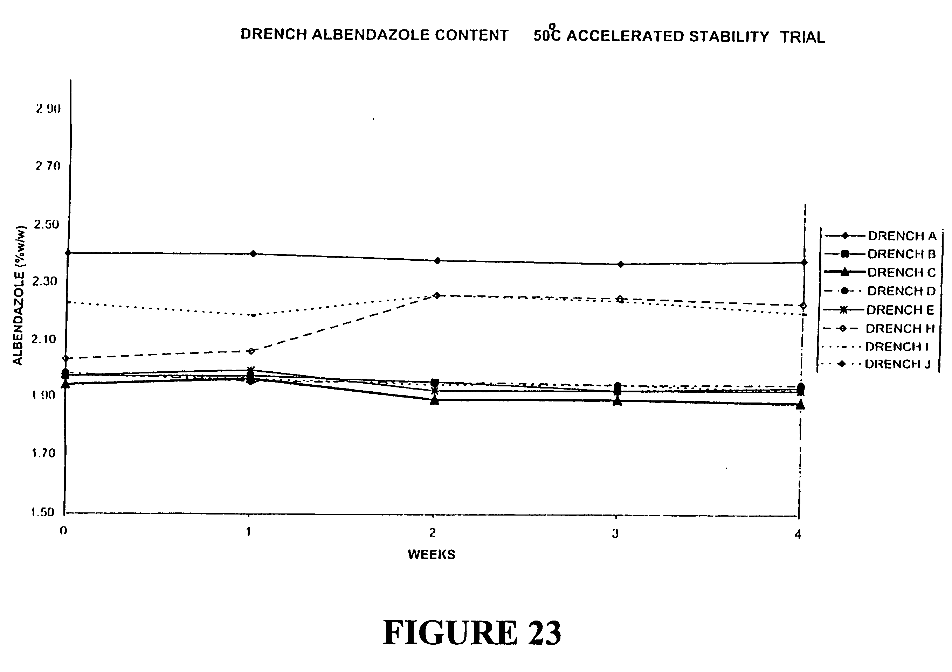

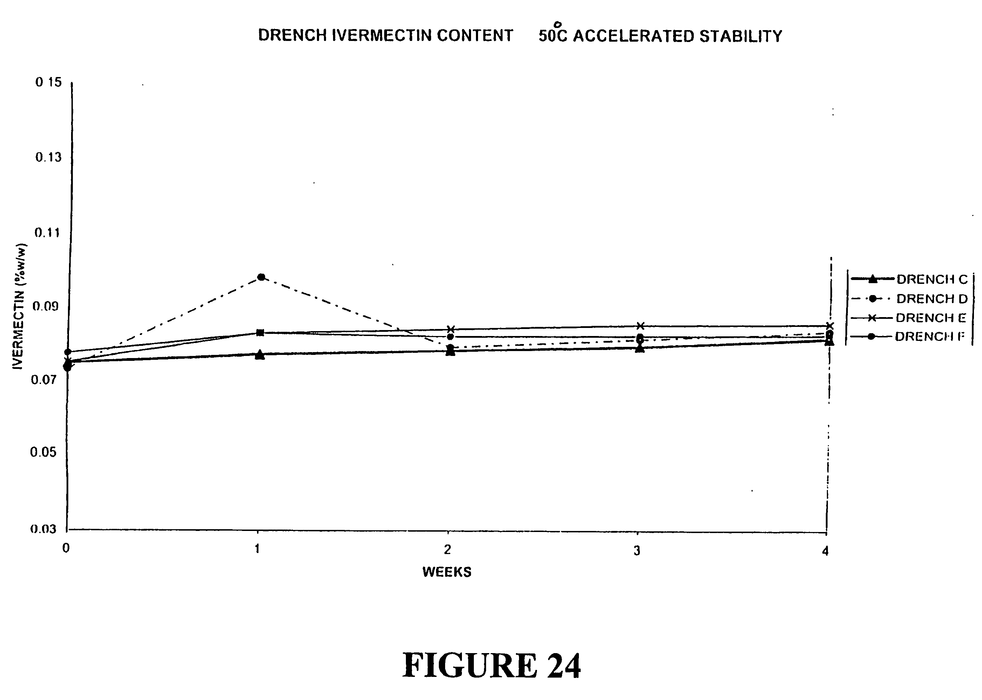

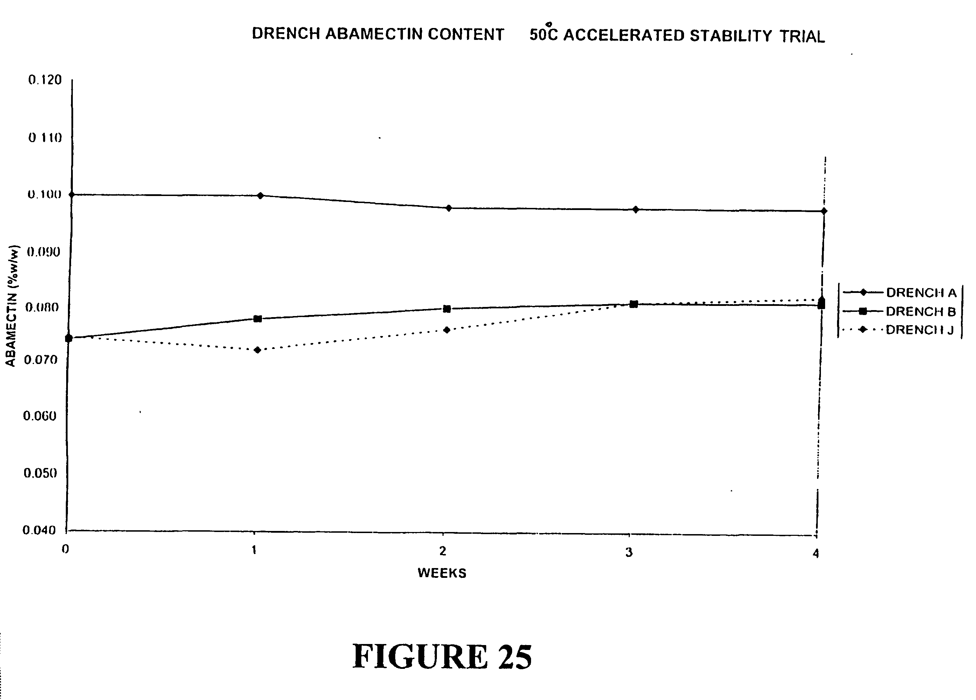

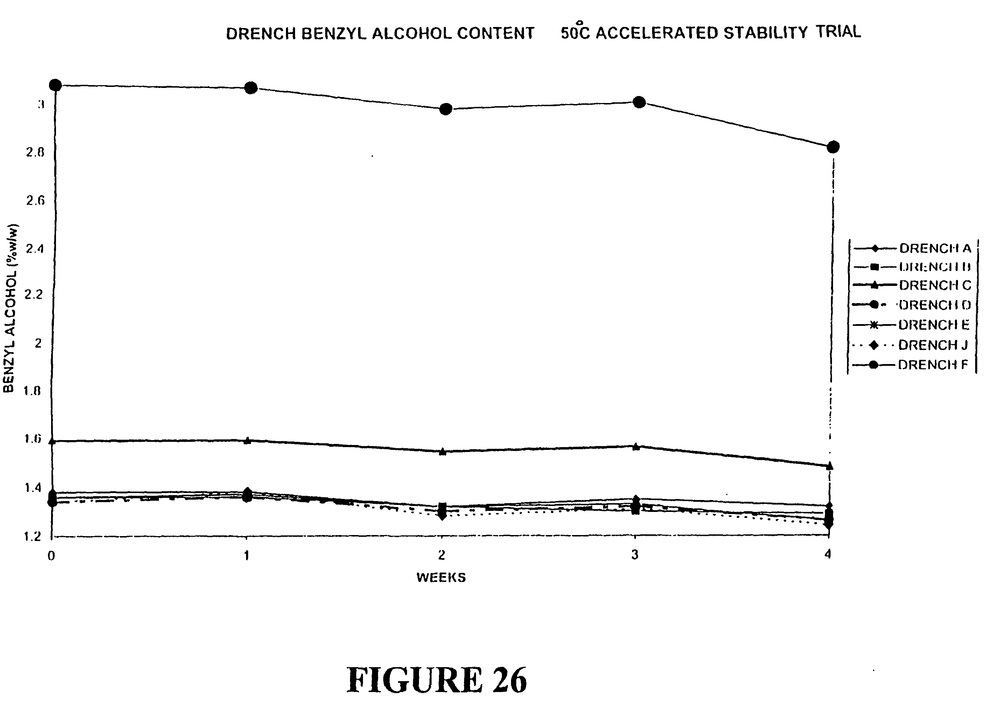

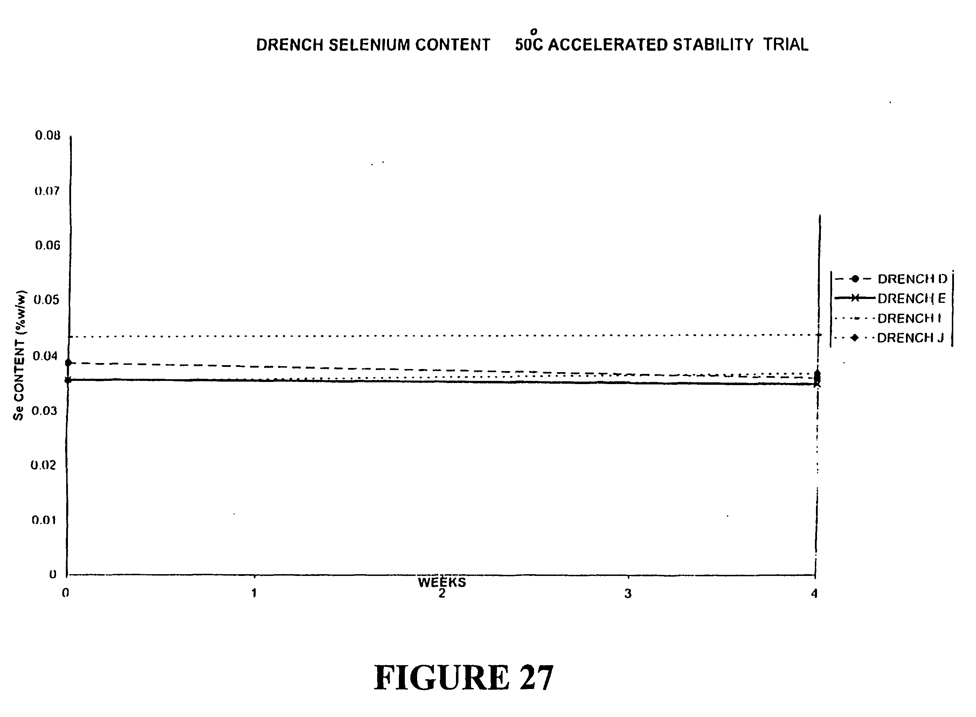

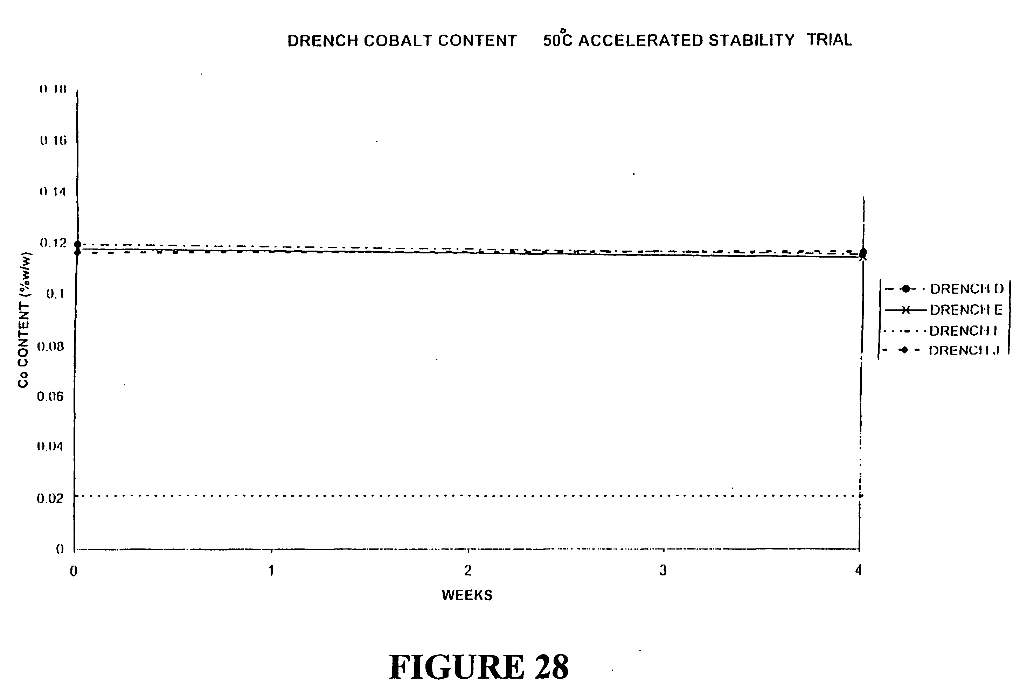

- pH change greatest for the unbuffered drench A, no other significant differences (Figure 17). The change in pH for all products was greatest in the first two weeks (especially after the first week). However, the changes in pH have not impacted on the physical or chemical stability of any of the formulations. The lack of buffering agent in drench A has not affected the stability of this drench relative to the others, at least not in the short term.
- Viscosity decrease was greatest in quad drench E, which started out with the highest initial viscosity (Figure 18). Correlates well with the observed particle size increase (Figure 19). The triple formulations of the present invention showed the best stability with respect to viscosity. In the test formulations the particles measured would predominantly be the oil droplets/particles.
- Drench F (IVOMEC™) has a very low viscosity (>100 x less than preferred formulations of the present invention), hence stability related decreases are not expected to be significant
- No significant trend in moisture content is apparent (Figure 20). The low values for A and B are due to the variability of the measurement technique - as seen on the weekly summary graph. The stability of the moisture content and the physical observations of the integrity of the containers (no change from initial) indicates that the packaging and closure system is adequate for the formulation products of the present invention in the short term at high temperature.
- Levamisole loss is consistent at around 10% for all of the drench products (Figure 21). Initial loss of active was rapid (from initial to 1 week through to 2 weeks) but then stabilised.
- No significant change in Praziquantel levels was observed (Figure 22).
- No significant change in Albendazole levels was observed. (Figure 23).
- The % Albendazole change for Drench H (VALBAZEN™) was calculated from week 2 to week 4, as the initial results appear to be erroneous.
- The Ivermectin (Figure 24) and abamectin (Figure 25) appears stable with similar trends between formulations and no obvious loss of active. The 1 week ivermectin result for drench D appears to have been erroneous.
- Benzyl alcohol loss (Figure 26) is consistent across all the products. Drench F (IVOMEC™ sheep drench) has twice the level of benzyl alcohol than the formulations of the present invention and also twice the water content.
- Selenium (Figure 27)and cobalt (Figure 28) levels in the mineralised drenches show no significant changes over the four week period.

### CONCLUSION:

After four weeks of storage at 50°C, ambient humidity:
- The formulations of the present invention show physical and chemical stability that is at least as good as products F, G, H and I available on the New Zealand market
- There is no difference in the physical or chemical stability of the Ivermectin products versus the abamectin products in formulations of the present invention.

### PARTICULATE CONTENT:

The stability of the formulation when albendazole particles were removed and substituted with Silicon dioxide and titanium dioxide particles was tested in the following formulations. These were used as pour-on formulations in cattle.

### A) Pour-on, Injectable and Gel Formulations:

Formulations were developed with higher loading of actives, substituting and loading particulate content using silicon dioxide and titanium dioxide. These formulations were developed to be used as pour-ons, injectible or as oral gels.
The following formulations were prepared.
LB99/96A - 0.5% Ivermectin + 1% Silicon Dioxide
LB99/96B - 0.5% Ivermectin + 1% Silicon Dioxide + 2.0% Titanium Dioxide as a UV screen.
LB99/96C - 0.5% Ivermectin + 10.0% Levamisole + 1% Silicon Dioxide with 2.0% Titanium Dioxide.
LB99/72A-0.1% Ivermectin, 3.75% Levamisole, 1.88% Praziquantel with 8% Silicon Dioxide.

**Table 14 -**

| **Ivermectin, Ivermectin/Levamisole and Ivermectin/Levamisole/Praziquantel** **Formulations With Silicon Dioxide and Titanium Dioxide Added** | | | | |
|---|---|---|---|---|
| **Material** | **LB99/96A** | **LB99/96B** | **LB99/96C** | **LB99/72A** |
| Soybean Oil | 40.00% | 40.00% | 40.00% | 15.00% |
| Teric 380 | 5.00% | 5.00% | 5.00% | 5.00% |
| Ivermectin @ 100% | 0.50% | 0.50% | 0.50% | 0.10% |
| Benzyl Alcohol | 5.00% | 5.00% | 5.00% | 1.50% |
| Levamisole HCl @ 100% | - | - | 10.00% | 3.75% |
| Colloidal Silicon Dioxide | 1.00% | 1.00% | 1.00% | 8.00% |
| Praziquantel | - | - | - | 1.88% |
| Titanium Dioxide | - | 2.00% | 2.00% | - |
| Propylene Glycol | 3.00% | 3.00% | 3.00% | - |
| NaOH (to pH 3.5) | 0.21% | 0.22% | 0.39% | 0.37% |
| Xanthum Gum | 0.50% | 0.50% | 0.10% | - |
| Citric Acid | 1.00% | 1.00% | 1.00% | 1.00% |
| Water to volume | To volume | To volume | To volume | To weight |
| Appearance | Off white fluid which separates on standing | White fluid which separates on standing | Homogeneous white fluid | Homogeneous white gel |
| pH | 3.5 | 3.5 | 3.5 | 3.5 |
| Viscosity % | 1,600cps | 1.800cps | 3,100cps | >100,000cps |

Note that Colloidal Silicon Dioxide was added to the formulation as a substitute for the solid component (such as Albendazole, Oxfendazole and Triclabendazole) that might desirably be found in the oral formulations. Titanium Dioxide filled a similar function to such benzimidazole solids insofar as stability is concerned as well as an UV screen.

### Observations:

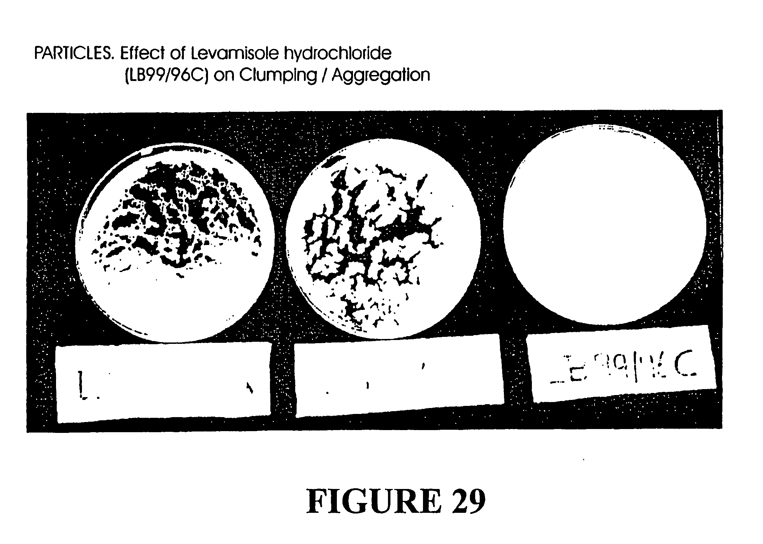

The solid component in LB99/96A and LB99/96B rapidly settled after agitation even though both formulations had five times as much Xanthan gum as LB99/96C. LB99/96C's higher viscosity and superior suspension properties is attributed to an interaction of Xanthan gum with the chloride portion of Levamisole Hydrochloride. See Figure 29.

Formulation LB99/96C was also considered suitable for an injectable formulation but ideally would have the silicon dioxide/titanium dioxide substituted with a readily biodegradable/metabolised particle and the levamisole hydrochloride substituted by levamisole phosphate to be less irritant at the injection site.

Formulation LB99/72A formed a white thick pourable gel which was considered to have application for example orally in horses, dogs and cats.

### B) Water Tolerance:

**Test -** The pour-on formulations listed in Table 14 (LB99/96A, LB99/96B and LB99/96C) were mixed with 10% extra water and any effect observed.
**Result -** No gelling. The additional water and drench phase mixed cleanly and no other reaction observed. This was also confirmed in other formulations including Example 12.

### C) Drenchability:

**Test -** Formulations of the present invention (such as LB 99/96A, LB99/96B and LB99/96C of Table 14) were applied though a standard drench gun containing residual water.
**Result -** Again no gelling occurred and the gun operated smoothly. Drenches of the present invention are unlikely to negatively affect the performance of the drench guns. The presence of oil in the formulation would likely act as a lubricant.

### FREEZE/THAW TRIALS:

The physical characteristic of drenches of the present invention when repeatedly frozen and then thawed was investigated. A series of Triclabendazole, Abamectin and Levamisole drenches, LB99/52A to LB99/52G (see Table 15) were formulated and was cycled between minus 4°C (- 4°C) and ambient conditions daily for 4 days. Their response to these conditions was compared to that of a number of current standard suspension drenches; SYSTAMEX™, VALBAZEN™ and FIRST DRENCH™.

**Table 15 -**

| **Test Formulations Used in Freeze Thaw Trials** | | | | | | | |
|---|---|---|---|---|---|---|---|
| **Material** | **LB99/52A** | **LB99/52B** | **LB99/52C** | **LB99/52D** | **LB99/52E** | **LB99/52F** | **LB99/52G** |
| **Soybean Oil** | 40.00% | 40.00% | 40.00% | 40.00% | 40.00% | 40.00% | 40.00% |
| **Teric 380** | 5.00% | 5.00% | 5.00% | 5.00% | 5.00% | 5.00% | 7.50% |
| **Abamectin @ 100%** | 0.10% | 0.10% | 0.10% | 0.10% | 0.10% | 0.10% | 0.10% |
| **Benzyl Alcohol** | 0.75% | 0.75% | 0.75% | 0.75% | 0.75% | 0.75% | 0.75% |
| **Triclabendazole@100%** | 2.50% | 2.50% | 2.50% | 2.50% | 2.50% | 2.50% | 2.50% |
| **Terasperse 4896** | 1.00% | 1.00% | 1.00% | 1.00% | 1.00% | 1.00% | 1.00% |
| **Terasperse 2500** | 0% | 1.00% | 2.00% | 3.00% | 4.00% | 2.00% | 2.00% |
| **Levamisole HCl @ 100%** | 3.75% | 3.75% | 3.75% | 3.75% | 3.75% | 3.75% | 3.75% |
| **Cobalt - EDTA** | 1.57% | 1.57% | 1.57% | 1.57% | 1.57% | 0% | 1.57% |
| **Sodium Selenate** | 0.12% | 0.12% | 0.12% | 0.12% | 0.12% | 0% | 0.12% |
| **Propylene Glycol** | 3.00% | 3.00% | 3.00% | 3.00% | 3.00% | 3.00% | 3.00% |
| **Keltrol F** | 0.3% | 0.30% | 0.30% | 0.30% | 0.30% | 0.30% | 0.30% |
| **Citric Acid** | 0.1% | 0.10% | 0.10% | 0.10% | 0.10% | 0.10% | 0.10% |
| **Water to volume** | As Required | As Required | As Required | As Required | As Required | As Required | As Required |
| LB99/52C is considered a "standard" with 1.0% Terasperse 4896, 2.0% Terasperse 2500, 5.0% Teric 380 and minerals. | | | | | | | |
| LB99/52A as per standard but no Terasperse 2500. | | | | | | | |
| LB99/52B as per standard but 1.0% Terasperse 2500. | | | | | | | |
| LB99/52D as per standard but 3.0% Terasperse 2500. | | | | | | | |
| LB99/52E as per standard but 4.0% Terasperse 2500. | | | | | | | |
| LB99/52F as per standard but no minerals. | | | | | | | |
| LB99/52G as per standard but 7.5% Teric 380. | | | | | | | |

### RESULTS - Table 15 Formulations

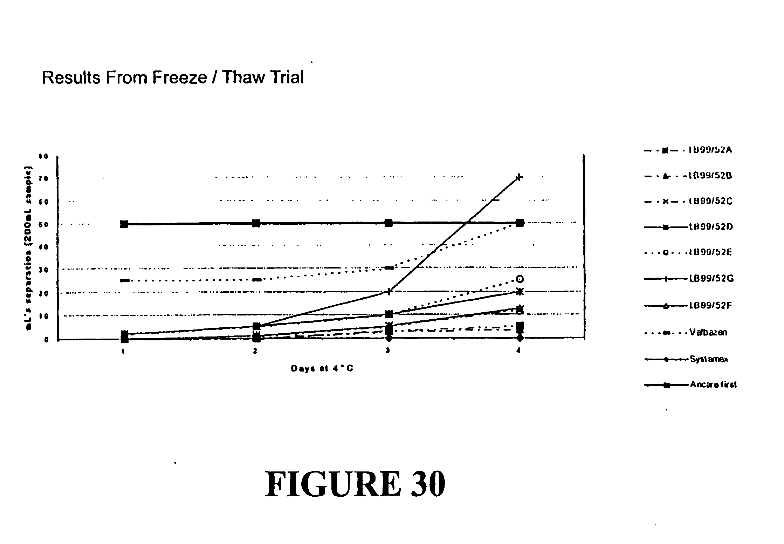

200mL's of each formulation was stored in clear 250mL glass bottles and examined for separation, homogeneity and sediment. These results are also presented in graph form in Figure 30.

### Terminology in Table 16

**Sep** ↑ or Separation top- mL's of clear material forming separate **above** any suspended material or sediment.
**Sep** ↓ or Separation Bottom- mL's of clear material forming separate **below** any suspended material.
**Hm** or Homogeneous - no indication of settling or sedimentation.

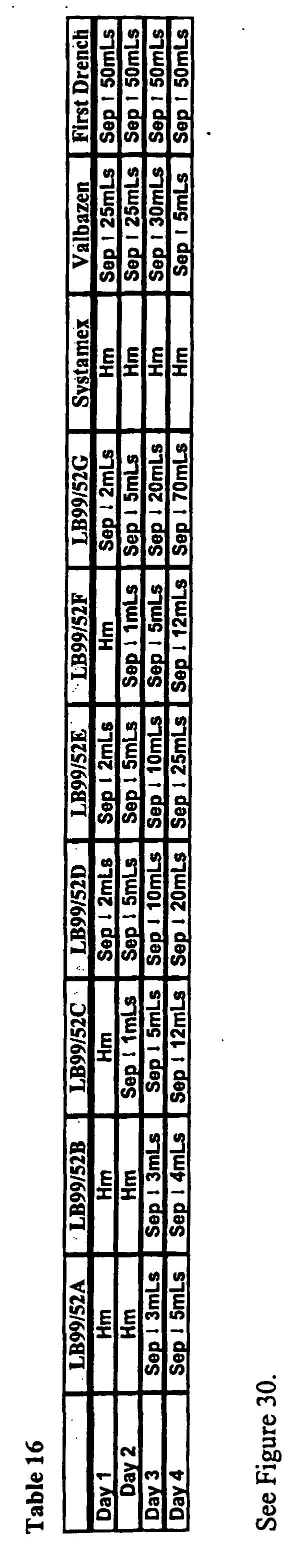

### Discussion:

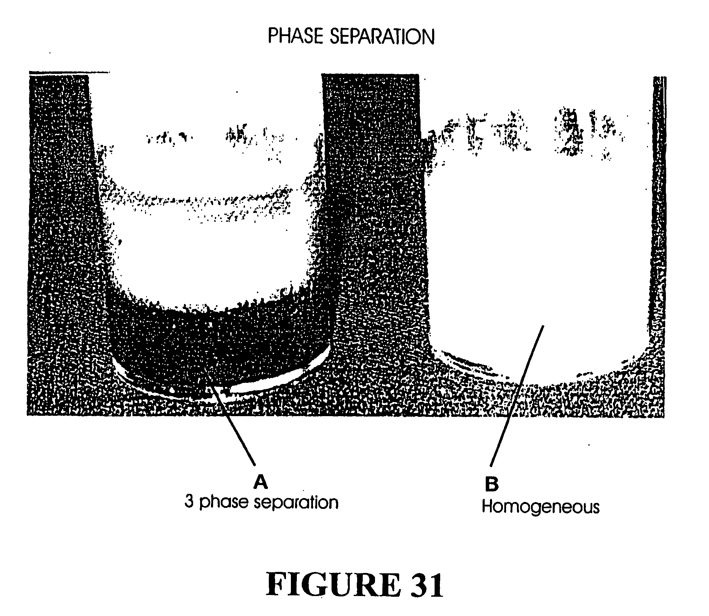

The following trends were evident;
1) Increasing the surfactant content (Teric 380 and Terasperse 2500) in a formulation of the present invention decreases it's physical stability under severe freeze thaw conditions.
2) Minerals have no noticeable effect on freeze/thaw properties.
3) Drenches of the present invention differ from standard suspension drenches in the way they separate. Standard drenches separate to form a clear layer on top, while drenches of the invention form a similar layer on the bottom. This may be due to density. Suspended solids are usually heavier than water and may sink, while oil-based emulsions, as in drenches of the present invention, are usually lighter than water and may remain in the top portion. In physically unstable formulations of the present invention there is a top phase of oil, a middle phase of suspended solids, and a bottom water layer phase. This is illustrated as A in Figure 31. This contrasts with the physically stable formulation (B of Figure 31) which remained homogeneous.
4) A standard formulation of the present invention containing 5.0% Teric 380 and 2.0% Terasperse 2500 appears to separate out less than VALBAZEN™ and FIRST DRENCH™ drenches.
5) All suspension drenches readily re-suspended with minimal agitation.
6) All drenches of the present invention readily re-emulsified with minimal agitation.
7) After storage in the freezer all drenches froze solid. The drenches of the present invention melted significantly faster than the traditional suspension drenches. Drenches of the present invention were fluid in less than 20 minutes while the suspension drenches took several hours.

### Stability Conclusions

The results of the accelerated stability trials at 30°C for up to 19 months and comparative stability at 50°C for 1 month, demonstrate that within the invention there are formulations with multiple actives that are chemically and physically stable including combinations of previously incompatible actives such as the ML's (ivermectin, abamectin) and Levamisole. In addition, the freeze/thaw trials, water tolerance, substitution and loading of actives demonstrate these are versatile and robust formulations suitable for field use. The presence of particulate matter, a suitable buffering system to maintain a stable acid pH, the presence of suitable and appropriate amounts of thickening agents, emulsifiers and oil have also been shown to determine the stability of these formulations. Surprisingly, increasing amounts of emulsifying agents were found to increase the physical instability and phase separation with freeze/thawing. The presence of a particulate material (such as a benzimidazole) and also levamisole hydrochloride was found to contribute positively to physical stability. Additionally not only were formulations found to be very versatile, capable of holding four actives simultaneously in one stable formulation, but the suspoemulsion's physical characteristics overcome some problems associated with current commercial suspensions. For example, in current benzimidazole suspensions, the particles with time settle and stick to the bottom of containers and are difficult to resuspend. This can affect the concentration and amount of active dosed. In the current invention, the particles settle toward the middle liquid layer not the bottom and so particles are readily available for resuspension.

The oil within the formulation also acts as lubricant in the drench gun. It is common farming practice to leave drench guns to sit with traditional suspensions within them. Over time there is settling out of particles and a drying out of the product including around the plunger. This can result in the particulate material abrading and damaging the drench gun when reused. In the current invention, the oil keeps the drench gun lubricated, making such damage less likely.

### Animal Trials Overview

The following animal trial data in cattle and sheep demonstrates that when given orally the combination of anthelmintics in the experimental formulations appears equally bio-available based on blood profiles (Trials NC001, NC002), when compared with commercial standard formulations alone, and this is supported by the egg count and worm kill data.

The benefits of combining previously incompatible actives together in one formulation is also demonstrated, with greater control of resistant or dose limiting gastro intestinal worms under experimental and field conditions. This benefit is demonstrated both with the oral formulations in sheep (Trials C052 and C0040) and as a pouron (Trial C012-400) in cattle.

In addition, mathematical models suggest there are long term benefits in the use of combination products with three or more actives from different action families both in extending and preserving the life of currently known anthelmintic or biocidal compounds.

### ANIMAL TRIALS

### • Cattle Pilot Study (NC 001) - Oral and Pour-on Treatment

Thirteen mixed breed weaner beef cattle (90-153 kg) from Northern New South Wales, Australia naturally infected with gastrointestinal roundworms were divided into four treatment groups by weight and nematode faecal egg count (FEC) into three groups of three animals and a fourth group of four animals.

Animals in group 2 were treated with the test combination formulation orally (Batch LB99/03, see example 15) at a dose of 1mL per 5 kg bodyweight. Group 3 of four animals had the same product (Batch LB99/03) applied as a pour-on along the midline of the upper back at a dose of 1 mL per 2.5 kg bodyweight. Group 4 received the same dose rate of the three actives as in group 2 given orally using three separate commercial formulations. Group 1 received no treatment (untreated control animals).

Animals were treated at Day 0 of the trial. Faeces for faecal egg count and blood samples for blood levels of active were collected at various times following treatment. Slaughter was at Day 14 of the trial where the total number of worms were counted in the gastrointestinal tract.

The results are presented below and in the drawings.

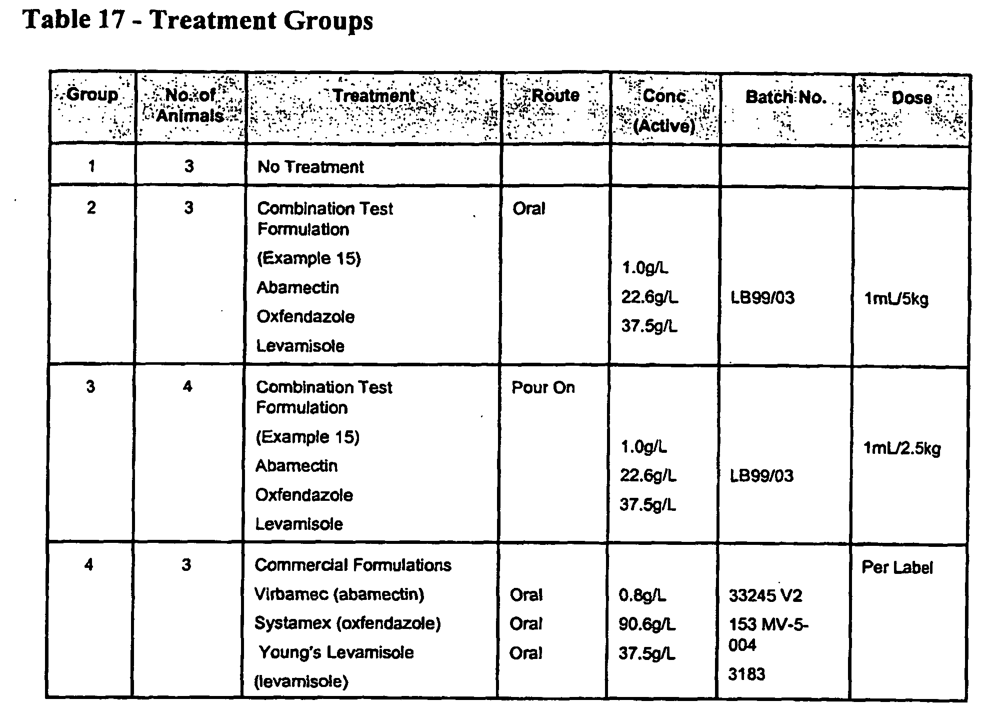

### RESULTS

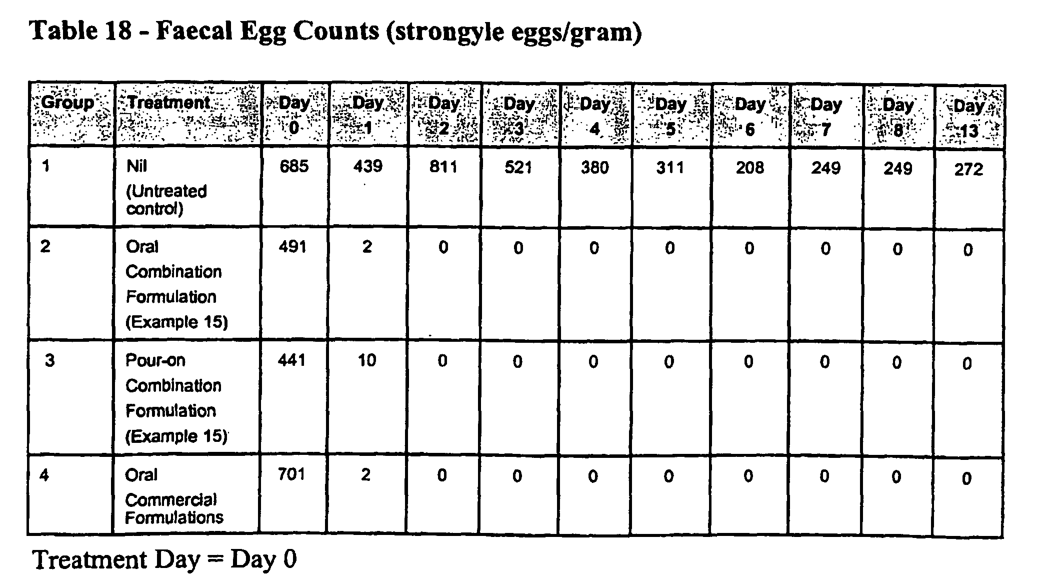

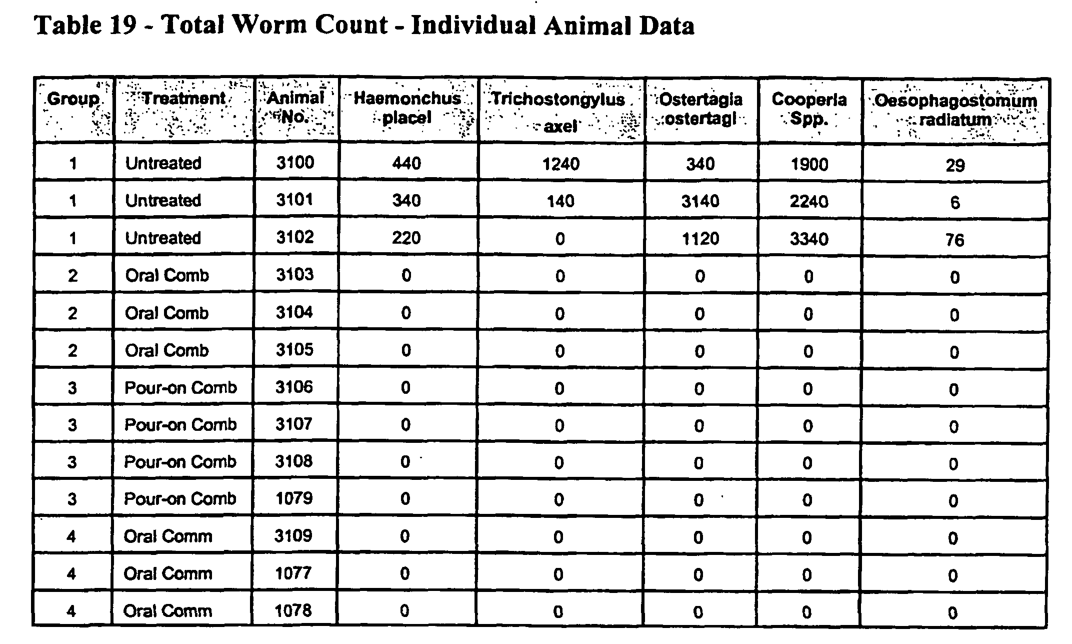

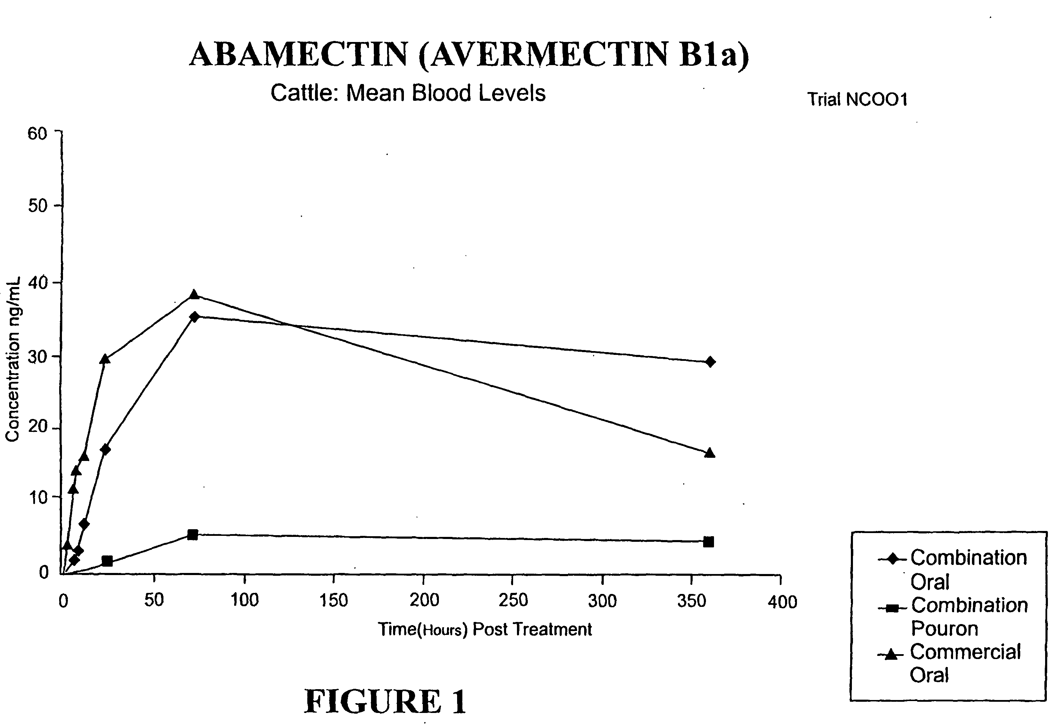

In the drawings
**Figure 1** is a plot of Avermectin B1a blood levels (ng/mL) against time (hours) for the different modes of administration,

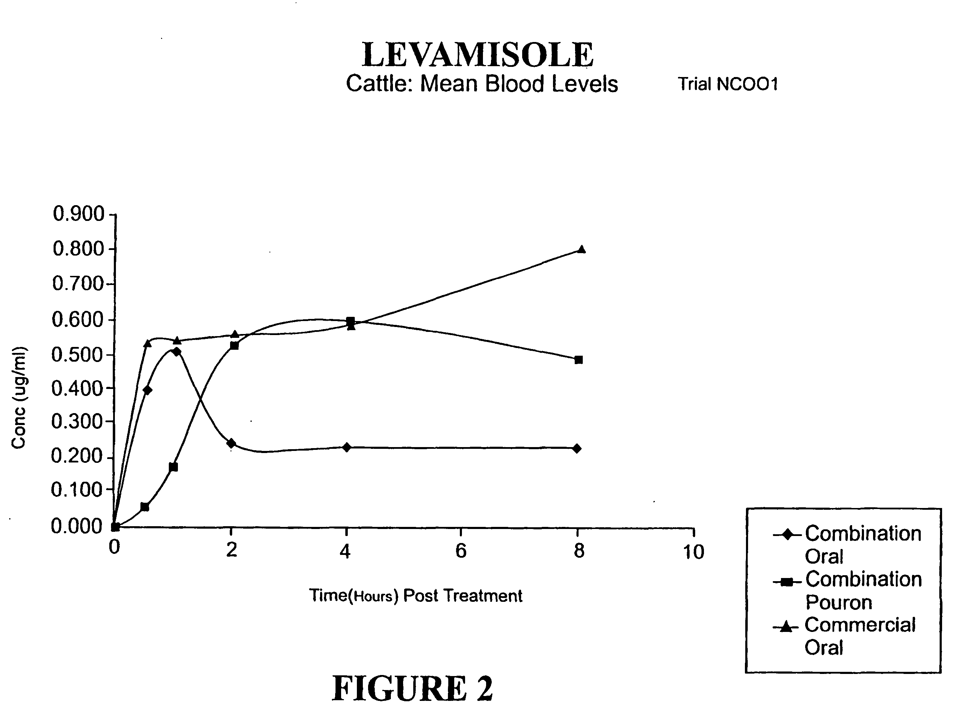

**Figure 2** is a plot of Levamisole blood levels (µg/ml) against time (hours) for the different modes of administration,

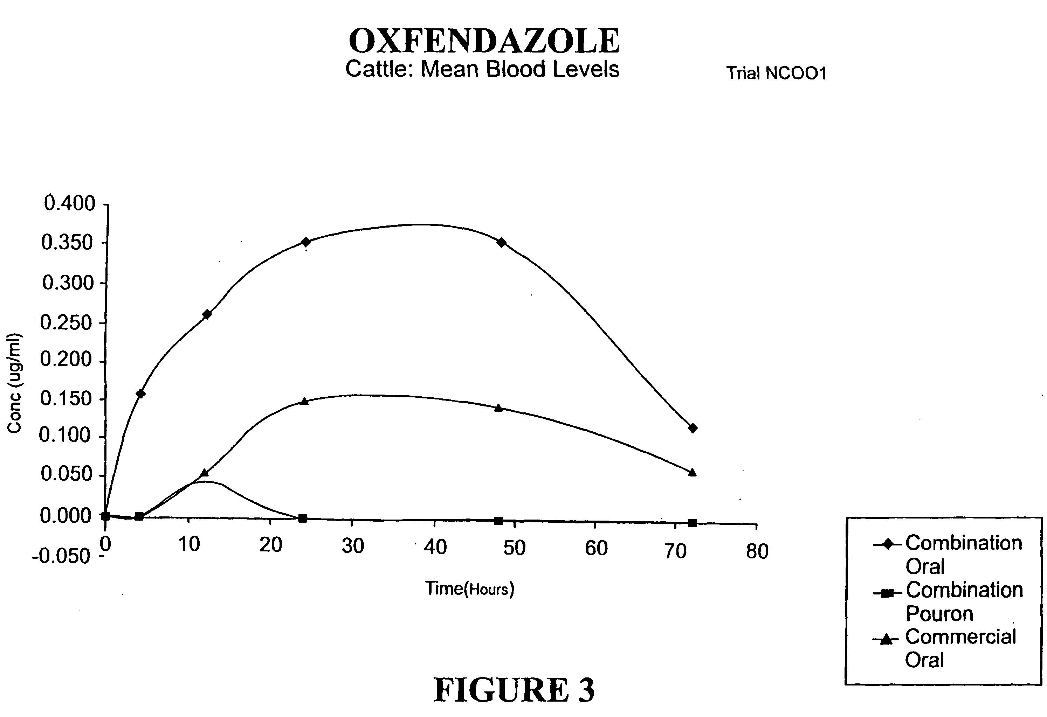

**Figure 3** is a plot of Oxfendazole blood levels (µg/ml) against time (hours) for the different modes of administration, and

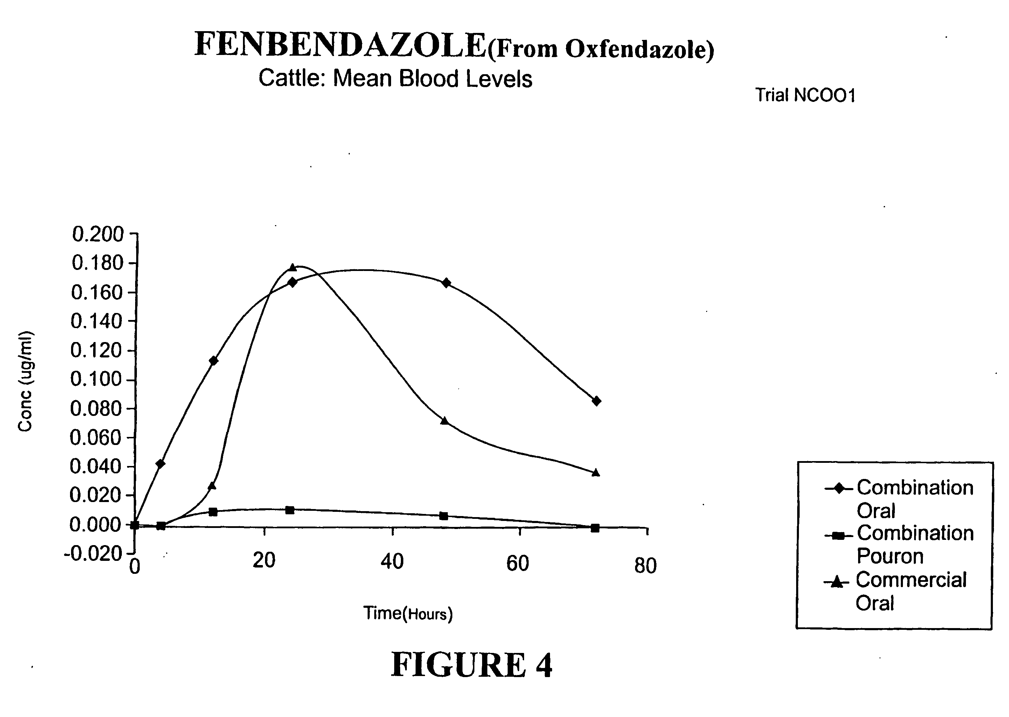

**Figure 4** is a plot of Fenbendazole blood levels (µg/ml) against time (hours) for the different modes of administration.

### RESULTS

The trial confirmed that the combination formulation (example 15, Batch LB99/03) was a highly effective anthelmintic in cattle both orally and as a pour-on. This is demonstrated by a complete (100%) reduction in faecal egg count, two days following treatment and a greater than 99.9% reduction of gastrointestinal nematodes compared with untreated controls.

The blood profiles of abamectin, levamisole and oxfendazole with the test product (example 15) given orally were essentially similar to those achieved in animals receiving the three commercial formulations of these actives. The higher blood levels of oxfendazole in the combination product were probably because of the oily nature of the formulation. Such differences have been seen previously between oily and aqueous benzimidazole formulations (Hennessy 1996). The blood profiles in pour-on treated animals shows that levamisole blood levels were similar to those achieved orally, the abamectin were ten times lower than with the commercial oral abamectin formulation and low levels of oxfendazole and fenbendazole (a metabolite) were detected in blood.

### • Sheep Pilot Study (NC 002) - Oral Treatment

Nine 17-20 month old merino hoggets from Northern New South Wales, Australia naturally infected with gastrointestinal roundworms were allocated on the basis of parasite faecal egg count (FEC) to 3 treatment groups of 3 animals, each group having a similar mean FEC.

At treatment day (day 0) animals in Group 1 received no treatment (untreated controls), Group 2 received the combination product orally as detailed in example 14 (Batch No. LB99/01) and Group 3 received the same dose of actives using 3 separate commercial formulations (abamectin, levamisole and albendazole) given orally.

Faeces for parasite FEC and blood for blood levels of active were collected at various times following treatment (day 0). Animals were slaughtered at day 14 following treatment, with total numbers of worms counted within the gastrointestinal tract.

The results are presented below and in the drawings.

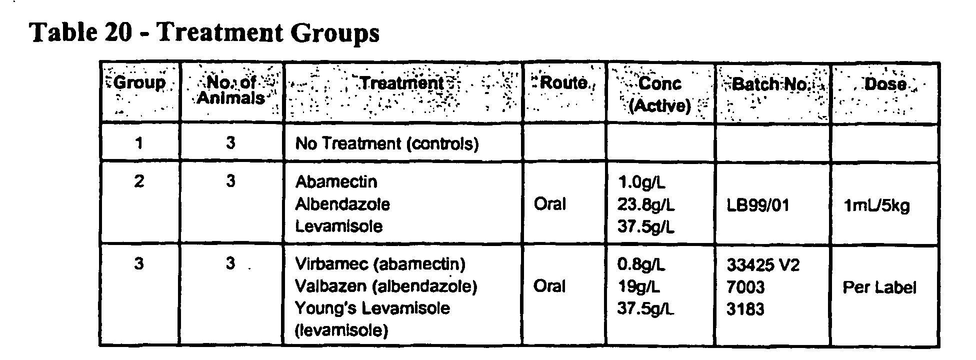

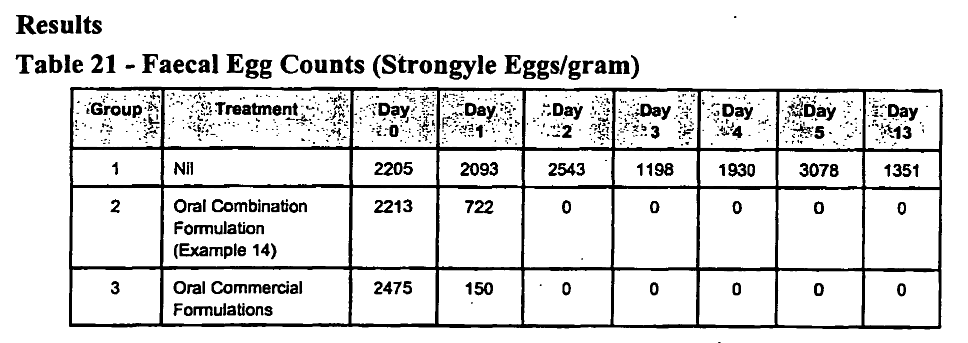

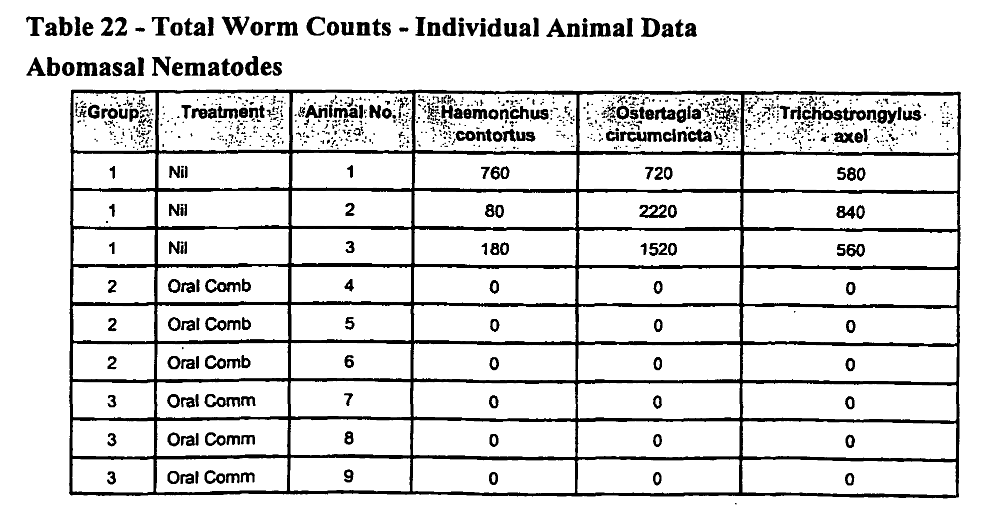

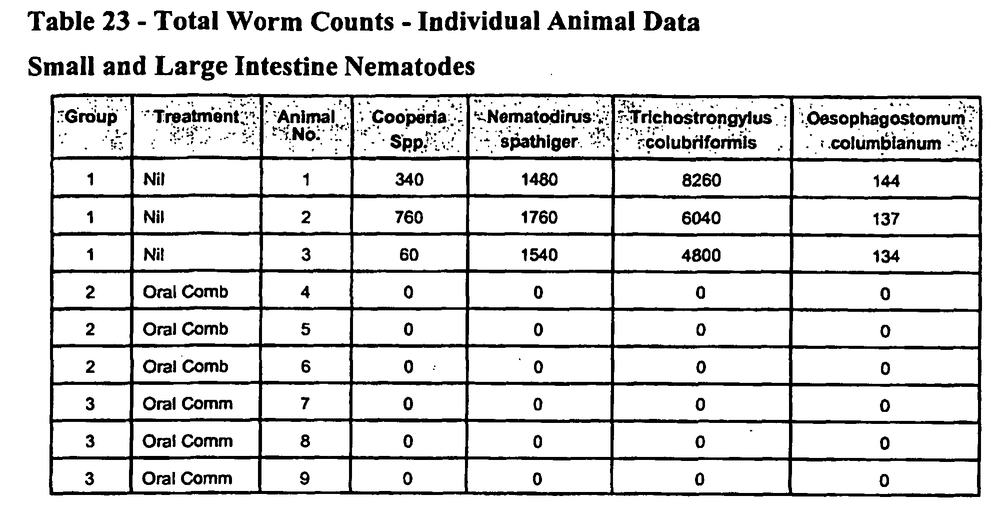

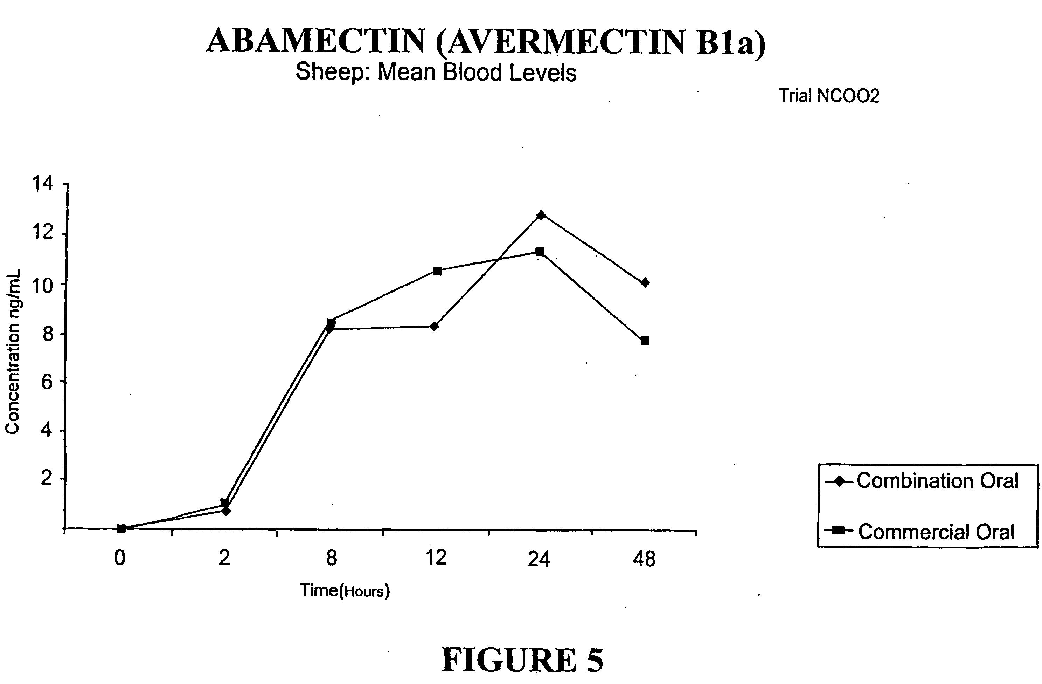

In the drawings
**Figure 5** is a plot of Avermectin B1a blood level (ng/mL) against time (hours) for the oral combination against a commercial oral formulation,

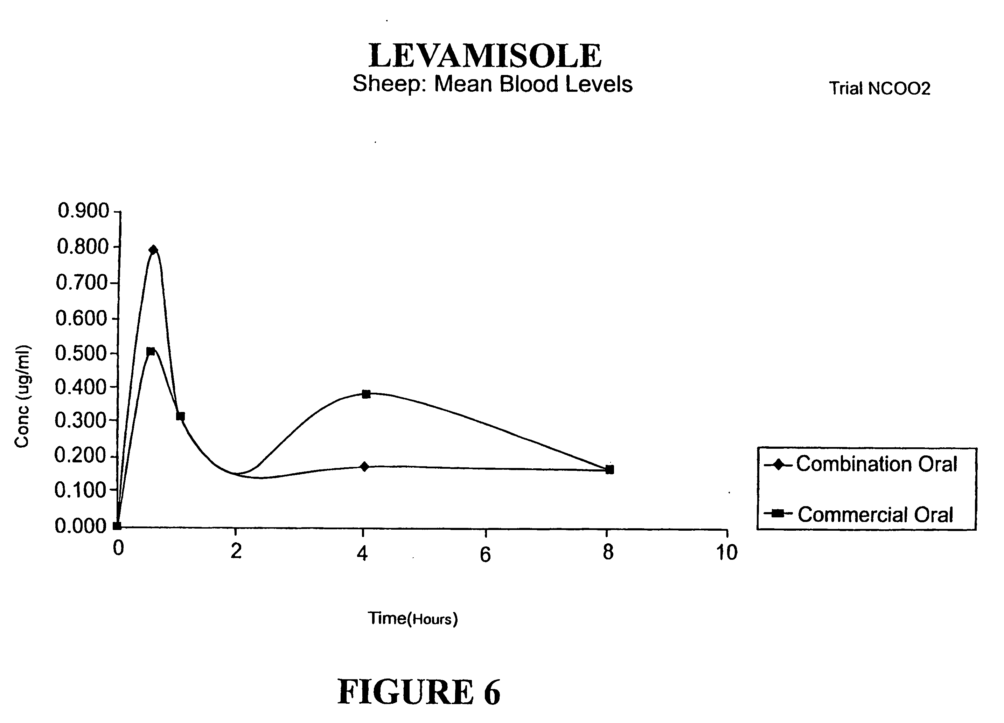

**Figure 6** is a plot of Levamisole blood level (µg/ml) against time (hours) for the oral combination against a commercial oral formulation, and

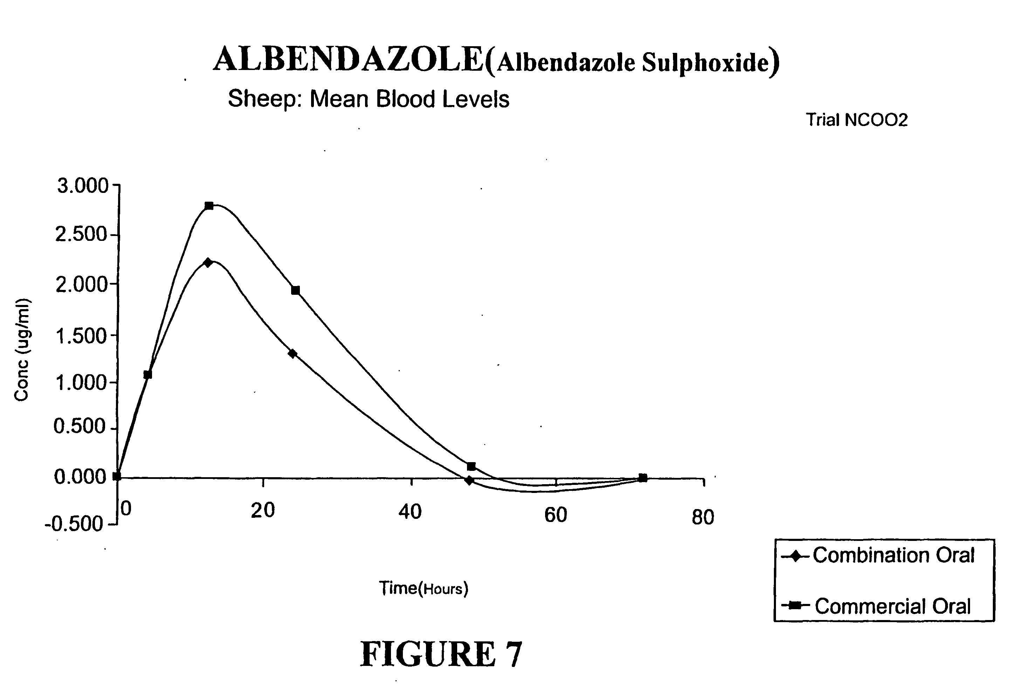

**Figure 7** is a plot of Albendazole blood levels (µg/ml) against time (hours) for the oral combination against a commercial oral formulation.

### Results

The trial confirmed that the combination formulation (example 14, Batch No. LB99/01) was a highly effective anthelmintic when given orally in sheep. This was demonstrated by a complete (100%) reduction in faecal egg count by day 2 and a greater than 99.9% reduction in all major worm species in the gastrointestinal tract.

The blood profiles of abamectin, levamisole and albendazole with the test product (example 14) were essentially similar to those achieved in animals receiving commercial formulations of the same three actives.

### CATTLE POUR-ON TRIAL (C012-400)

### • Cattle

Thirty Friesian and Friesian cross weaner beef cattle (113-189kg) naturally infected with gastrointestinal roundworms were randomly allocated into 5 treatment groups of 6 animals with mean faecal egg counts per group of 100-183 eggs per gram at treatment day.

One group (treatment A) remained as untreated controls, the other 4 treatment groups were treated with a pour-on formulation at Day 0 as described in Table 24 using a syringe held 3-5cm above the animal skin with the products applied along the midline of the back between the shoulder blades (withers) in a line approximately 15cm long. This area was chosen to prevent calves licking themselves and they were observed after pour-on treatment to ensure that licking of other calves, particularly the pour-on site, did not occur.

In this trial the benzimidazole (albendazole or oxfendazole) which is particulate and exists in the water phase had been substituted with fine particles of silicon dioxide (SiO₂) alone or in combination with titanium dioxide (TiO₂). See Formulations LB 99/96A, LB 99/96B, LB99/96C illustrated in Table 14). This was to determine if titanium dioxide which is used in sunscreens could significantly protect the ivermectin in the formulation. Ivermectin is degraded by UV light. Additionally the ivermectin content was increased from 0.1% to 0.5% to reduce the volume that needed to be applied to the animal (1mL per 10kg liveweight). This also allowed a comparison with a commercial ivermectin formulation IVOMEC™ pour-on which has a similar concentration of ivermectin.

The animals were slaughtered at day 8 and faeces (for faecal egg count) and the abomasum (stomach) and small and large intestines were collected for total gastrointestinal roundworm count. The results are presented in Tables 25 and 26. There were insufficient numbers of large intestinal worms to allow comparison.

**Table 24 -**

| **Treatment groups and formulation data Treatment Groups** | | | | | | |
|---|---|---|---|---|---|---|
| **Group** | **No. of Animals** | **Treatment Controls** | **% or g/100mL Concentration** | **Dose Rate (mLs per kg bodyweight (BW)** | **Batch No.** | **Expiry or Manufacture Date** |
| A | 6 | Controls | | | | |
| B | 6 | Commercial Ivomec Pour-on (B) | . | 1mL per 10kg | HJ11640 | Exp 12-2001 |
| | | Ivermectin | 0.5% | | | |
| C | 6 | Test Product C | | 1mL per 10kg | LB 99/96A | Date of Man |
| | | Ivermectin | 0.5% | | | 11/99 |
| | | Silicon Dioxide | 1% | | | |
| D | 6 | Test Product D | | 1mL per 10kg | LB 99/96B | Date of Man |
| | | Ivermectin | 0.5% | | | 11/99 |
| | | Silicon Dioxide | 1% | | | |
| | | Titanium Dioxide | 2% | | | |
| E | 6 | Test Product E | | 1mL per 10kg | LB 99/96C | Date of Man |
| | | Ivermectin | 0.5% | | | 11/99 |
| | | Levamisole hydrochloride | 10% | | | |
| | | Silicon Dioxide | 1% | | | |
| | | Titanium Dioxide | 2% | | | |

### Results

Only the test pour-on formulation containing both ivermectin and levamisole (formulation LB 99/96C) significantly reduced the faecal egg counts in treated animals. The egg count in Group E reaching zero (100% reduction) at Day 8 post treatment. This can be explained by the greater *Cooperia* worm kill in the small intestine of this treatment group. *Cooperia* is a highly prolific egg producing worm. Of these pour-ons only the ivermectin and levamisole combination (formulation LB 99/96C) was highly effective in removing adult *Cooperia* with a 95% or greater reduction in *Cooperia* worm numbers compared with untreated controls.

Both the commercial ivermectin formulation and the three test ivermectin pour-on formulations (LB 99/96A, LB 99/96B and LB 99/96C) were highly effective against adult stages of the abomasal worm *Ostertagia.* Titanium dioxide did not provide any significant improvement in worm kill (based on comparison of worm kill between formulation LB 99/96A and LB 99/96B). There was no significant skin or hair changes observed at the pour-on skin site suggesting the formulations were well tolerated and suitable as pour-on formulations. Observations of the formulations at the time of pour-on application were that pour-on formulations LB 99/96A and LB99/96B both appeared physically unstable with rapid separation of the formulation. This was not true of the ivermectin and levamisole formulation (LB 99/96C) which remained homogeneous. See photo Figure 17. This was an unexpected observation. In this trial the addition of levamisole hydrochloride to the test ivermectin pour-ons significantly extended the spectrum of activity of the pour-on to include *Cooperia* and also appears to contribute to the formulations physical stability.

### POUR-ON CATTLE TRIAL (CO12-400)

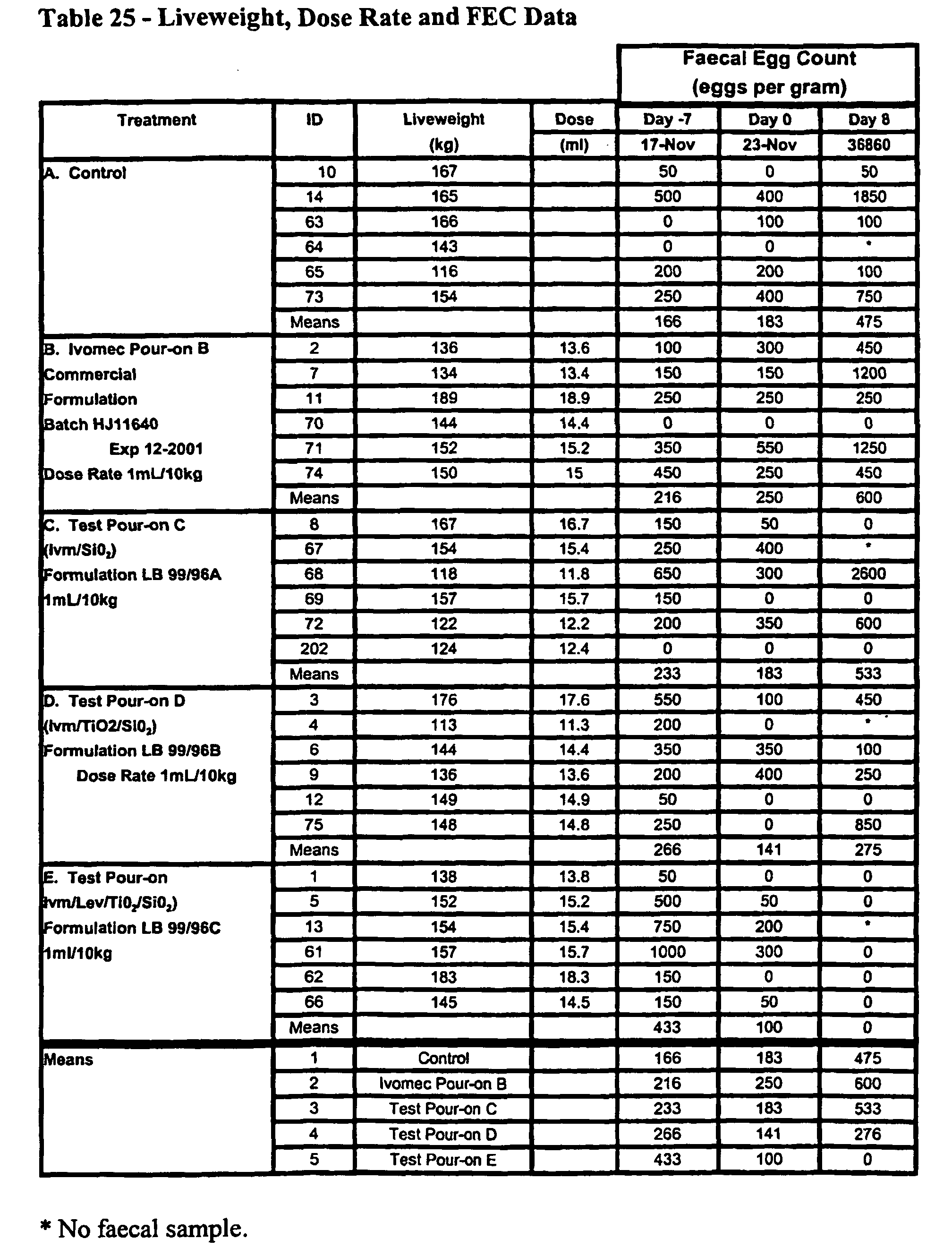

### POUR-ON CATTLE TRIAL (CO12-400)

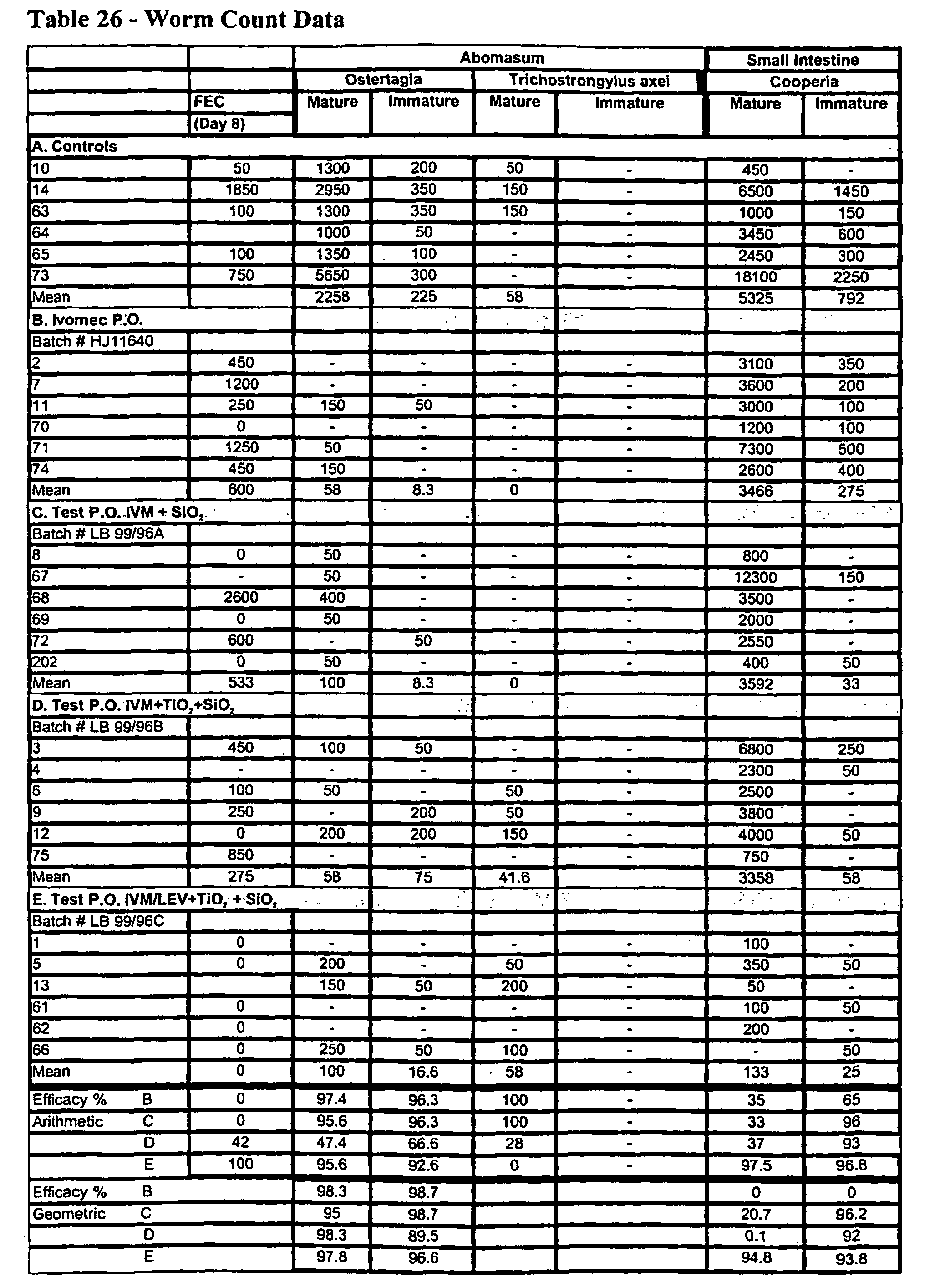

### SHEEP TRIAL (CO52)

An anthelmintic drench trial was conducted on a sheep farm in North Auckland, New Zealand using 67 Romney and Romney cross suffock lambs (18-35kg bodyweight) of mixed sex. The farm had a history of illthrift, scouring and lamb deaths consistent with gastrointestinal roundworm parasitism despite regular 4 weekly oral drenching.

The 67 lambs were randomly allocated by egg count into 4 groups of 10 lambs (Groups A, B, C, D) and 3 groups of 9 lambs (Groups E, F, G). The 3 groups of 9 lambs (E, F G) were drenched with a commercial formulation from one of the three broad spectrum anthelmintic drench families consisting of albendazole, a member of the benzimidazole or white drenches (Group E), levamisole, a member of the imidazothiazole / tetrahydropyrimidazole or clear drenches (Group F) and Ivermectin, a member of the macrocyclic lactones (Group G). Of the 4 remaining groups, Group A remained as untreated controls, Group B received the unmineralised test product (formulation 00-27C) containing all 3 actives (albendazole, levamisole, ivermectin), Group C received the same basic formulation but with the addition of selenium as the sodium selenate (formulation 00-27B) and Group D received the same basic formulation but with the addition of both selenium and cobalt with cobalt given as the cobalt chelate (formulation 00-27A) - See Table 27.

All animals were weighed and dosed by syringe to the nearest 0.1ml with the appropriate product for that group. Six days after oral treatment the animals were slaughtered and faecal samples were collected at slaughter. The liveweight, dose rates and faecal egg count data are presented in Table 28.

### TEST

### FORMULATIONS FOR SHEEP TRIAL (CO52):

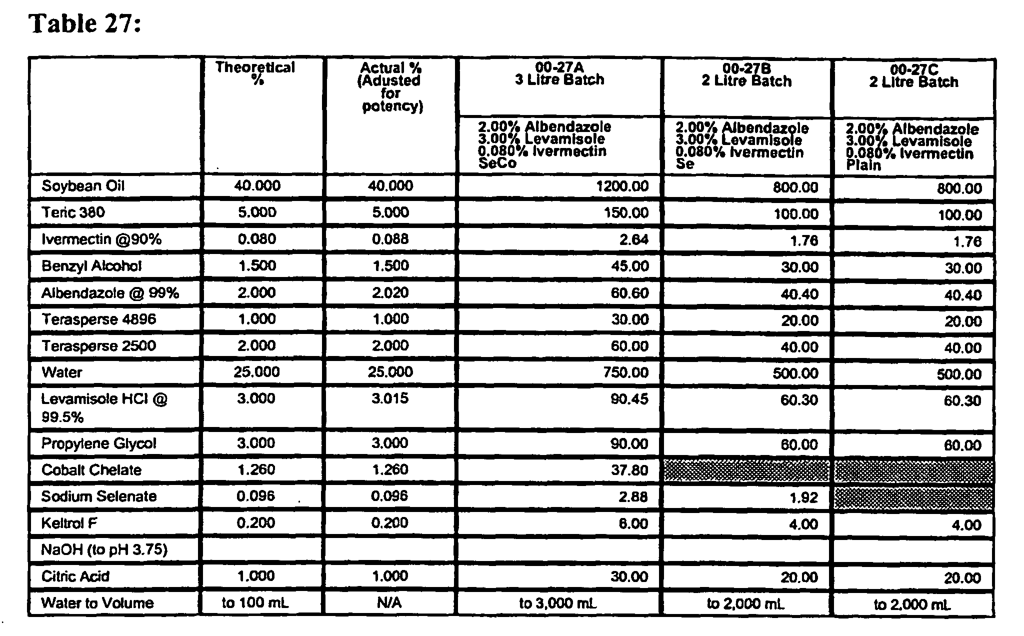

### Results:

The egg counts at slaughter in the untreated animals and the less effective anthelmintics were considerably higher than at treatment day (Day 0). The most likely explanation for this is reduced volume and flow of intestinal contents. This is likely the result of reduced food intake and a greater colonic absorption of water because of yarding the previous day (Day 5) increasing the concentration of eggs per gram of faeces. Despite this, a comparison with the egg counts of the untreated control animals is valid and gave reductions in egg count of 79% for ivermectin, 63% for albendazole and only 22% for levamisole (based on arithmetic mean).

These egg count reductions suggest resistance to all 3 drench families and are well below reductions typically seen with these drench families in susceptible worm populations. All test combination formulations (00-27A, 00-27B, 00-27C) gave 100% reductions in egg count, with greater than 95% reduction considered to indicate a highly effective anthelmintic treatment.

This trial confirmed the benefits of combining all 3 actives in the test formulations.

This combination resulted in a highly effective anthelmintic drench, despite resistance being present on this farm to each of the 3 anthelmintic drench families when given separately.

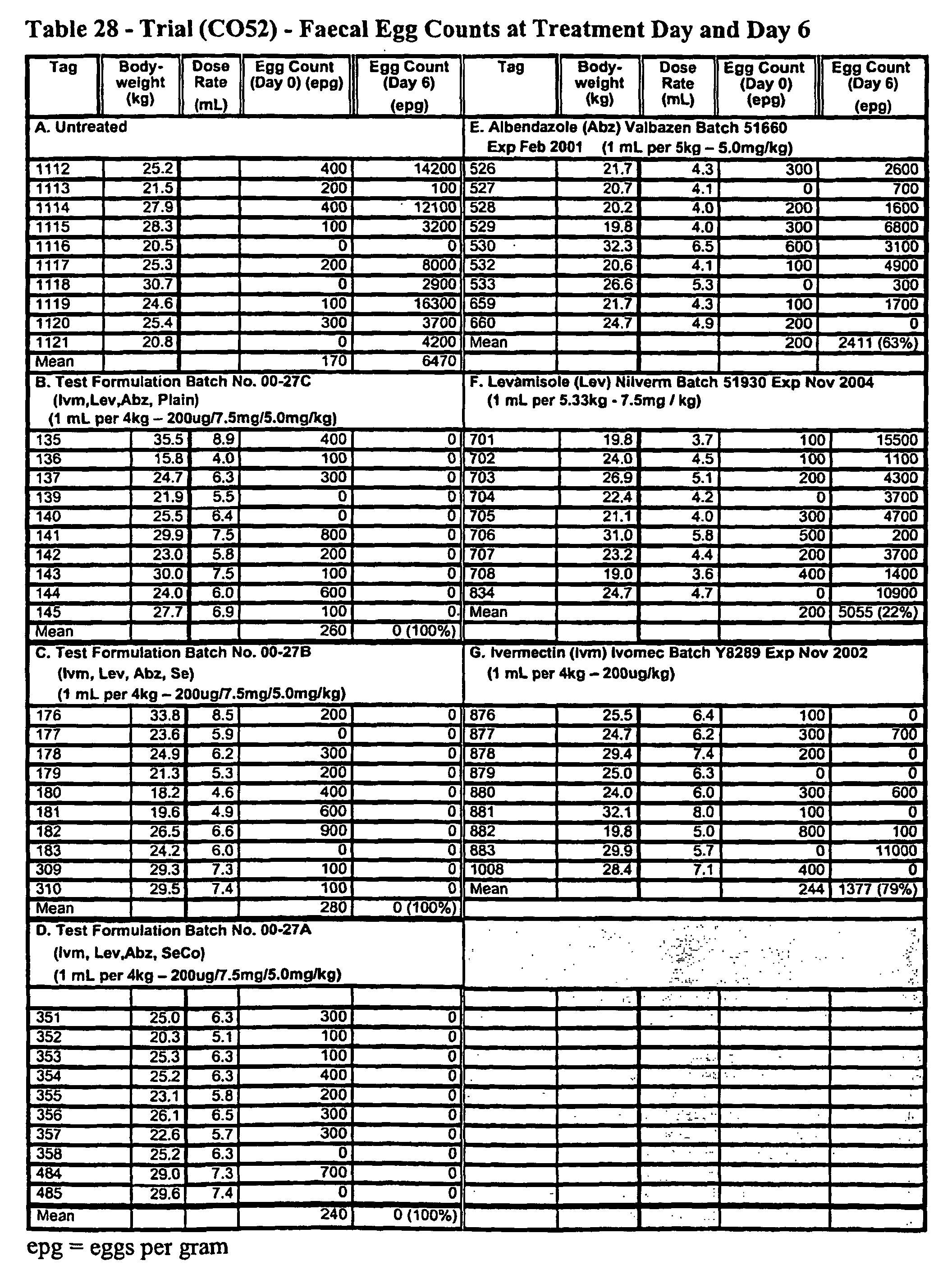

### TRIAL C0040 - SHEEP ROUNDWORM AND TAPEWORM TRIAL

Fifty three pasture fed 6-8 month old Romney lambs from the southern North Island, New Zealand that were naturally infected with tapeworms, were orally dosed with three times the standard dose of ivermectin (3 x 0.2 = 0.6mg/kg liveweight) to remove their naturally acquired roundworm infection while leaving their tapeworm burden. The animals were then housed indoors on slats to prevent natural reinfections with roundworms and each lamb was orally dosed daily for 5 days with a similar mixture of 5,000 roundworm larvae (total 25,000 larvae) consisting primarily of *Haemonchus, Trichostrongylus, Ostertagia* and *Nematodirus.* The larvae originated from four New Zealand sheep farms, three of which had evidence of resistance to one or more drench families. Lambs were injected with 0.5mL of a corticosteroid (opticortenol) two days prior to artificial oral larval infection. This was done to assist establishment of the worm larvae. 25 days after infection the animals were restrictively allocated to groups by bodyweight and also the presence of tapeworm infection, to one of seven treatment groups (Groups A-G). Four treatment groups (the untreated control and test products Groups A-D) had 8 animals per group, and three groups (E-G) contained 7 animals per group. The groups with 7 animals per group (E, F, G) were orally dosed with a single standard commercial product. All animals were slaughtered at Day 7 and Day 8 after treatment and total numbers of gastrointestinal roundworms and tapeworms counted. The treatment groups, liveweight and formulation and dose rate data are summarised in Table 29. The worm count data, including tapeworm data is summarised in Table 30.

### Results

### Roundworms

All products including the three test formulations (LC00/14, 99/99B, LB00/09) were highly effective (greater than 95% reduction in worm numbers) against *Haemonchus* except albendazole which reduced numbers by 93% (based on arithmetic means). The *Ostertagia* however showed greater resistance with only 54% removal of worm numbers with ivermectin alone and 64% removal by albendazole alone. Levamisole was still effective at 96% removal but the greatest removal (>99.9%) was achieved with the three combination test products.

Against *Nematodirus* in the small intestine, ivermectin and the three test products were fully effective (100% reduction), levamisole removed 99% of adult *Nematodirus* and albendazole removed only 59%.

All formulations were fully effective (100%) against *Trichostrongylus* in the small intestine except albendazole which was 80% effective.

### Tapeworms

While there appeared to be a slight trend to less *Moniezia* volume in albendazole treated animals, only the test formulation LB00/09 with the addition of praziquantel was effective in removing the entire tapeworm (scolex and segments).

### Conclusion

Levamisole alone and the combination test formulations (LB00/14, 99/99B, LB00/09) were highly effective against all roundworm species in this trial. Despite not having roundworms with marked resistance to levamisole, the three test formulations removed more worms than any of the single active commercial formulations alone indicating the benefits of combining the actives together in one single formulation. Additionally only the test formulation with praziquantel was effective in removing the tapeworm *Moniezia.*

### TRIAL C0040

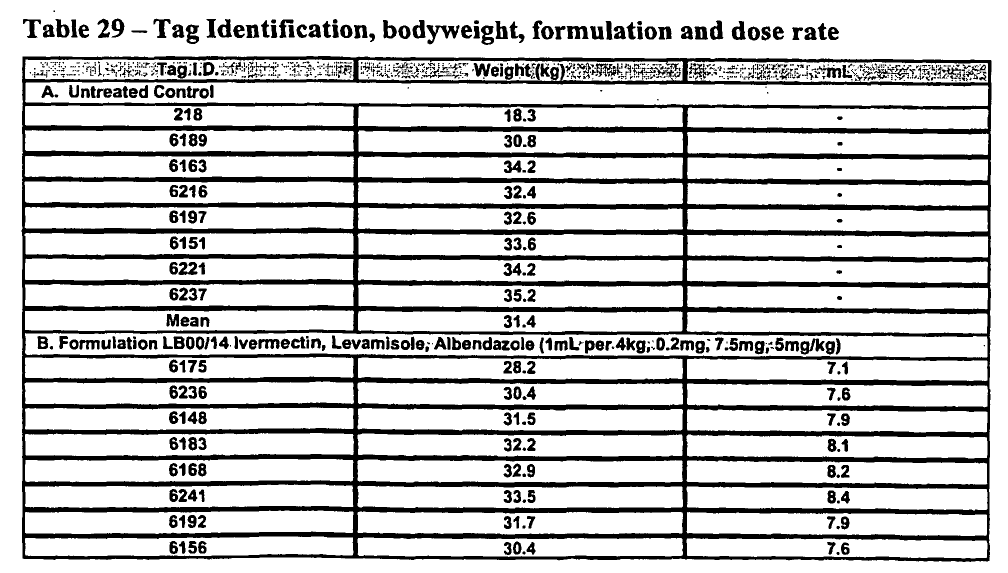

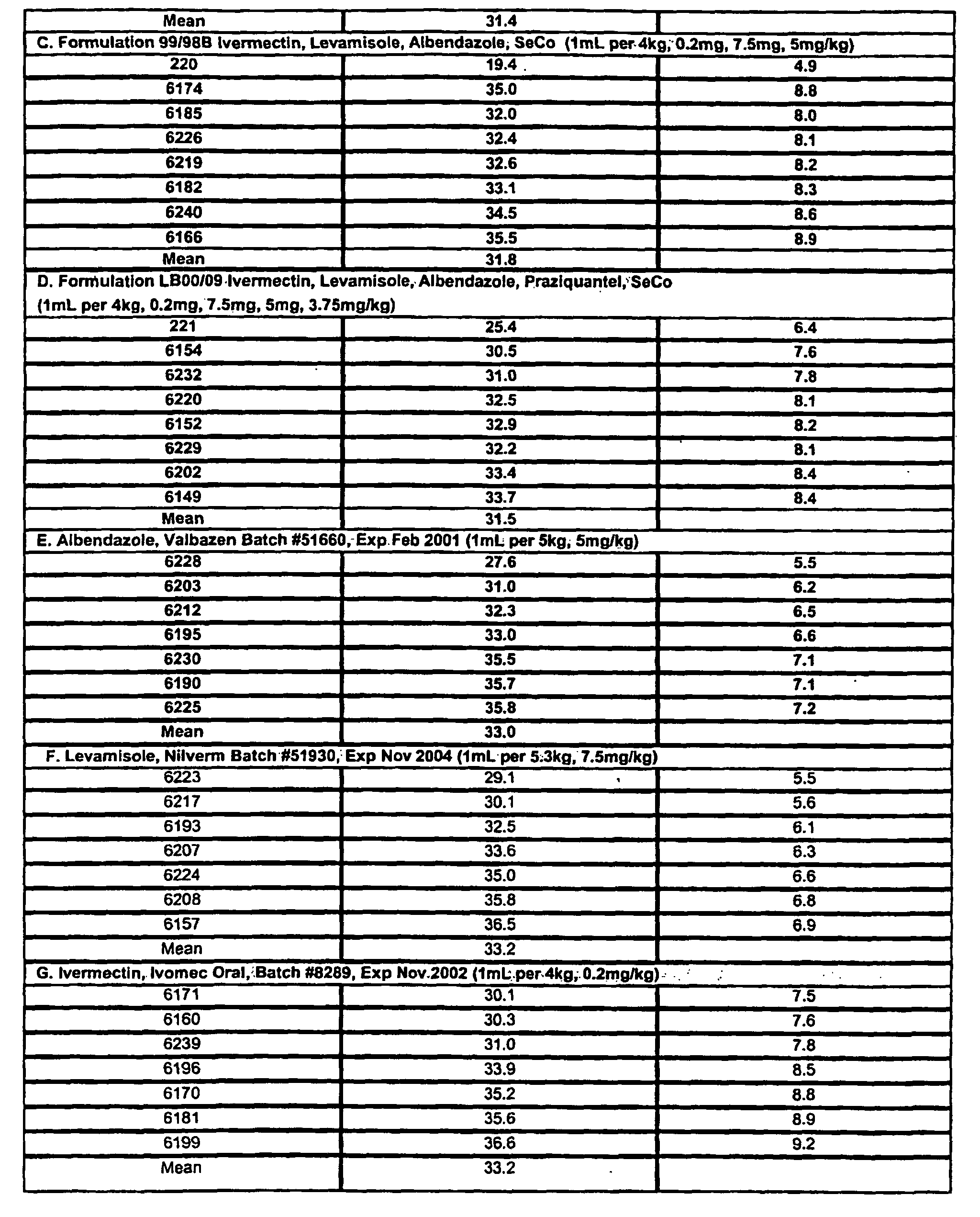

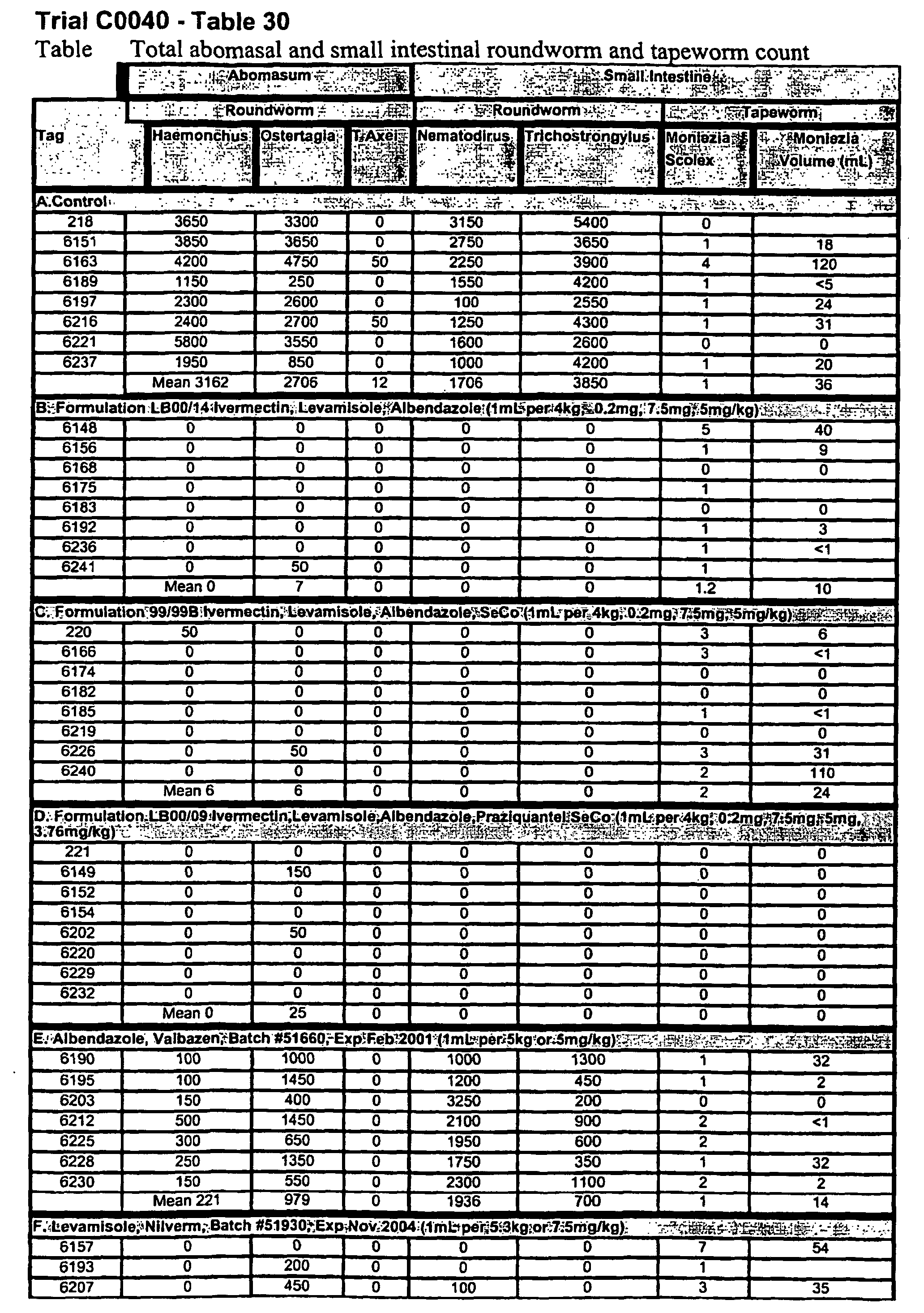

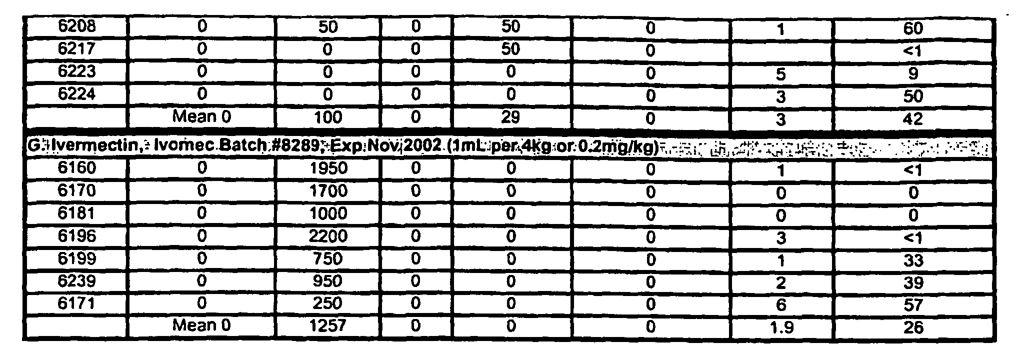

## Claims

1. **A storage stable veterinary composition** comprising or including
(i) at least one lipophilic active ingredient chosen from the class of macro cyclic lactones (hereinafter referred to a "ML") as a first active ingredient,
(ii) at least one organic liquid carrier which carries at least most of the said first active ingredient to define an organic liquid phase,
(iii) levamisole as a second active ingredient,
(iv) an aqueous phase which comprises at least water which carries at least most of the said second active ingredient; and
(v) optionally a particulate content,
**wherein** said aqueous phase has a pH of less than 7,
**and wherein** there is a present in the said aqueous phase an emulsifying agent or agents,
**and wherein** said phases exist in, or can be shaken or agitated into, the form of an emulsion with said particulate content, if any, at least substantially present in the aqueous phase.

2. A composition of claim 1, wherein the said at least one lipophilic active ingredient is avermectin.

3. A composition of claim 2, wherein the avermectin is abamectin or ivermectin.

4. A composition as claimed in any one of claims 1 to 3 further including benzimidazole as a third active ingredient and wherein the said third active ingredient is part of the said aqueous phase.

5. A composition as claimed in any one of claims 1 to 4, wherein there is present in said aqueous phase a particulate content.

6. A composition as claimed in any one of claims 1 to 5, wherein the composition includes at least one of the group consisting of a thickening agent, an antiflocculant, an antifreeze agent and an antimicrobial agent.

7. A composition as claimed in any one of the preceding claims wherein said aqueous phase has a pH of less than 6.

8. A composition of claim 7, wherein said aqueous phase has a pH of less than 5.

9. A composition as claimed in claim 8, wherein said aqueous phase has a pH of less than 4.

10. A composition as claimed in any one of the preceding claims, wherein said aqueous phase includes a buffering system to buffer the pH.

11. A composition as claimed in claim 10, wherein said buffering system is a citric acid/citrate salt system.

12. A composition as claimed in any one of the preceding claims wherein said at least one organic liquid carrier is or includes one or more of an oil and emollient ester.

13. A composition as claimed in claim 12 wherein said at least one organic liquid carrier is at least one mineral or vegetable oil.

14. A composition as claimed in claim 12 or claim 13, wherein said organic liquid phase includes a co-solvent to the at least one oil.

15. A composition as claimed in claim 14, wherein said co-solvent is selected from diols, glycol ethers and an alcohol having multiple carbons.

16. A composition as claimed in claim 14 or claim 15, wherein said organic liquid phase includes benzyl alcohol as a co-solvent.

17. A composition as claimed in claim 6 wherein the particulate content of the aqueous phase is either an active agent or an inert substance.

18. A composition as claimed in claim 17 wherein the particulate active agent is a biocide.

19. A composition as claimed in any one of the preceding claims which includes in one or other, or both, of the partitioned phases one or more of the group comprising minerals and vitamins.

20. A composition of claim 4, wherein the benzimidazole is albendazole.

21. A composition as claimed in any one of the preceding claims comprising:
about 0.08% w/v invermectin,
about 3% w/v levamisole, and
about 2% w/v albendazole wherein different liquid carrier phases substantially partition ivermectin from the levamisole, and
wherein the albendazole is particulate and at least in part in an aqueous phase with the levamisole, such aqueous phase being buffered to a pH appropriate for the levamisole and its stability, and
wherein the ivermectin is substantially in an organic phase.

22. A composition of any one of the preceding claims in the form of an emulsion.

23. A composition of any one of the preceding claims for the treatment of helminthiasis.

24. Use of the composition of any one of claims 1 to 22 in the manufacture of a medicament for treating helminthiasis.

## Patentansprüche

1. Lagerbeständige veterinärmedizinische Zusammensetzung, umfassend oder enthaltend
(i) mindestens einen lipophilen Wirkstoff, ausgewählt aus der Klasse der makrocyclischen Lactone (nachfolgend als "ML" bezeichnet) als ersten Wirkstoff,
(ii) mindestens einen organischen flüssigen Träger, welcher zumindest den größten Teil des ersten Wirkstoffs trägt, um eine organische flüssige Phase zu definieren,
(iii) Levamisol als zweiten Wirkstoff,
(iv) eine wässrige Phase, welche mindestens Wasser umfasst, welche zumindest den größten Teil des zweiten Wirkstoffs trägt, und
(v) optional einen partikulären Anteil,
worin die wässrige Phase einen pH von weniger als 7 aufweist, und
worin in der wässrigen Phase ein oder mehrere Emulgiermittel vorhanden sind, und
worin die Phasen in einer Form einer Emulsion vorliegen oder durch Schütteln oder Bewegen in Form einer Emulsion gebracht werden können, wobei der partikuläre Anteil, falls vorhanden, zumindest teilweise in der wässrigen Phase vorhanden ist.

2. Zusammensetzung nach Anspruch 1, worin der mindestens eine lipophile Wirkstoff Avermectin ist.

3. Zusammensetzung nach Anspruch 2, worin das Avermectin Abamectin oder Ivermectin ist.

4. Zusammensetzung nach einem der Ansprüche 1 bis 3, weiter umfassend Benzimidazol als dritten Wirkstoff, und worin der dritte Wirkstoff Teil der wässrigen Phase ist.

5. Zusammensetzung nach einem der Ansprüche 1 bis 4, worin in der wässrigen Phase ein partikulärer Anteil vorhanden ist.

6. Zusammensetzung nach einem der Ansprüche 1 bis 5, worin die Zusammensetzung mindestens einen Bestandteil enthält aus der Gruppe umfassend ein Verdickungsmittel, ein Mittel gegen Ausflockung, ein Frostschutzmittel und ein Konservierungsmittel.

7. Zusammensetzung nach einem der vorhergehenden Ansprüche, worin die wässrige Phase einen pH von weniger als 6 aufweist.

8. Zusammensetzung nach Anspruch 7, worin die wässrige Phase einen pH von weniger als 5 aufweist.

9. Zusammensetzung nach Anspruch 8, worin die wässrige Phase einen pH von weniger als 4 aufweist.

10. Zusammensetzung nach Anspruch einem der vorhergehenden Ansprüche, worin die wässrige Phase ein Puffersystem umfasst, um den pH abzupuffern.

11. Zusammensetzung nach Anspruch 10, worin das Puffersystem ein Citronensäure/Citratsalz-System ist.

12. Zusammensetzung nach einem der vorhergehenden Ansprüche, worin der mindestens eine organische flüssige Träger mindestens eine Substanz, ausgewählt aus einem Öl und einem weichmachenden Ester ist oder enthält.

13. Zusammensetzung nach Anspruch 12, worin der mindestens eine organische flüssige Träger mindestens ein Mineralöl oder Pflanzenöl ist.

14. Zusammensetzung nach Anspruch 12 oder 13, worin die organische flüssige Phase neben dem mindestens einen Öl ein Co-Lösungsmittel enthält.

15. Zusammensetzung nach Anspruch 14, worin das Co-Lösungsmittel ausgewählt ist aus Diolen, Glycolethern und einem Alkohol mit mehreren Kohlenstoffen.

16. Zusammensetzung nach Anspruch 14 oder Anspruch 15, worin die organische flüssige Phase als Co-Lösungsmittel Benzylalkohol enthält.

17. Zusammensetzung nach Anspruch 6, worin der partikuläre Anteil der wässrigen Phase entweder ein Wirkstoff oder eine inerte Substanz ist.

18. Zusammensetzung nach Anspruch 17, worin der partikuläre Wirkstoff ein Biozid ist.

19. Zusammensetzung nach einem der vorhergehenden Ansprüche, welches in einer oder der anderen oder beiden der geteilten Phasen mindestens eine Substanz der Gruppe enthält, welche Mineralien und Vitamine umfasst.

20. Zusammensetzung nach Anspruch 4, worin das Benzimidazol Albendazol ist.

21. Zusammensetzung nach einem der vorhergehenden Ansprüche, umfassend:
etwa 0,08 % w/v Invermectin,
etwa 3% w/v Levamisol und
etwa 2% w/v Albendazol,
worin unterschiedliche flüssige Trägerphasen im wesentlichen das Invermectin vom Levamisol trennen, und
worin das Albendazol partikulär ist und zumindest teilweise mit dem Levamisol in einer wässrigen Phase vorliegt, wobei diese wässrige Phase so abgepuffert ist, dass der pH für das Levamisol und dessen Stabilität geeignet ist, und
worin das Ivermectin im wesentlichen in einer organischen Phase vorliegt.

22. Zusammensetzung nach einem der vorhergehenden Ansprüche in Form einer Emulsion.

23. Zusammensetzung nach einem der vorhergehenden Ansprüche zur Behandlung einer Wurmerkrankung.

24. Verwendung der Zusammensetzung nach einem der Ansprüche 1 bis 22 für die Herstellung eines Arzneimittels zur Behandlung einer Wurmerkrankung.

## Revendications

1. Composition vétérinaire stable au stockage, comprenant ou renfermant
(i) au moins un ingrédient actif lipophile choisi dans la catégorie des lactones macrocycliques (désignées ci-après par "ML") comme premier ingrédient actif,
(ii) au moins un véhicule liquide organique qui porte au moins la plus grande partie dudit premier ingrédient actif pour définir une phase liquide organique,
(iii) du lévamisole comme second ingrédient actif,
(iv) une phase aqueuse qui comprend au moins de l'eau qui porte au moins la plus grande partie dudit second ingrédient actif ; et
(v) facultativement, une quantité de particules,
**dans laquelle** ladite phase aqueuse a un pH inférieur à 7,
**et dans laquelle** sont présents dans ladite phase aqueuse un ou plusieurs agents émulsionnants,
**et dans laquelle** lesdites phases existent, ou peuvent être agitées par secousse ou agitées, sous forme d'une émulsion avec ladite quantité de particules, s'il en existe une quelconque, au moins substantiellement présente dans la phase aqueuse.

2. Composition suivant la revendication 1, dans laquelle ledit au moins un ingrédient actif lipophile est une avermectine.

3. Composition suivant la revendication 2, dans laquelle l'avermectine est l'abamectine ou l'ivermectine.

4. Composition suivant l'une quelconque des revendications 1 à 3, comprenant en outre du benzimidazole comme troisième ingrédient actif et dans laquelle ledit troisième ingrédient actif fait partie de ladite phase aqueuse.

5. Composition suivant l'une quelconque des revendications 1 à 4, dans laquelle est présente dans ladite phase aqueuse une quantité de particules.

6. Composition suivant l'une quelconque des revendications 1 à 5, ladite composition comprenant au moins un agent du groupe consistant en un agent épaississant, un antifloculant, un agent antigel et un agent antimicrobien.

7. Composition suivant l'une quelconque des revendications précédentes, dans laquelle ladite phase aqueuse a un pH inférieur à 6.

8. Composition suivant la revendication 7, dans laquelle ladite phase aqueuse a un pH inférieur à 5.

9. Composition suivant la revendication 8, dans laquelle ladite phase aqueuse a un pH inférieur à 4.

10. Composition suivant l'une quelconque des revendications précédentes, dans laquelle ladite phase aqueuse comprend un système tampon pour tamponner le pH.

11. Composition suivant la revendication 10, dans laquelle ledit système tampon est système d'acide citrique/sel citrate.

12. Composition suivant l'une quelconque des revendications précédentes, dans laquelle ledit au moins un véhicule liquide organique est ou comprend un ou plusieurs des agents consistant en une huile et un ester émollient.

13. Composition suivant la revendication 12, dans laquelle ledit au moins un véhicule liquide organique est au moins une huile minérale ou végétale.

14. Composition suivant la revendication 12 ou la revendication 13, dans laquelle ladite phase liquide organique comprend un cosolvant pour au moins une huile.

15. Composition suivant la revendication 14, dans laquelle ledit cosolvant est choisi entre des diols, des éthers de glycols et un alcool ayant une multiplicité d'atomes de carbone.

16. Composition suivant la revendication 14 ou la revendication 15, dans laquelle ladite phase liquide organique comprend l'alcool benzylique comme cosolvant.

17. Composition suivant la revendication 6, dans laquelle la quantité de particules de la phase aqueuse est constituée d'un agent actif ou d'une substance inerte.

18. Composition suivant la revendication 17, dans laquelle l'agent actif en particules est un biocide.

19. Composition suivant l'une quelconque des revendications précédentes, qui comprend dans l'une ou l'autre ou les deux des phases présentant un partage un ou plusieurs des membres du groupe comprenant des substances minérales et des vitamines.

20. Composition suivant la revendication 4, dans laquelle le benzimidazole est l'albendazole.

21. Composition suivant l'une quelconque des revendications précédentes, comprenant :
environ 0,08 % en poids/volume d'ivermectine,
environ 3 % en poids/volume de lévamisole, et
environ 2 % en poids/volume d'albenzadole, dans laquelle des phases de véhicule liquide différentes provoquent un partage substantiel entre l'ivermectine et le lévamisole, et
dans laquelle l'albendazole est en particules et au moins en partie dans une phase aqueuse avec le lévamisole, cette phase aqueuse étant tamponnée à un pH approprié pour le lévamisole et sa stabilité, et
dans laquelle l'ivermectine est substantiellement dans une phase organique.

22. Composition suivant l'une quelconque des revendications précédentes, sous forme d'une émulsion.

23. Composition suivant l'une quelconque des revendications précédentes, pour le traitement de l'helminthiase.

24. Utilisation de la composition suivant l'une quelconque des revendications 1 à 22 dans la production d'un médicament destiné au traitement de l'helminthiase.
